# EUROPÄISCHE PATENTSCHRIFT

(11) **EP 0 837 847 B1**
(45) Veröffentlichungstag und Bekanntmachung des Hinweises auf die Patenterteilung: **18.09.2002**
(21) Anmeldenummer: 96922005.2
(22) Anmeldetag: 17.06.1996
(51) Int. Cl.: C07D 207/38, C07D 307/60, C07D 333/32, C07D 309/32, C07D 279/06, A01N 43/36, A01N 43/86, A01N 43/08, A01N 43/10

(54) **2,4,5-TRISUBSTITUIERTE PHENYLKETOENOLE ZUR VERWENDUNG ALS PESTIZIDE UND HERBIZIDE**
2,4,5-TRISUBSTITUTED PHENYLKETO-ENOLS FOR USE AS PESTICIDES AND HERBICIDES
PHENYLCETO-ENOLS 2,4,5-TRISUBSTITUES S'UTILISANT COMME PESTICIDES ET HERBICIDES

(30) Priorität: 28.06.1995 DE 19523471; 25.01.1996 DE 19602524
(43) Veröffentlichungstag der Anmeldung: 29.04.1998
(73) Patentinhaber: BAYER AG, 51368 Leverkusen (DE)
(72) Erfinder: LIEB, Folker, D-51375 Leverkusen (DE); HAGEMANN, Hermann, D-51375 Leverkusen (DE); WIDDIG, Arno, D-51519 Odenthal (DE); RUTHER, Michael, D-40789 Monheim (DE); FISCHER, Reiner, D-40789 Monheim (DE); BRETSCHNEIDER, Thomas, D-53797 Lohmar (DE); ERDELEN, Christoph, D-42799 Leichlingen (DE); WACHENDORFF-NEUMANN, Ulrike, D-56566 Neuwied (DE); SANTEL, Hans-Joachim, D-51371 Leverkusen (DE); DOLLINGER, Markus, D-51381 Leverkusen (DE); GRAFF, Alan, D-51427 Bergisch Gladbach (DE); MENCKE, Norbert, D-51381 Leverkusen (DE); TURBERG, Andreas, D-40699 Erkrath (DE); DAHMEN, Peter, D-41470 Neuss (DE)
(86) Internationale Anmeldenummer: EP9602606
(87) Internationale Veröffentlichungsnummer: WO97001535

(56) Entgegenhaltungen:
- EP-A- 0 596 298
- EP-A- 0 613 885
- EP-A- 0 647 637
- WO-A-95/01358
- WO-A-95/01971
- WO-A-95/14013
- WO-A-95/20572
- WO-A-95/26954
- DE-A- 4 410 420

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide und/oder Herbizide wirksam sind.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, DE 44 40 594, WO 94/01 997 und WO 95/01 358).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A 0 647 637 und WO 95/26345 bekannt.

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785 beschrieben.

Die akarizide und insektizide Wirksamkeit und/oder Wirkungsbreite, und die Pflanzenverträglichkeit dieser Verbindungen, insbesondere gegenüber Kulturpflanzen, ist jedoch nicht immer ausreichend.

Es wurden nun neue Verbindungen der Formel (I) 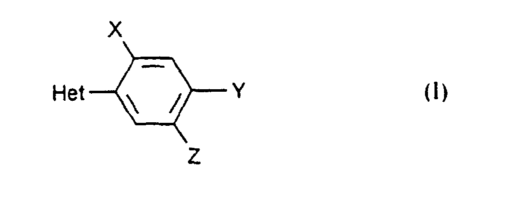 gefunden,
in welcher
- X: für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy, Cyano oder Nitro,
- Y: für Halogen, C₁-C₆-Alkyl, C₁-C₆-Alkoxy, C₁-C₄-Halogenalkyl, C₁-C₄-Halogenalkoxy, Cyano oder Nitro,
- Z: für Halogen, C₁-C₆-Alkyl, C₁-C₆-Alkoxy, C₁-C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₇-alkylthio oder
- Y und Z: gemeinsam für ein gegebenenfalls durch Halogen, C₁-C₆-Alkyl, C₁-C₆-Alkoxy oder C₁-C₄-Halogenalkyl substituiertes C₃-C₄-Alkandiyl oder C₃-C₄-Alkendiyl, in welchen gegebenenfalls ein bis drei Glieder durch Sauerstoff, Schwefel, Stickstoff oder eine Carbonylgruppe unabhängig voneinander ersetzt sein können, wobei X eine der obengenannten Bedeutungen hat stehen
- Het: für eine der Gruppen 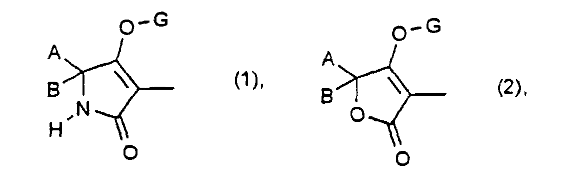 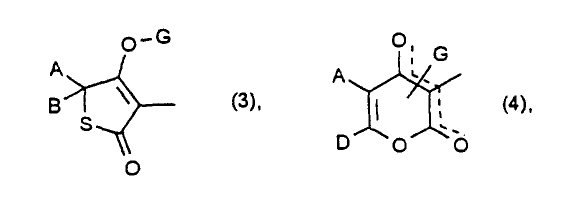 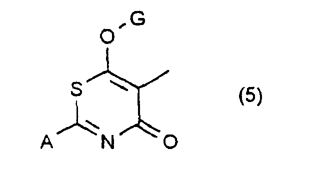
- A: für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff.
- B: für Wasserstoff, C₁-C₁₂-Alkyl oder C₁-C₈-Alkoxy-C₁-C₆alkyl oder
- A, B: und das Kohlenstoffatom an das sie gebunden sind, für C₃-C₁₀-Cycloalkyl oder C₅-C₁₀-Cycloalkenyl, worin gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, für C₅-C₆-Cycloalkyl, welches durch eine gegebenenfalls ein oder zwei Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind für C₃-C₈-Cycloalkyl oder C₅-C₈-Cycloalkenyl, in dem zwei Substituenten gemeinsam mit Kohlenstoffatomen, an die sie gebunden sind, für jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl stehen, worin jeweils gegebenenfalls eine Methylengruppe durch Sauerstoff oder Schwefel ersetzt ist.
- D: für Wasserstoff, für jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 bis 6 Ringatomen und ein oder zwei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein oder zwei Heteroatomen aus der Reihe Sauerstoff, Schwefel und Stickstoff oder
- A und D: gemeinsam für eine C₃-C₆-Alkandiyl-, C₃-C₆-Alkendiyl- oder C₄-C₆-Alkadiendiylgruppe, in welchen jeweils gegebenenfalls eine Methylengruppe durch Sauerstoff oder Schwefel ersetzt ist und welche jeweils gegebenenfalls substituiert sind durch Halogen oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy oder durch eine weitere, einen ankondensierten Ring bildende C₃-C₆-Alkandiyl-, C₃-C₆-Alkendiyl- oder C₄-C₆-Alkadiendiylgruppe, in welchen gegebenenfalls jeweils eine Methylengruppe durch Sauerstoff oder Schwefel ersetzt ist und welche gegebenenfalls durch C₁-C₆-Alkyl substituiert sind, oder
- A und D: gemeinsam für eine C₃-C₆-Alkandiyl- oder C₃-C₆-Alkendiylgruppe, worin jeweils gegebenenfalls eine der folgenden Gruppen 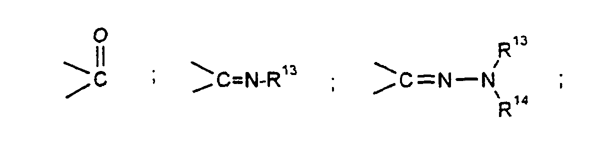 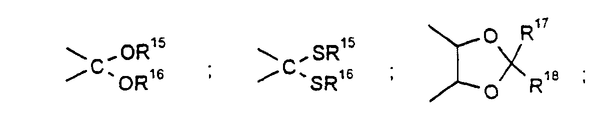 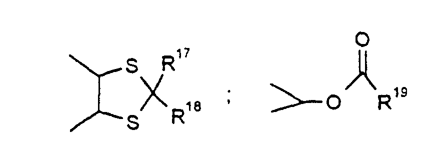 oder 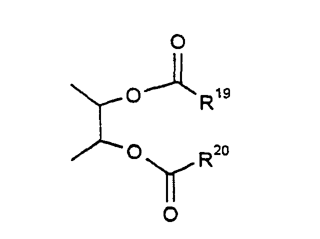 enthalten ist,
- G: für Wasserstoff (a) oder für eine der Gruppen 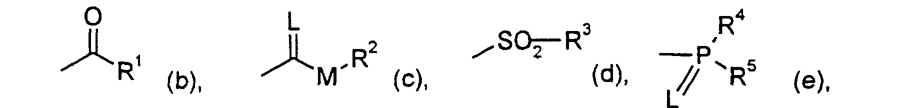 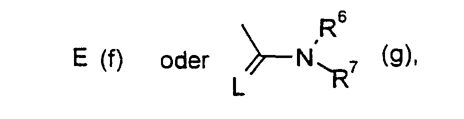 in welchen
E für ein Metallionäquivalent oder ein Ammoniumion steht,
L für Sauerstoff oder Schwefel steht und
M für Sauerstoff oder Schwefel steht.
- R¹: für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff.
- R²: für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.
- R³: für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
- R⁴ und R⁵: unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio.
- R⁶ und R⁷: unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest, in welchem gegebenenfalls eine Methylengruppe durch Sauerstoff oder Schwefel ersetzt ist.
- R¹³: für Wasserstoff oder jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄alkoxy.
- R¹⁴: für Wasserstoff oder C₁-C₈-Alkyl oder
- R¹³ und R¹⁴: gemeinsam für C₄-C₆-Alkandiyl.
- R¹⁵ und R¹⁶: sind gleich oder verschieden und für C₁-C₆-Alkyl oder
- R¹⁵ und R¹⁶: gemeinsam für einen C₂-C₄-Alkandiylrest, der gegebenenfalls durch C₁-C₆-Alkyl oder durch gegebenenfalls durch Halogen, C₁-C₄-Alkyl, C₁-C₄-Halogenalkyl, C₁-C₄-Alkoxy, C₁-C₄-Halogenalkoxy, Nitro oder Cyano substituiertes Phenyl substituiert ist.
- R¹⁷ und R¹⁸: unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
- R¹⁷ und R¹⁸: gemeinsam mit dem Kohlenstoffatom, an das sie gebunden sind, für gegebenenfalls durch C₁-C₄-Alkyl substituiertes C₅-C₇-Cycloalkyl, in dem gegebenenfalls eine Methylengruppe durch Sauerstoff oder Schwefel ersetzt ist.
- R¹⁹ und R²⁰: unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino. stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in ublicher Art und Weise getrennt werden können. Sowohl die reinen Isomeren als auch die Isomerengemische, deren Herstellung und Verwendung sowie diese enthaltende Mittel sind Gegenstand der vorliegenden Erfindung Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (5) der Gruppe Het ergeben sich folgende hauptsächliche Strukturen (I-1) bis (I-5): 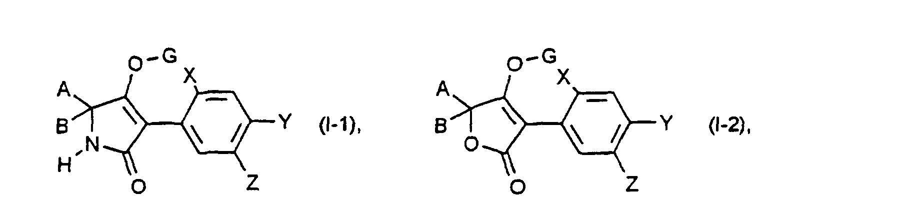 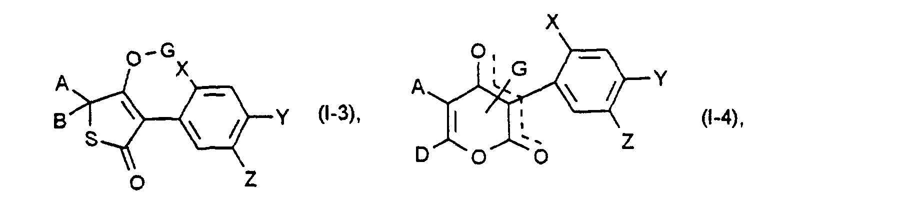 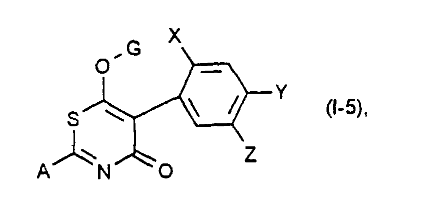 worin
A, B, D, G, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 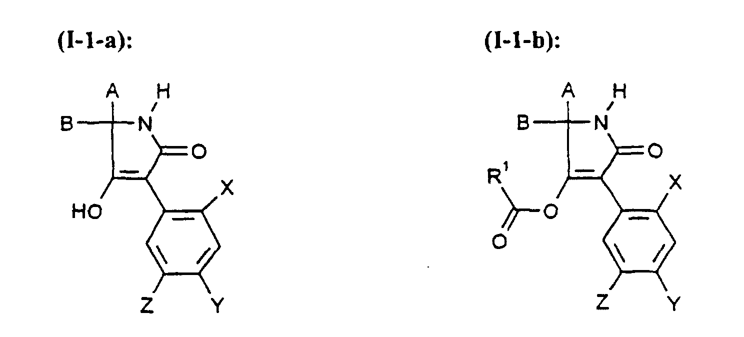 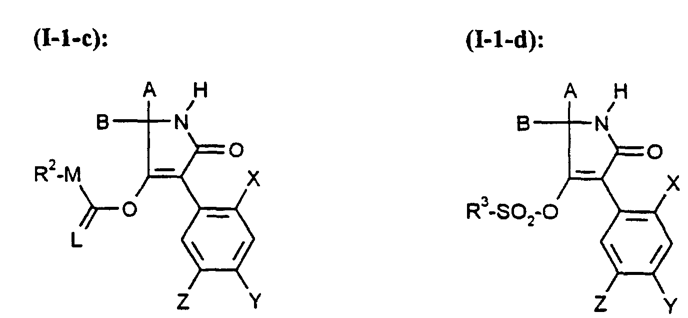 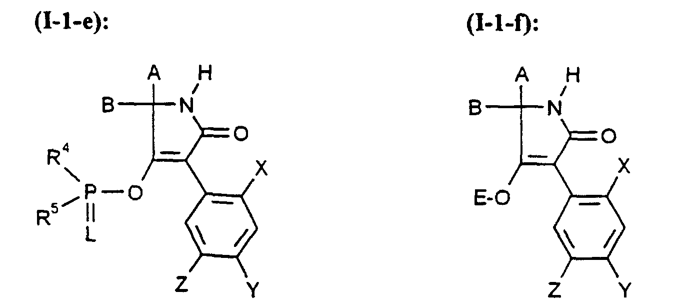 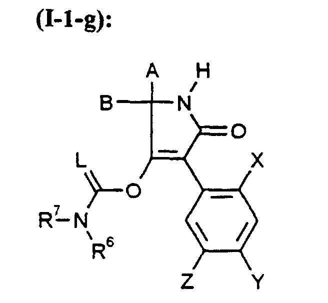 worin
A, B, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn Het für die Gruppe (2) steht, 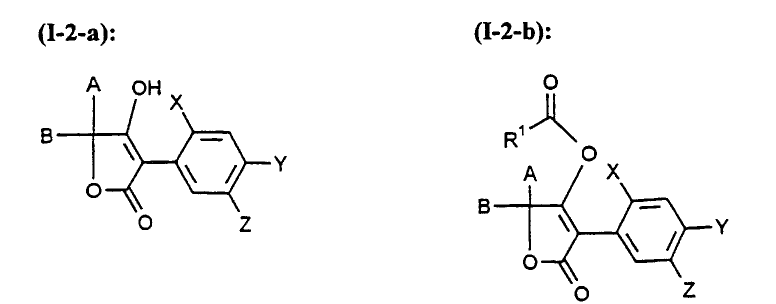 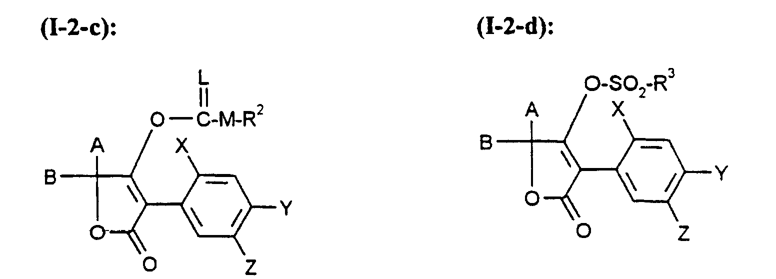 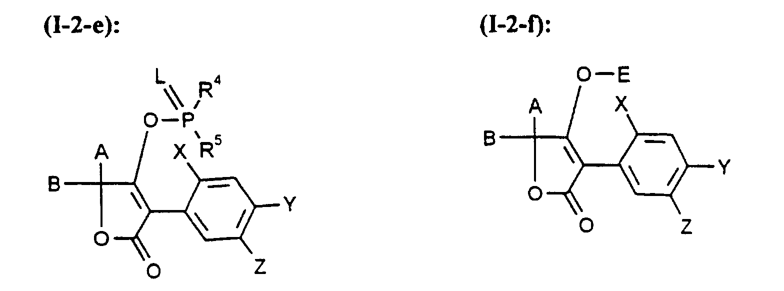 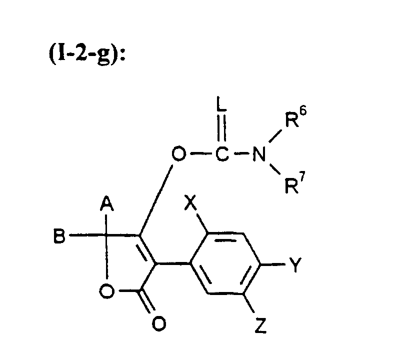 worin
A, B, E, L, M, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn Het für die Gruppe (3) steht, 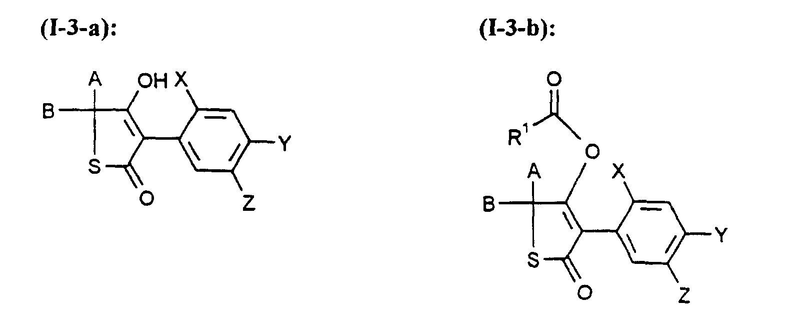 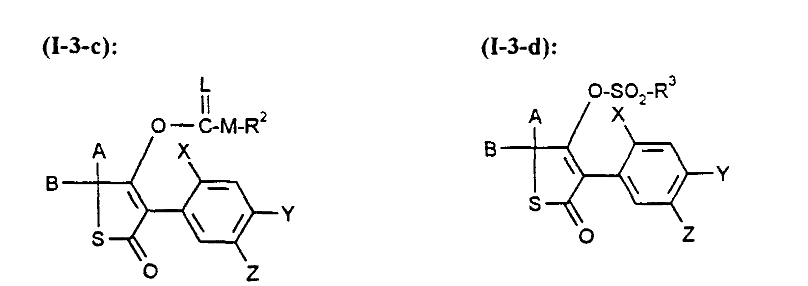 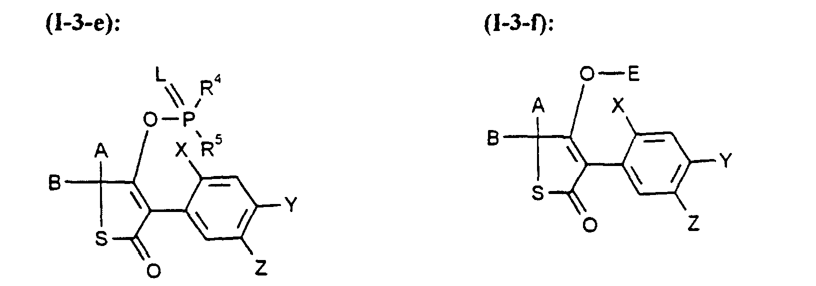 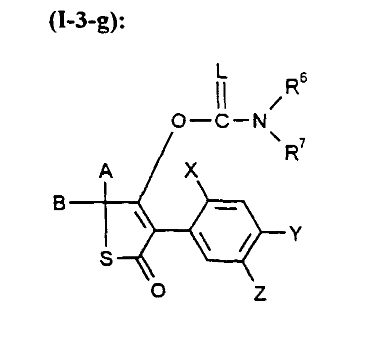 worin
A, B, E, L, M, X, Y, Z, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Formen der Formeln (I-4)ₐ und (I-4)_{b} vorliegen, 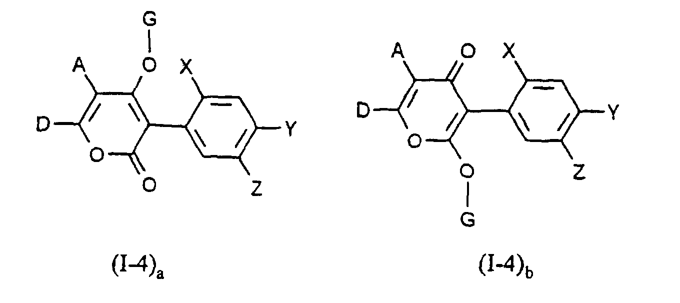 was durch die gestrichelte Linie in der Formel (I-4) zum Ausdruck gebracht werden soll.

Die Verbindungen der Formeln (I-4)ₐ und (I-4)_{b} können sowohl als Gemische als auch in Form ihrer reinen Isomeren vorliegen. Gemische der Verbindungen der Formeln (I-4)ₐ und (I-4)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn Het für die Gruppe (4) steht, 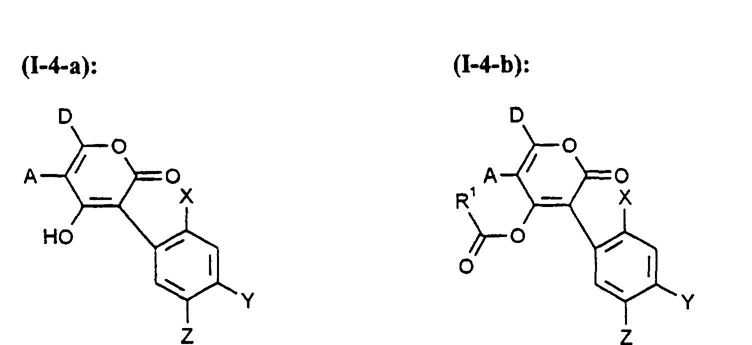 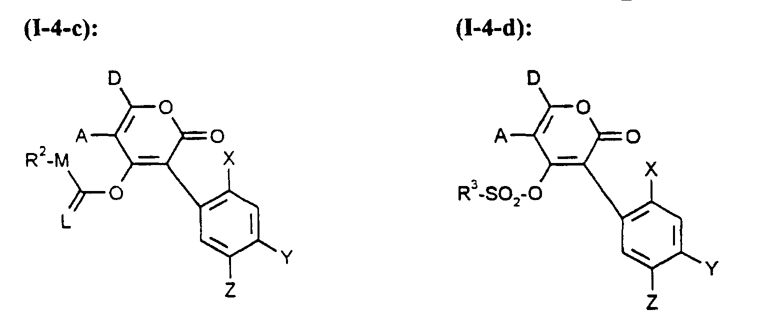 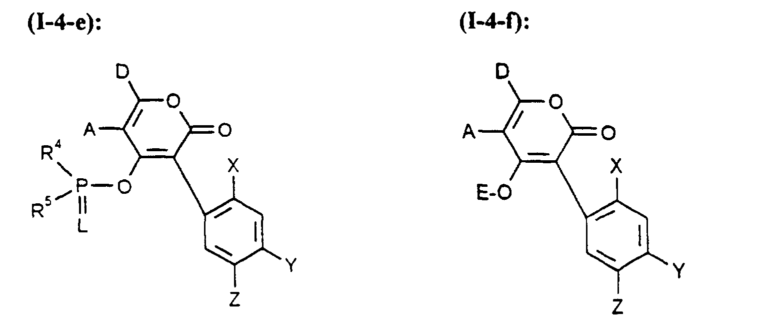 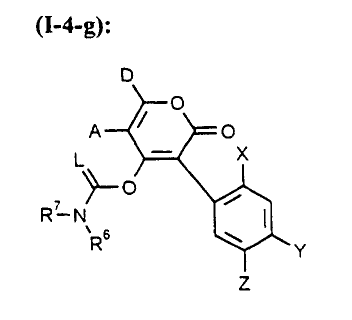 worin
A, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn Het für die Gruppe (5) steht, 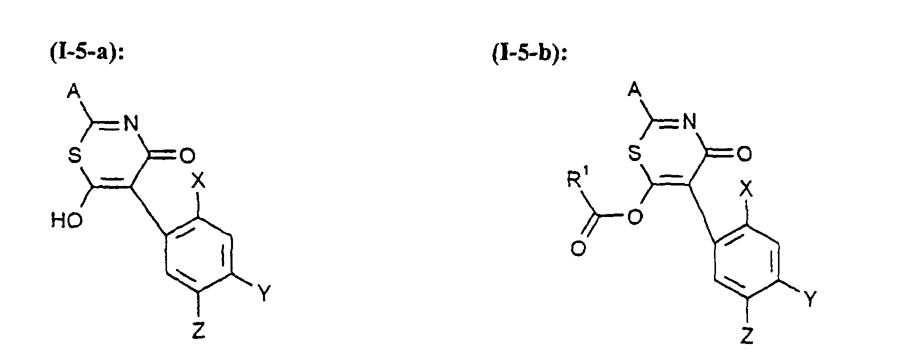 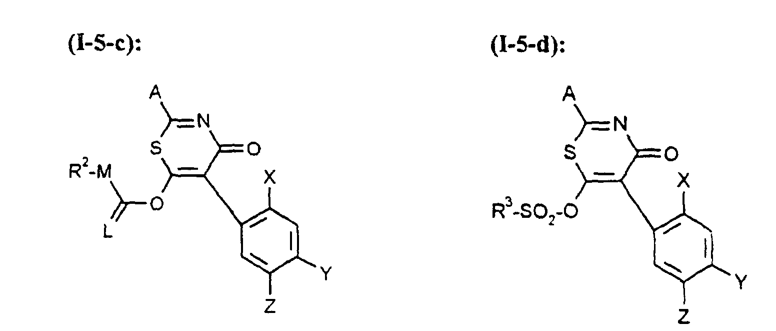 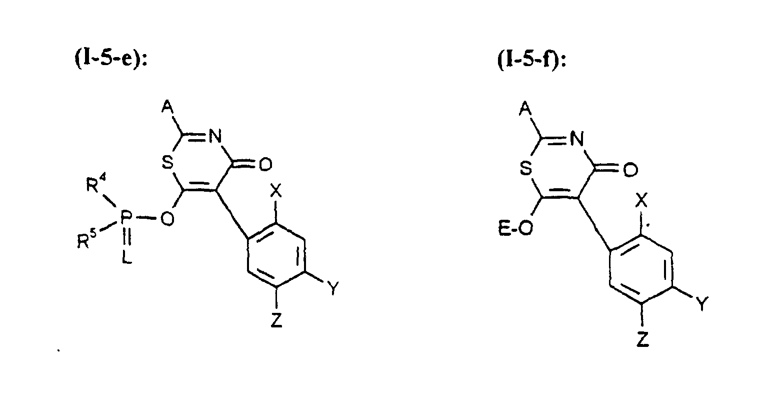 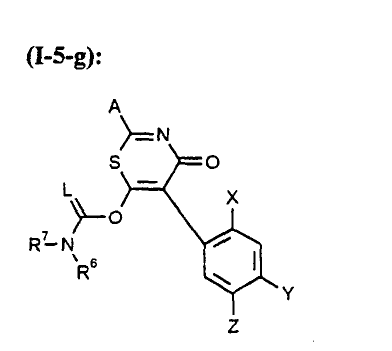 worin
A, E, L, M, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält Verbindungen der Formel (I-1-a) 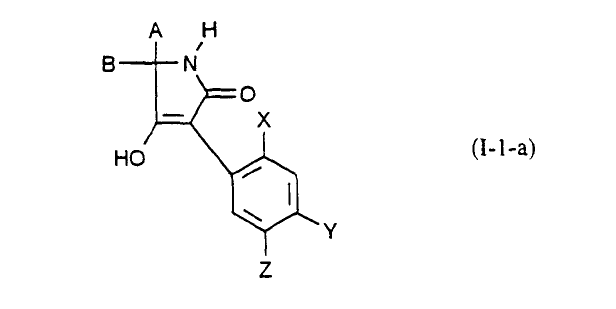 in welcher
   A, B, X, Y und Z die oben angegebenen Bedeutungen haben,
   wenn man
   Verbindungen der Formel (II) 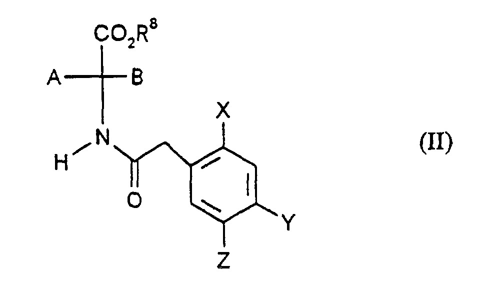 in welcher
   A, B, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man Verbindungen der Formel (I-2-a) 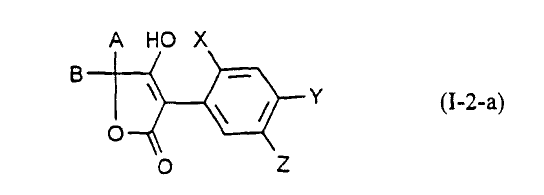 in welcher
   A, B, X, Y und Z die oben angegebenen Bedeutungen haben,
   erhält, wenn man
   Verbindungen der Formel (III) 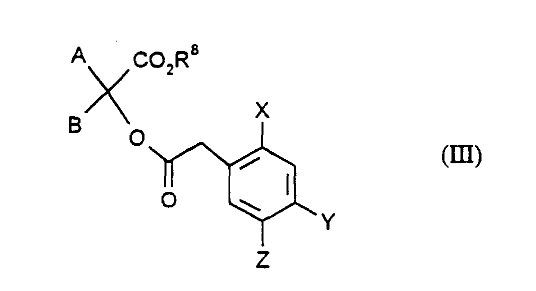 in welcher
   A, B, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, daß man Verbindungen der Formel (I-3-a) 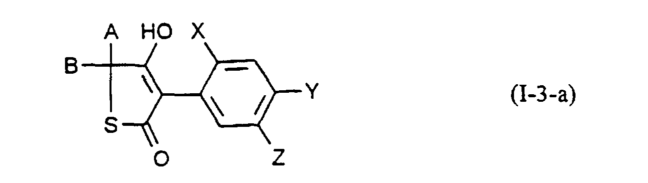 in welcher
   A, B, X, Y und Z die oben angegebenen Bedeutungen haben,
   erhält, wenn man
   Verbindungen der Formel (IV) 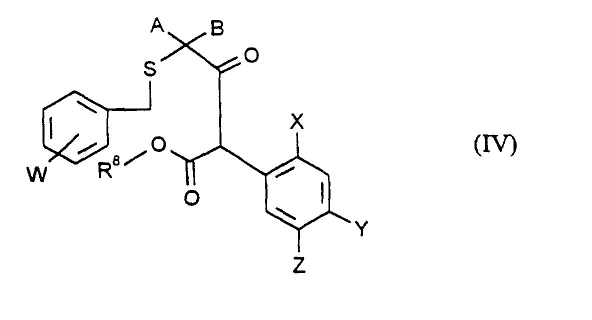 in welcher
   A, B, X, Y, Z und R⁸ die oben angegebenen Bedeutungen haben und
   - W: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin wurde gefunden, daß man die Verbindungen der Formel (I-4-a) 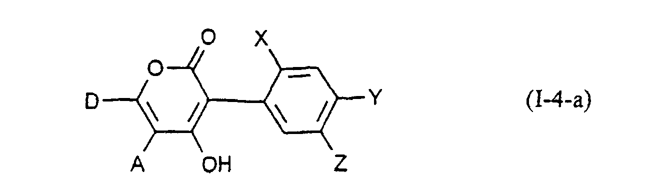 in welcher
   A, D, X, Y und Z die oben angegebenen Bedeutungen haben,
   erhält, wenn man
   Verbindungen der Formel (V) 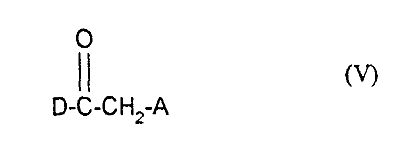 in welcher
   - A und D: die oben angegebenen Bedeutungen haben,
   oder deren Silylenolether der Formel (Va) 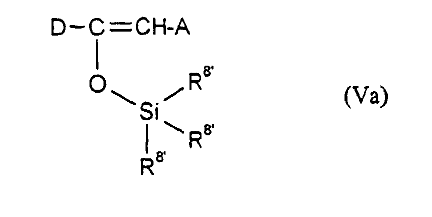 in welcher
   - A und D: die oben genannte Bedeutung haben und
   - R^{8'}: für Alkyl (bevorzugt Methyl) steht,
   mit Verbindungen der Formel (VI) 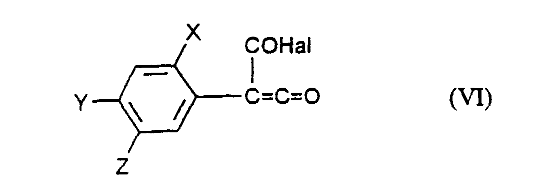 in welcher
   - X, Y und Z: die oben angegebenen Bedeutungen haben und
   - Hal: für Halogen (vorzugsweise für Chlor oder Brom) steht,
   gegebenenfalls in Gegenwart eines Verdünnungsmittels und gegebenenfalls in Gegenwart eines Säureakzeptors umsetzt.
(E) Weiterhin wurde gefunden, daß man die Verbindungen der Formel (I-5-a) 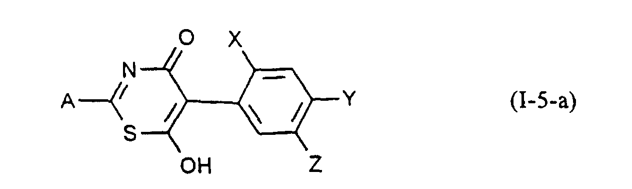 in welcher
   - A, X, Y und Z: die oben angegebenen Bedeutungen haben,
   erhält, wenn man Verbindungen der Formel (VII) 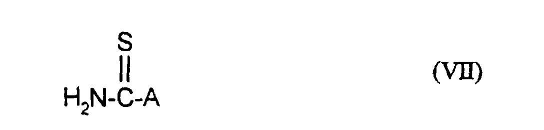 in welcher
   - A: die oben angegebene Bedeutung hat,
   mit Verbindungen der Formel (VI) 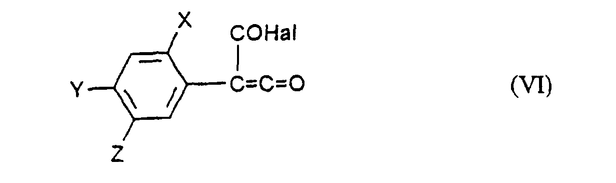 in welcher
   - Hal, X, Y und Z: die oben angegebenen Bedeutungen haben,
   gegebenenfalls in Gegenwart eines Verdünnungsmittels und gegebenenfalls in Gegenwart eines Säureakzeptors umsetzt.
   Außerdem wurde gefunden,
(F) daß man die Verbindungen der oben gezeigten Formeln (I-1-b) bis (I-5-b), in welchen A, B, D, R¹, X, Y und Z die oben angebenen Bedeutungen haben, erhält, wenn man Verbindungen der oben gezeigten Formeln (I-1-a) bis (1-5-a), in welchen A, B, D, X, Y und Z die oben angegebenen Bedeutungen haben,
α) mit Säurehalogeniden der Formel (VIII) 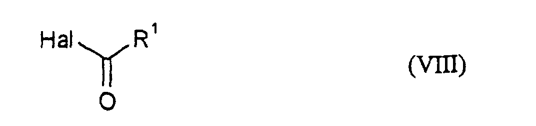 in welcher
   - R¹: die oben angegebene Bedeutung hat und
   - Hal: für Halogen (insbesondere Chlor oder Brom) steht
   oder
β) mit Carbonsäureanhydriden der Formel (IX)

   R¹-CO-O-CO-R¹ (IX)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(G) daß man die Verbindungen der oben gezeigten Formeln (I-1-c) bis (I-5-c), in welchen A, B, D, R², M, X, Y und Z die oben angegebenen Bedeutungen haben und L für Sauerstoff steht, erhält, wenn man Verbindungen der oben gezeigten Formeln (I-1-a) bis (I-5-a), in welchen A, B, D, X, Y und Z die oben angegebenen Bedeutungen haben, jeweils
   mit Chlorameisensaureestern oder Chlorameisensäurethioestern der Formel (X)

   R²-M-CO-Cl (X)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(H) daß man Verbindungen der oben gezeigten Formeln (I-1-c) bis (I-5-c), in welchen A, B, D, R², M, X, Y und Z die oben angegebenen Bedeutungen haben und L für Schwefel steht, erhält, wenn man Verbindungen der oben gezeigten Formeln (I-1-a) bis (I-5-a), in welchen A, B, D, X, Y und Z die oben angegebenen Bedeutungen haben, jeweils
   α) mit Chlormonothioameisensäureestern oder Chlordithioameisensäureestern der Formel (XI) 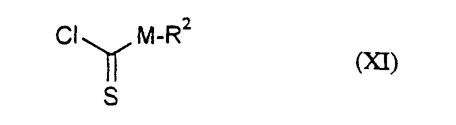 in welcher
      - M und R²: die oben angegebenen Bedeutungen haben,
      gegebenenfalls in Gegenwart eines Verdünnungsmittels und gegebenenfalls in Gegenwart eines Säurebindemittels umsetzt
      oder
   β) mit Schwefelkohlenstoff und anschließend mit Verbindungen der Formel (XII)

      R²-Hal (XII)
   in welcher
   - R²: die oben angegebene Bedeutung hat und
   - Hal: für Chlor, Brom oder Iod steht,
   gegebenenfalls in Gegenwart eines Verdünnungsmittels und gegebenenfalls in Gegenwart einer Base umsetzt,
(I) daß man Verbindungen der oben gezeigten Formeln (I-1-d) bis (I-5-d), in welchen A, B, D, R³, X, Y und Z die oben angegebenen Bedeutungen haben, erhält, wenn man Verbindungen der oben gezeigten Formeln (I-1-a) bis (I-5-a), in welchen A, B, D, X, Y und Z die oben angegebenen Bedeutungen haben, jeweils
   mit Sulfonsäurechloriden der Formel (XIII)

   R³-SO₂-Cl (XIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(J) daß man Verbindungen der oben gezeigten Formeln (I-1-e) bis (I-5-e), in welchen A, B, D, L, R⁴, R⁵, X, Y und Z die oben angegebenen Bedeutungen haben, erhält, wenn man Verbindungen der oben gezeigten Formeln (I-1-a) bis (I-4-a), in welchen A, B, D, X, Y und Z die oben angegebenen Bedeutungen haben, jeweils
   mit Phosphorverbindungen der Formel (XIV) 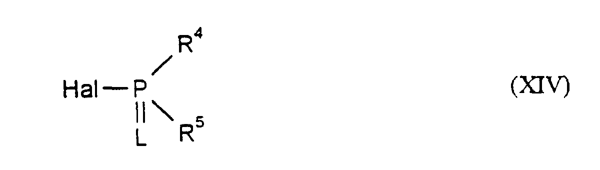 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(K) daß man Verbindungen der oben gezeigten Formeln (I-1-f) bis (I-5-f), in welchen A, B, D, E, X, Y und Z die oben angegebenen Bedeutungen haben, erhält, wenn man Verbindungen der Formeln (I-1-a) bis (I-5-a), in welchen A, B, D, X, Y und Z die oben angegebenen Bedeutungen haben, jeweils
   mit Metallverbindungen oder Aminen der Formeln (XV) oder (XVI)

   Me(OR¹⁰)ₜ (XV)

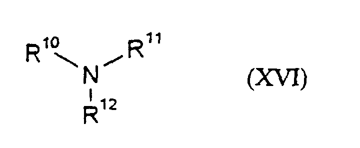

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(L) daß man Verbindungen der oben gezeigten Formeln (I-1-g) bis (I-5-g), in welchen A, B, D, L, R⁶, R⁷, X, Y und Z die oben angegebenen Bedeutungen haben, erhält, wenn man Verbindungen der oben gezeigten Formeln (I-1-a) bis (I-5-a), in welchen A, B, D, X, Y und Z die oben angegebenen Bedeutungen haben, jeweils
   α) mit Isocyanaten oder Isothiocyanaten der Formel (XVII)

      R⁶-N=C=L (XVII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XVIII) 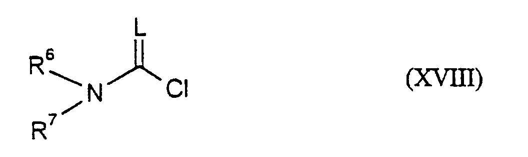 in welcher
      - L, R⁶ und R⁷: die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen und darüber hinaus sehr gut pflanzenverträglich, insbesondere gegenüber Kulturpflanzen, sind

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erlautert
- X: steht besonders bevorzugt für Fluor, Chlor, Brom, C₁-C₄-Alkyl, C₁-C₄-Alkoxy, C₁-C₂-Halogenalkyl, C₁-C₂-Halogenalkoxy, Cyano oder Nitro,
- Y: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro,
- Z: steht besonders bevorzugt für Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄-Alkoxy, C₁-C₂-Haloalkyl, C₁-C₂-Haloalkoxy, Nitro oder Cyano substituiertes Phenoxy oder Benzyloxy, oder
- Y und Z: stehen besonders bevorzugt für gegebenenfalls durch Fluor, Chlor, Brom, C₁-C₄-Alkyl, C₁-C₄-Alkoxy oder C₁-C₂-Halogenalkyl substituiertes C₃-C₄-Alkandiyl oder C₃-C₄-Alkendiyl, in welchem gegebenenfalls ein oder zwei nicht direkt benachbarte Glieder durch Sauerstoff, Schwefel oder Stickstoff unabhängig voneinander ersetzt sein können, wobei X eine der obengenannten Bedeutungen hat.
- Het: steht besonders bevorzugt für eine der Gruppen 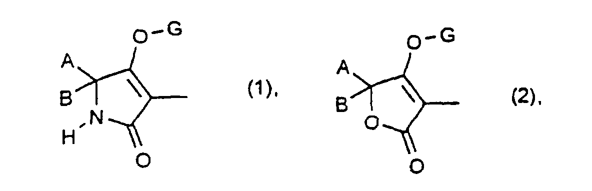 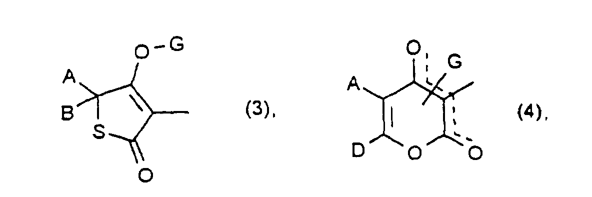 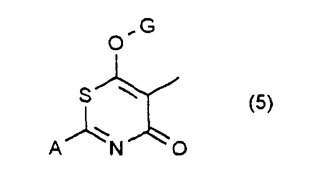
- A: steht besonders bevorzugt für Wasserstoff, für jeweils gegebenenfalls durch Fluor oder Chlor substituiertes C₁-C₁₀-Alkyl, C₂-C₆-Alkenyl, C₁-C₈-Alkoxy-C₁-C₆-alkyl, Poly-C₁-C₆-alkoxy-C₁-C₆-alkyl oder C₁-C₈-Alkylthio-C₁-C₆-alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl.
- B: steht besonders bevorzugt für Wasserstoff, C₁-C₁₀-Alkyl oder C₁-C₆-Alkoxy-C₁-C₄-alkyl oder
- A, B: und das Kohlenstoffatom an das sie gebunden sind, stehen besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert sind oder
- A, B: und das Kohlenstoffatom, an das sie gebunden sind, stehen besonders bevorzugt für C₅-C₆-Cycloalkyl, welches durch eine gegebenenfalls ein oder zwei Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Ring bildet oder
- A, B: und das Kohlenstoffatom, an das sie gebunden sind, stehen besonders bevorzugt für C₃-C₆-Cycloalkyl oder C₅-C₆-Cycloalkenyl, in dem zwei Substituenten gemeinsam mit den Kohlenstoffatomen, an die sie gebunden sind, für jeweils gegebenenfalls durch C₁-C₄-Alkyl, C₁-C₄-Alkoxy, Fluor, Chlor oder Brom substituiertes C₃-C₅-Alkandiyl, C₃-C₅-Alkendiyl oder Butadiendiyl stehen, worin jeweils gegebenenfalls eine Methylengruppe durch Sauerstoff oder Schwefel ersetzt ist.
- D: steht besonders bevorzugt für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl oder
- A und D: stehen gemeinsam besonders bevorzugt für eine C₃-C₅-Alkandiyl- oder C₃-C₅-Alkendiylgruppe, worin jeweils gegebenenfalls ein Kohlenstoffatom durch Sauerstoff oder Schwefel ersetzt ist und welche gegebenenfalls substituiert sind durch Fluor, Chlor oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy oder
worin jeweils gegebenenfalls eine der folgenden Gruppen: 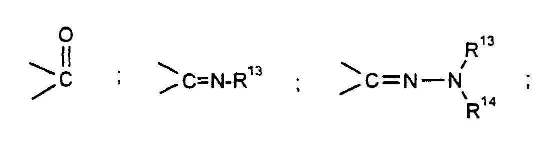 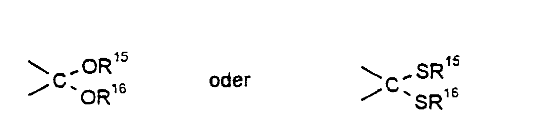 enthalten ist;
- G: steht besonders bevorzugt für Wasserstoff (a) oder für eine der Gruppen 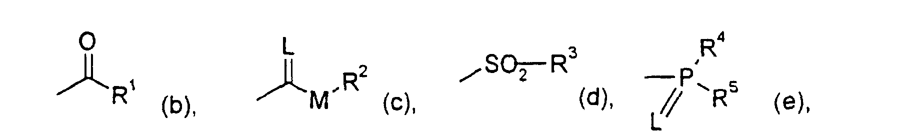 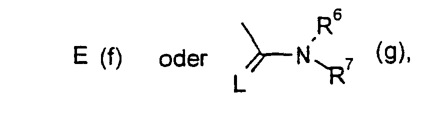 in welchen
E für ein Metallionäquivalent oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor. Brom oder C₁-C₄-Alkyl substituiertes Pyrazolyl, Thiazolyl, Pyridyl, Pyrimidyl. Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls durch Fluor oder Chlor substituiertes C₁-C₆-Alkyl oder jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl.
- R⁴ und R⁵: stehen unabhängig voneinander besonders bevorzugt für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.
- R¹³: steht besonders bevorzugt für Wasserstoff oder jeweils gegebenenfalls durch Fluor oder Chlor substituiertes C₁-C₆-Alkyl oder C₁-C₆-Alkoxy, für gegebenenfalls durch Fluor, C₁-C₂-Alkyl oder C₁-C₂-Alkoxy substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₅-Alkyl, C₁-C₅-Alkoxy, C₁-C₂-Halogenalkyl, C₁-C₂-Halogenalkoxy, Nitro oder Cyano substituiertes Phenyl, Phenyl-C₁-C₃alkyl oder Phenyl-C₁-C₂-alkyloxy.
- R¹⁴: steht besonders bevorzugt für Wasserstoff oder C₁-C₆-alkyl oder
- R¹³ und R¹⁴: stehen gemeinsam besonders bevorzugt für C₄-C₆-Alkandiyl.
- R¹⁵ und R¹⁶: sind gleich oder verschieden und stehen besonders bevorzugt für C₁-C₄-Alkyl oder
- R¹⁵ und R¹⁶: stehen zusammen besonders bevorzugt für einen C₂-C₃-Alkandiylrest, der gegebenenfalls durch C₁-C₄-Alkyl oder durch gegebenenfalls durch Fluor, Chlor, Brom, C₁-C₂-Alkyl, C₁-C₂-Halogenalkyl, C₁-C₂-Alkoxy, C₁-C₂-Halogenalkoxy, Nitro oder Cyano substituiertes Phenyl substituiert ist.
- X: steht ganz besonders bevorzugt für Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro,
- Y: steht ganz besonders bevorzugt für Wasserstoff, Fluor, Chlor, Brom, Methyl, Ethyl, Propyl, iso-Propyl, Butyl, iso-Butyl, tert.-Butyl, Methoxy, Ethoxy, Propoxy, iso-Propoxy, Trifluormethyl, Trifluormethoxy, Difluormethoxy, Cyano oder Nitro,
- Z: steht ganz besonders bevorzugt für Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro, oder
- Y und Z: stehen ganz besonders bevorzugt für gegebenenfalls durch Fluor, Chlor, Methyl, Ethyl, Propyl, iso-Propyl, Methoxy, Ethoxy, Propoxy, iso-Propoxy oder Trifluormethyl substituiertes C₃-C₄-Alkandiyl, in welchem gegebenenfalls zwei nicht direkt benachbarte Glieder durch Sauerstoff ersetzt sind, wobei X eine der oben genannten Bedeutungen hat.
- Het: steht ganz besonders bevorzugt für eine der Gruppen 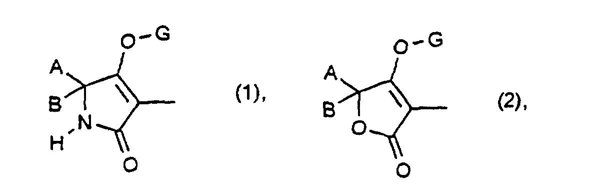 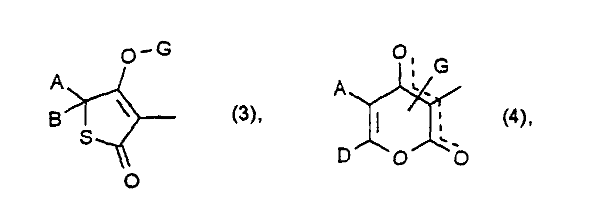 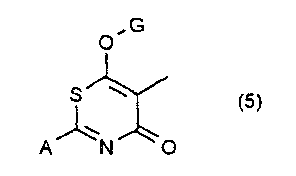
- A: steht ganz besonders bevorzugt für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄-alkyl, Poly-C₁-C₄-alkoxy-C₁-C₄-alkyl oder C₁-C₆-Alkylthio-C₁-C₄-alkyl, oder für gegebenenfalls durch Fluor, Chlor, Methyl oder Methoxy substituiertes C₃-C₆-Cycloalkyl, in welchem gegebenenfalls eine oder zwei Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Pyridyl oder Benzyl.
- B: steht ganz besonders bevorzugt für Wasserstoff, C₁-C₈-Alkyl oder C₁-C₄-Alkoxy-C₁-C₂-alkyl oder
- A, B: und das Kohlenstoffatom an das sie gebunden sind, stehen ganz besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
- A, B: und das Kohlenstoffatom, an das sie gebunden sind, stehen ganz besonders bevorzugt für C₅-C₆-Cycloalkyl, welches durch eine gegebenenfalls ein Sauerstoff- oder Schwefelatom enthaltende Alkylendiyl- oder durch eine Alkylendioxyl-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, stehen ganz besonders bevorzugt für C₃-C₆-Cycloalkyl oder C₅-C₆-Cycloalkenyl, in dem zwei Substituenten gemeinsam mit den Kohlenstoffatomen, an die sie gebunden sind, für C₃-C₄-Alkandiyl, C₃-C₄-Alkendiyl oder Butadiendiyl stehen, worin jeweils gegebenenfalls eine Methylengruppe durch Sauerstoff oder Schwefel ersetzt ist.
- D: steht ganz besonders bevorzugt für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl,
oder
- A und D: stehen gemeinsam ganz besonders bevorzugt für eine C₃-C₅-Alkandiyloder C₃-C₅-Alkendiylgruppe, worin jeweils gegebenenfalls eine Methylengruppe durch Sauerstoff oder Schwefel ersetzt ist und welche gegebenenfalls durch Fluor, Chlor oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy substituiert sind.
- G: steht ganz besonders bevorzugt für Wasserstoff (a) oder für eine der Gruppen 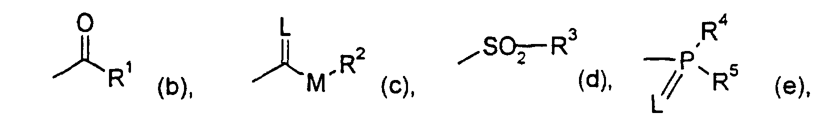 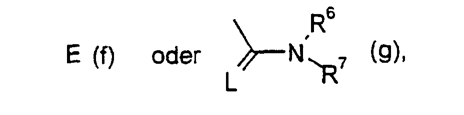 in welchen
E für ein Metallionäquivalent oder ein Ammoniumion steht,
L für Sauerstoff oder Schwefel steht und
M für Sauerstoff oder Schwefel steht.
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R³: steht ganz besonders bevorzugt für gegebenenfalls durch Fluor oder Chlor substituiertes Methyl, Ethyl, Propyl, iso-Propyl, Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl.
- R⁴ und R⁵: stehen unabhängig voneinander ganz besonders bevorzugt für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

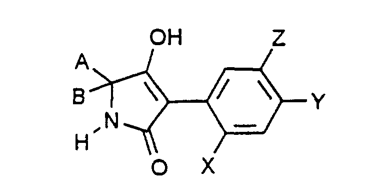

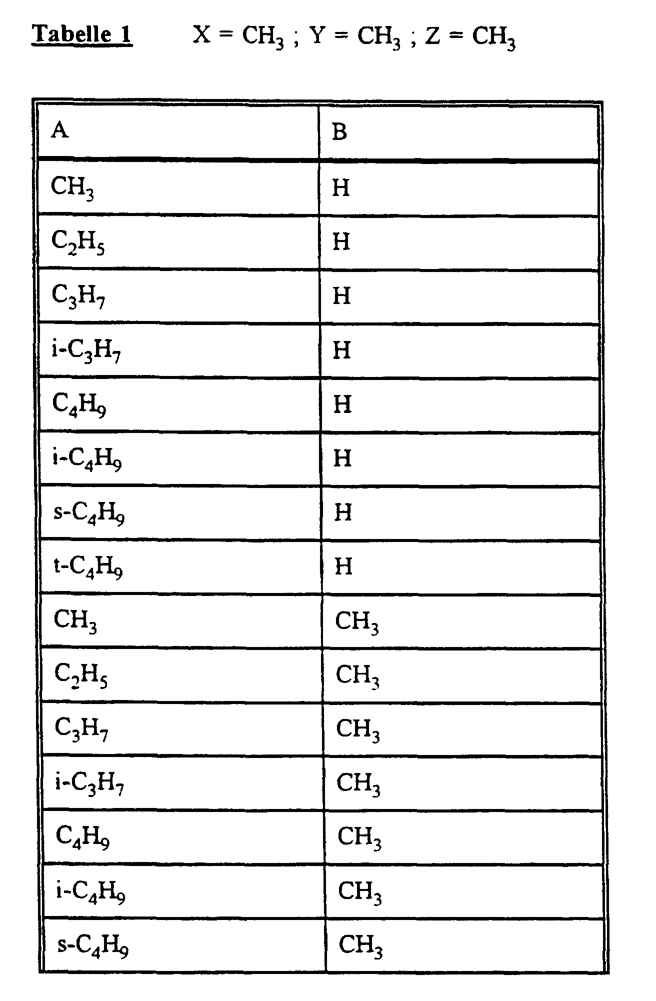

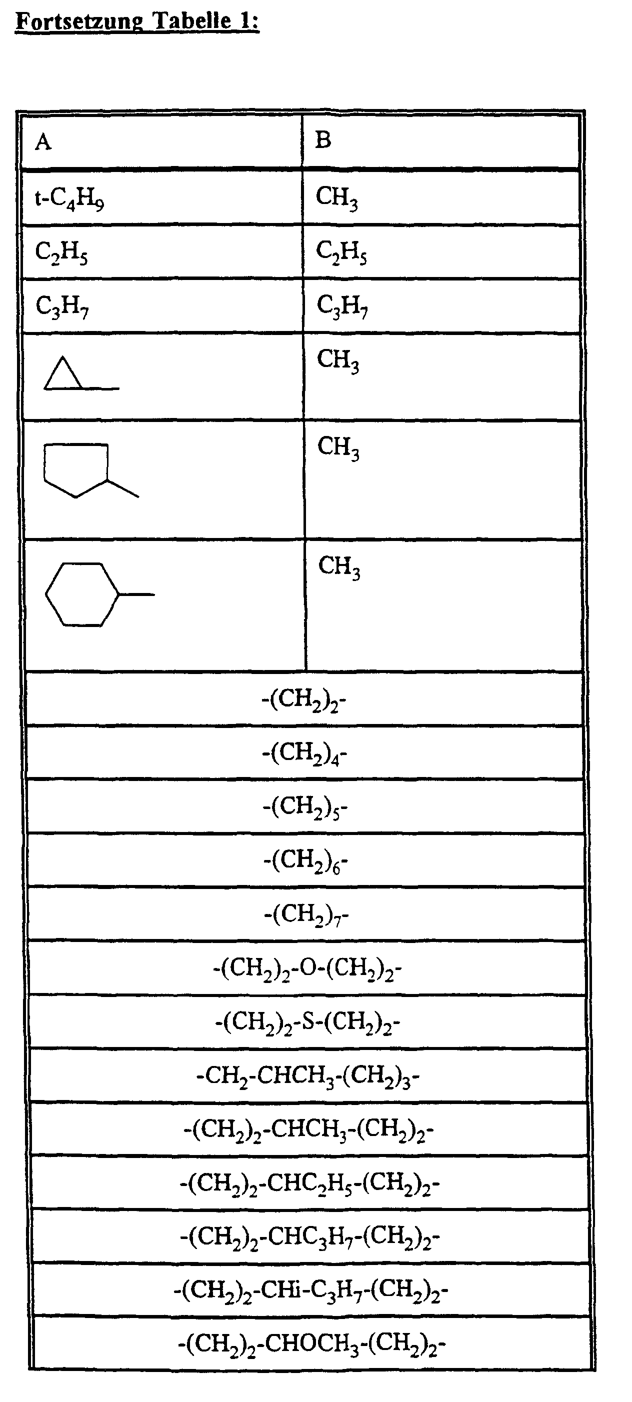

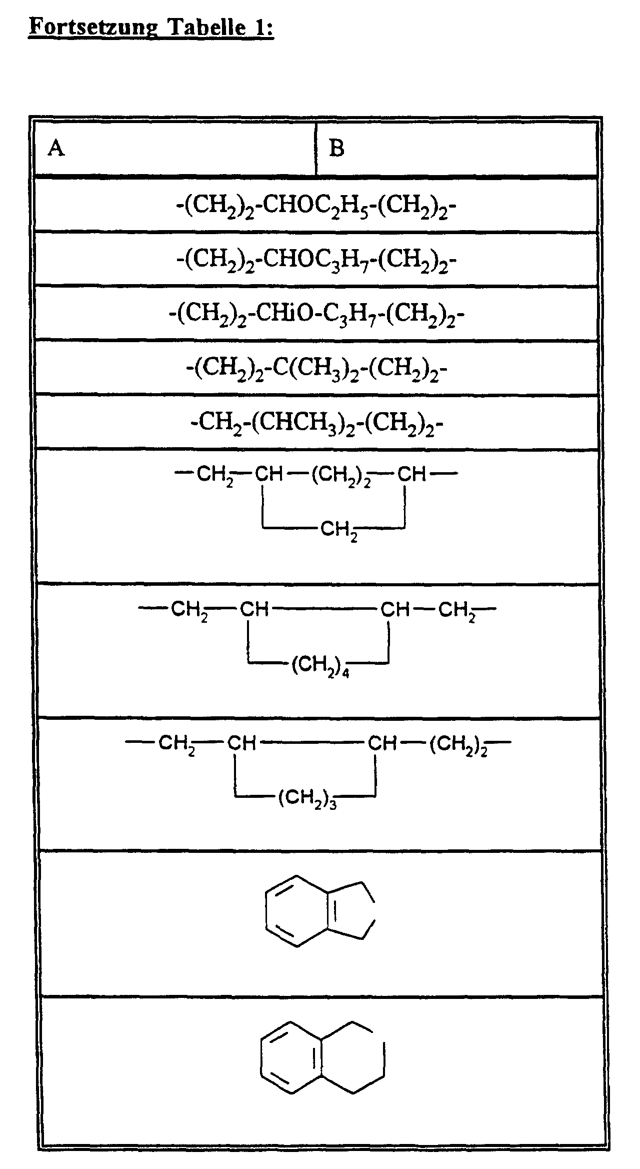

Tabelle 2: A und B haben die gleiche Bedeutung wie in Tabelle 1 mit X = CH₃ ; Y = Cl ; Z = CH₃

Tabelle 3: A und B haben die gleiche Bedeutung wie in Tabelle 1 mit X = Cl ; Y = Cl ; Z = Cl

Im einzelnen seien außer den bei den Herstellungsbeispielen genannten Verbindungen die folgenden Verbindungen der Formel (I-2-a) genannt:

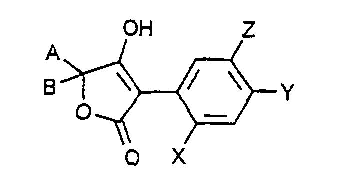

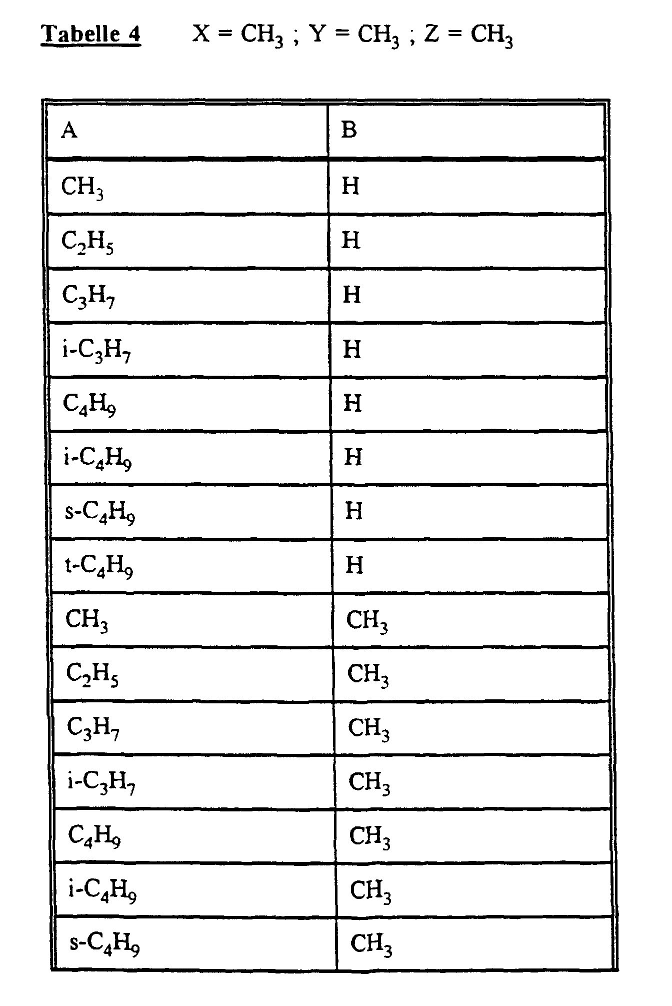

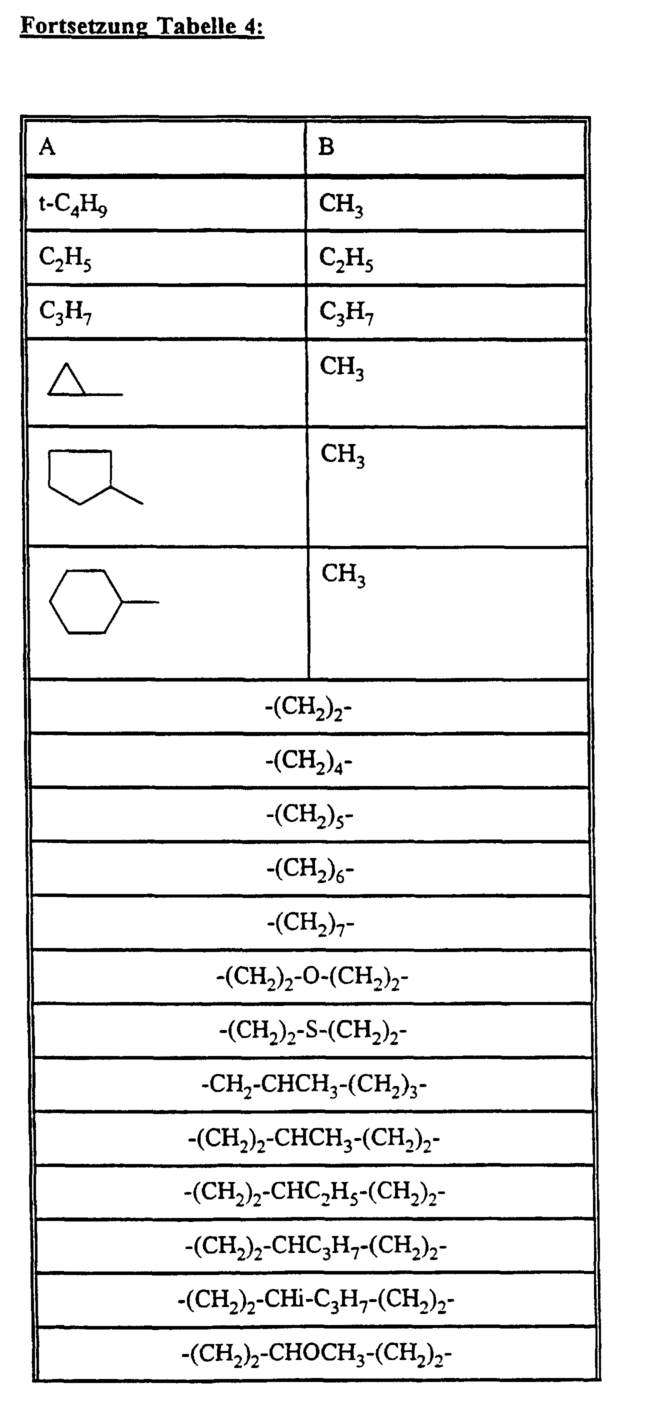

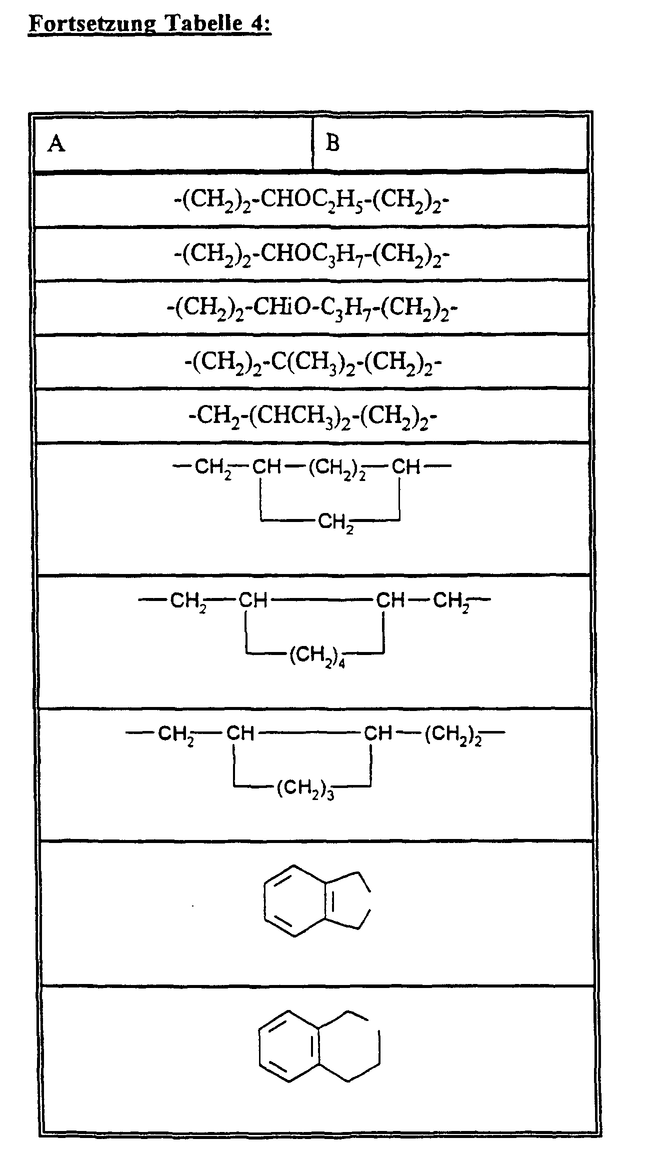

Tabelle 5: A und B haben die gleiche Bedeutung wie in Tabelle 4 mit X = CH₃ ; Y = Cl ; Z = CH₃

Tabelle 6: A und B haben die gleiche Bedeutung wie in Tabelle 4 mit X = Cl ; Y = Cl ; Z = Cl

Im einzelnen seien außer den bei den Herstellungsbeispielen genannten Verbindungen die folgenden Verbindungen der Formel (I-3-a) genannt:

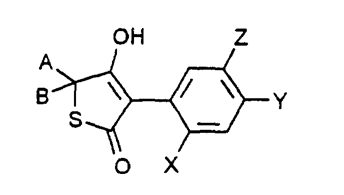

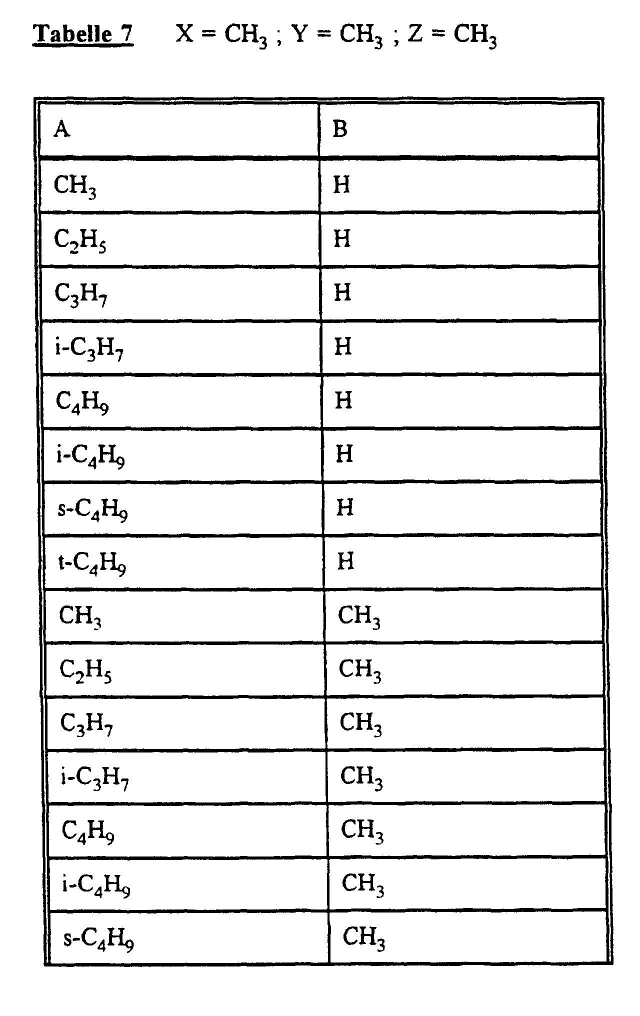

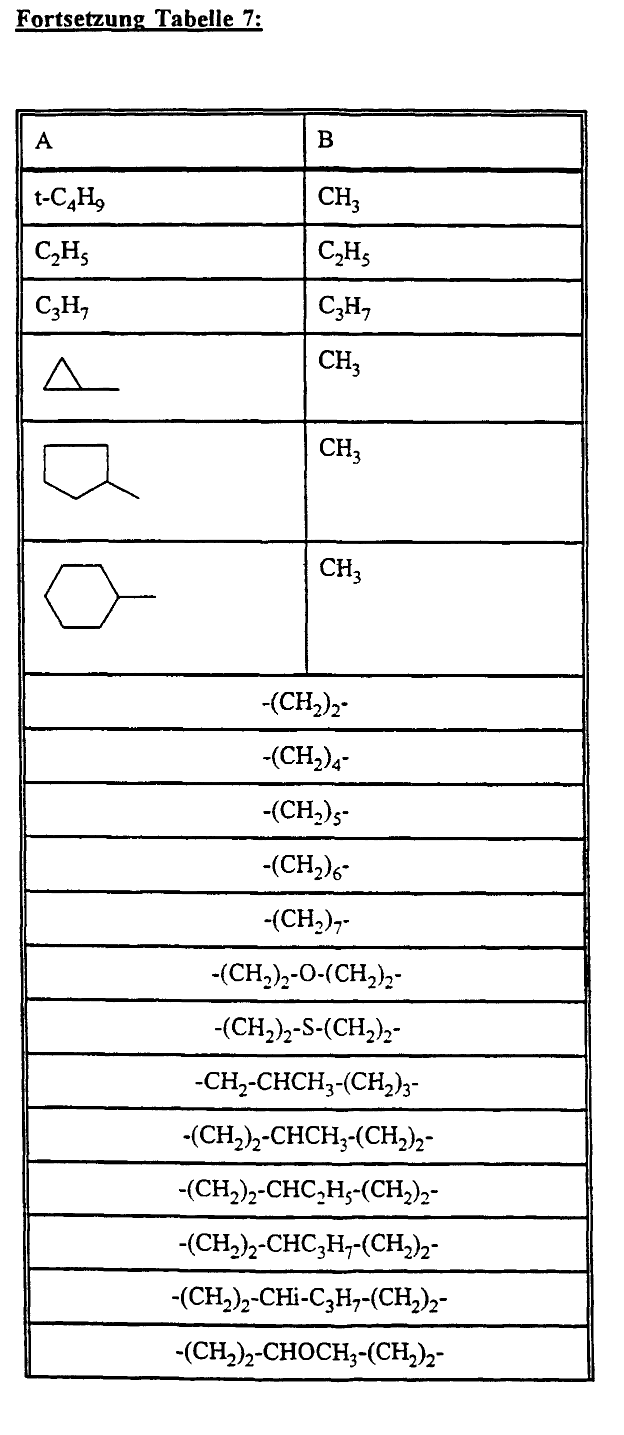

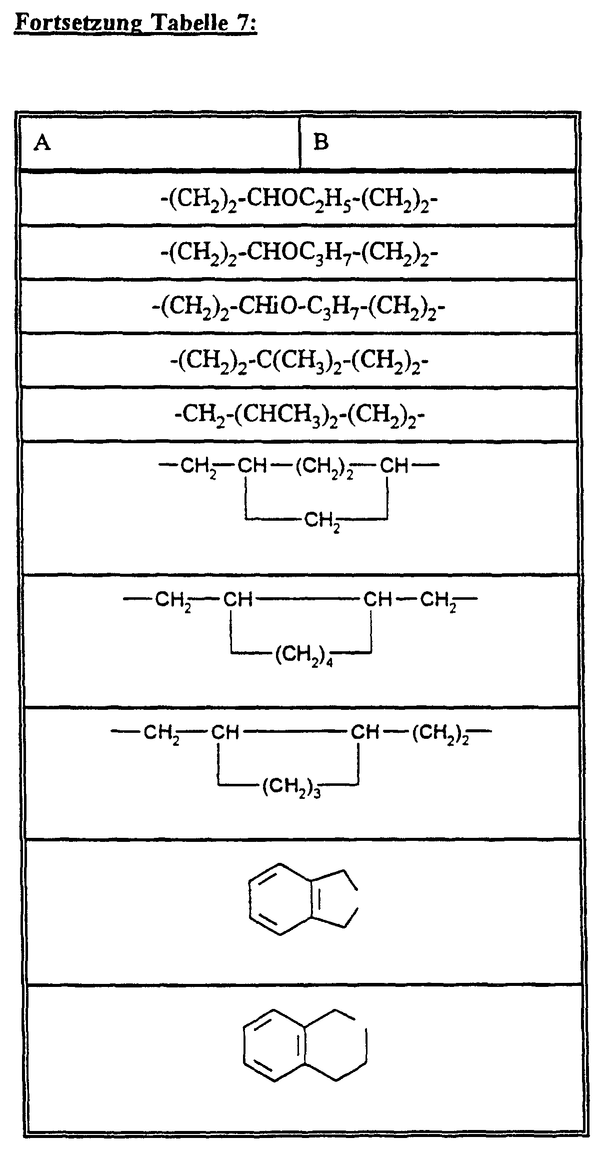

Tabelle 8: A und B haben die gleiche Bedeutung wie in Tabelle 7 mit X = CH₃ ; Y = Cl ; Z = CH₃

Tabelle 9: A und B haben die gleiche Bedeutung wie in Tabelle 7 mit X = Cl ; Y = Cl ; Z = Cl

Im einzelnen seien außer den bei den Herstellungsbeispielen genannten Verbindungen die folgenden Verbindungen der Formel (I-4-a) genannt:

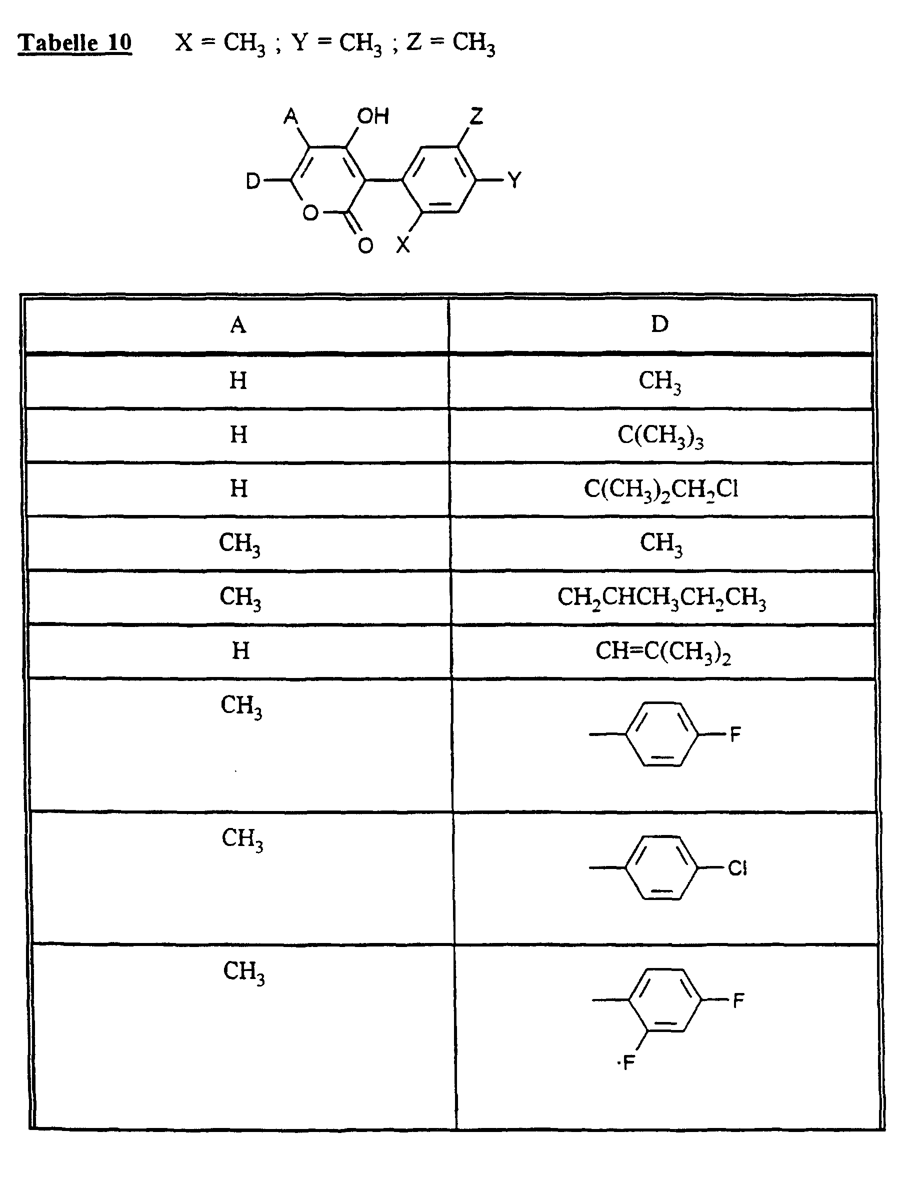

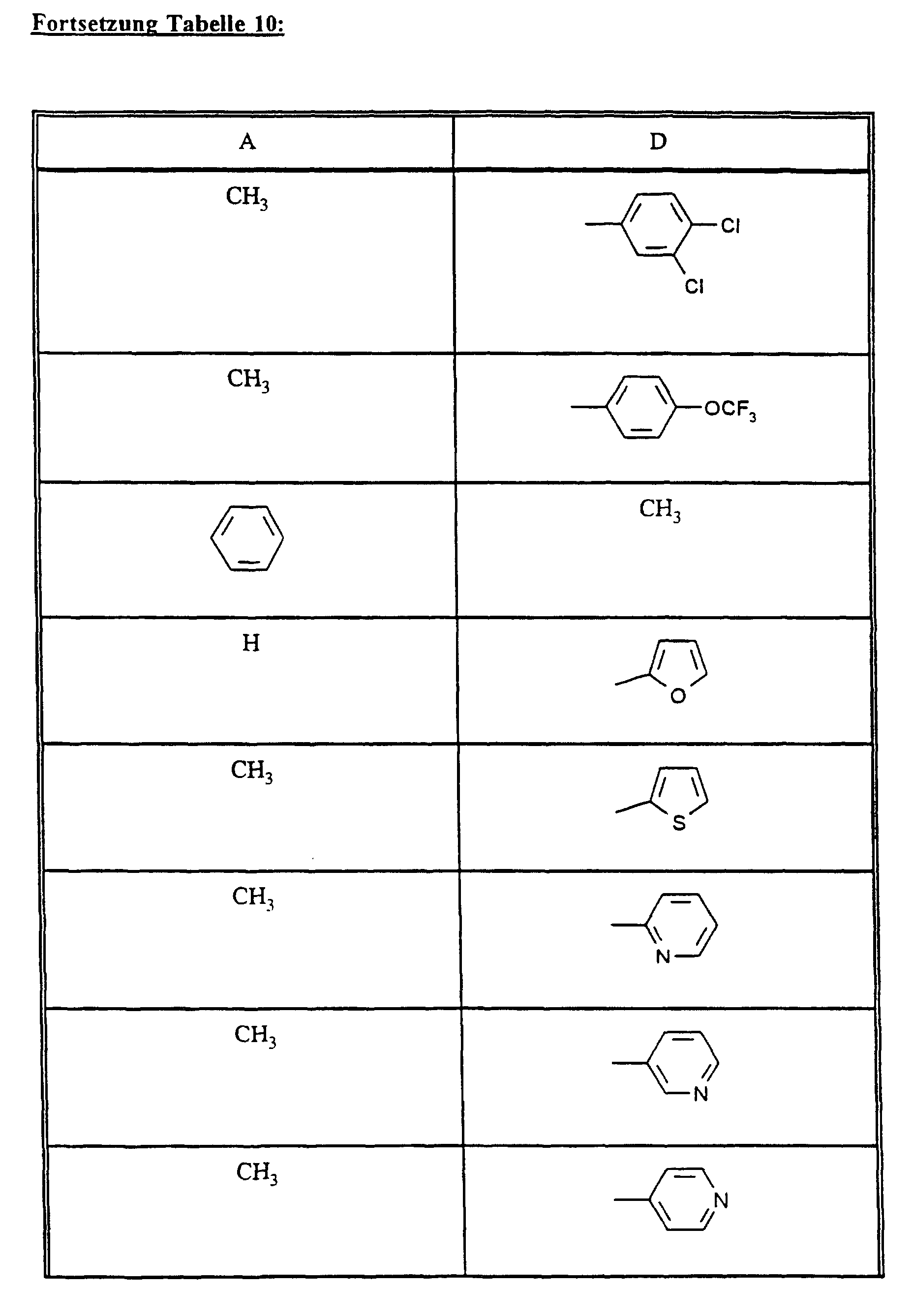

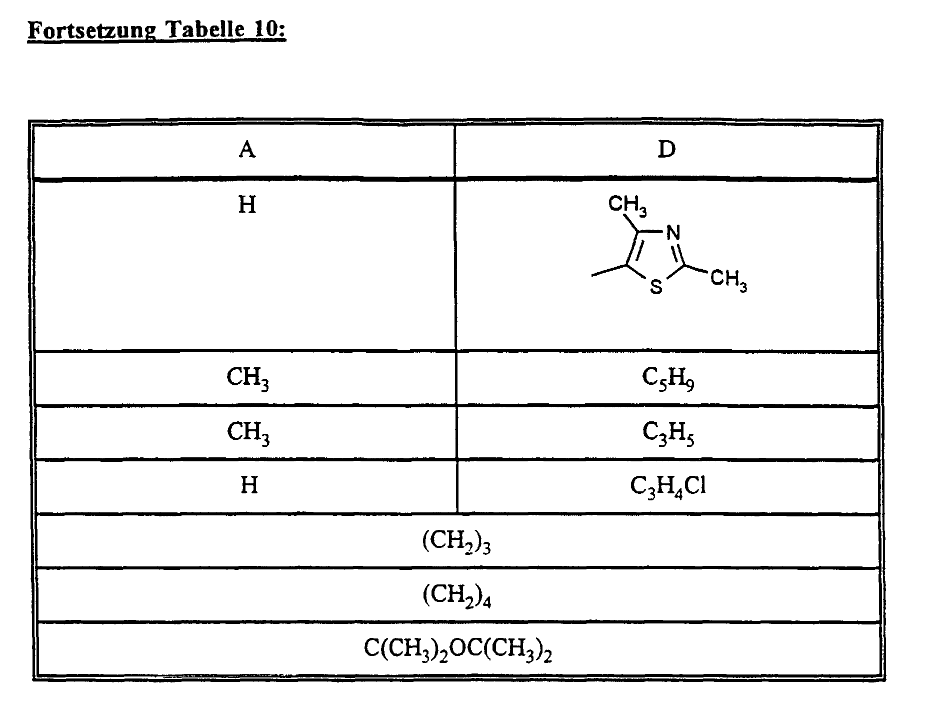

Tabelle 11: A und D haben die gleiche Bedeutung wie in Tabelle 10 mit X = CH₃ ; Y = Cl ; Z = CH₃

Tabelle 12: A und D haben die gleiche Bedeutung wie in Tabelle 10 mit X = Cl ; Y = Cl ; Z = Cl

Im einzelnen seien außer den bei den Herstellungsbeispielen genannten Verbindungen die folgenden Verbindungen der Formel (I-5-a) genannt:

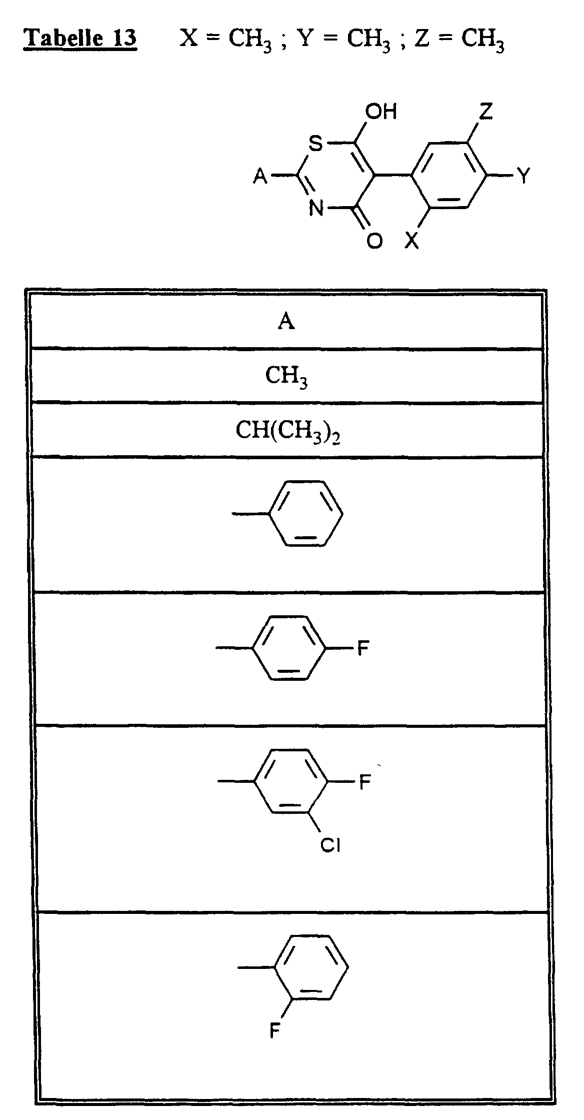

Tabelle 14: A hat die gleiche Bedeutung wie in Tabelle 13 mit X = CH₃ ; Y = Cl ; Z = CH₃

Tabelle 15: A hat die gleiche Bedeutung wie in Tabelle 13 mit X = Cl ; Y = Cl ; Z = Cl

Verwendet man gemäß Verfahren (A) N-[(4,5-Dichlor-2-methyl)-phenylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

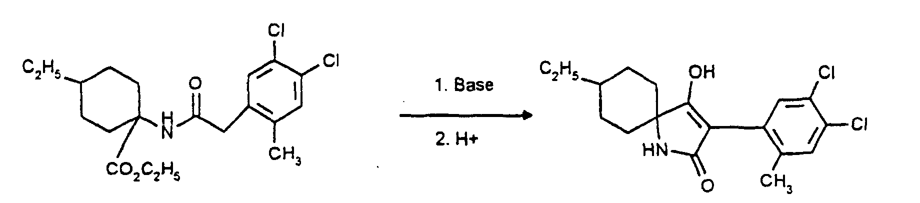

Verwendet man gemäß Verfahren (B) O-[(2,5-Dichlor-4-methyl)-phenylacetyl]-hydroxyessigsäureethylester, so kann der Verlauf des erfindungsgemäßen Verfahrens durch folgendes Reaktionsschema wiedergegeben werden:

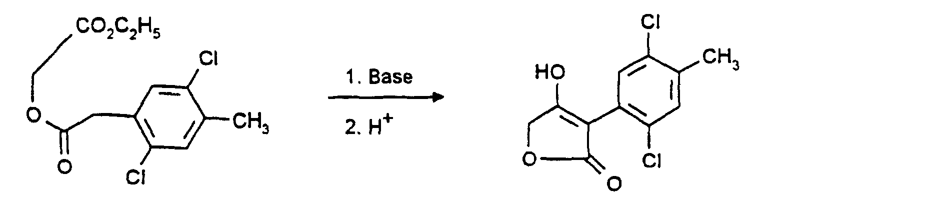

Verwendet man gemäß Verfahren (C) 2-[(2-Chlor-4,5-dimethyl)-phenyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

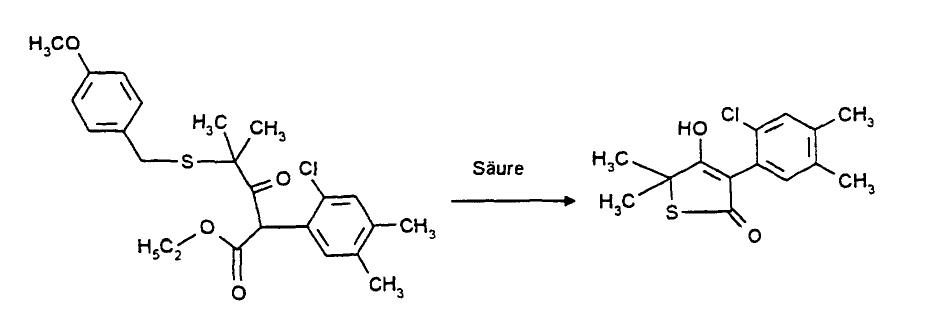

Verwendet man beispielsweise gemäß Verfahren (D) (Chlorcarbonyl)-2-[(4,5-Dichlor-2-methyl)-phenyl]-keten und Aceton als Ausgangsverbindungen, so kann der Verlauf des erfindungsgemäßen Verfahrens durch das folgende Reaktionsschema wiedergegeben werden:

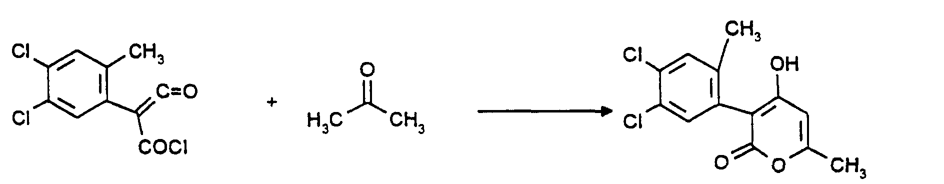

Verwendet man beispielsweise gemäß Verfahren (E) (Chlorcarbonyl)-2-[(2,4,5-Trimethyl)-phenyl]-keten und Thiobenzamid als Ausgangsverbindungen, so kann der Verlauf des erfindungsgemäßen Verfahrens durch das folgende Reaktionsschema wiedergegeben werden:

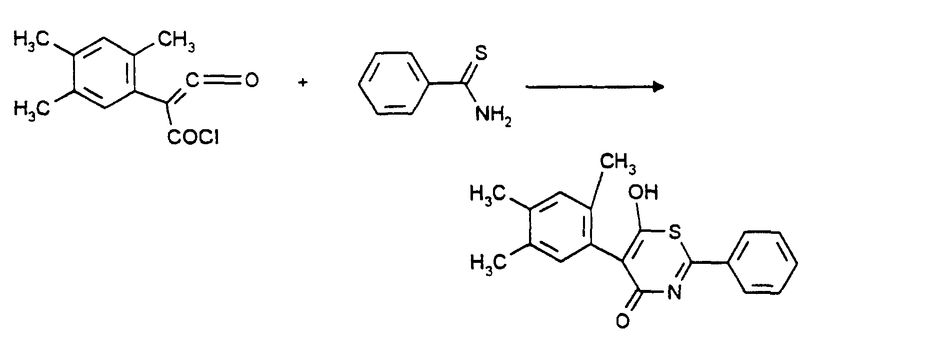

Verwendet man gemäß Verfahren (Fα) 3-[(2,5-Dichlor-4-methyl)-phenyl]-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

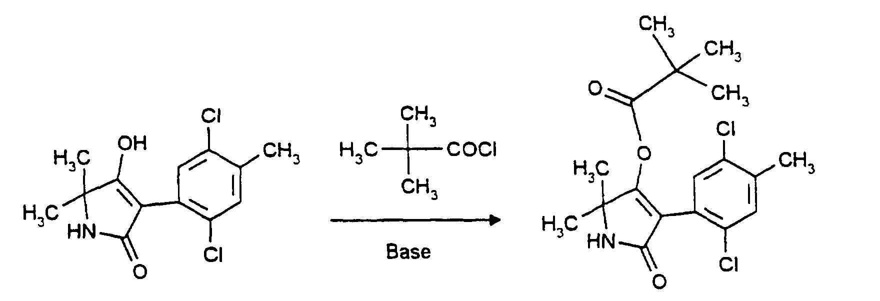

Verwendet man gemäß Verfahren (F) (Variante β) 3-[(2,4,5-Trichlor)-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

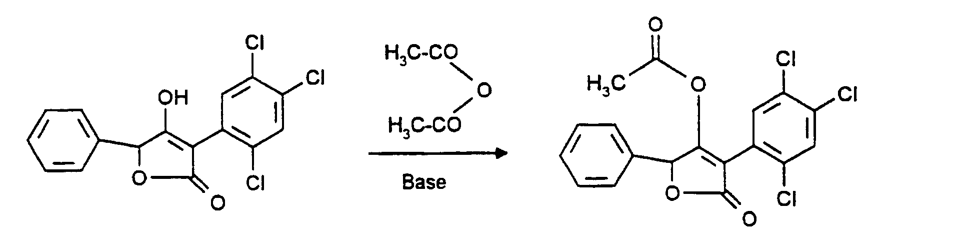

Verwendet man gemäß Verfahren (G) 8-[(2,4-Dichlor-5-methyl)-phenyl]-5,5-pentamethylen-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

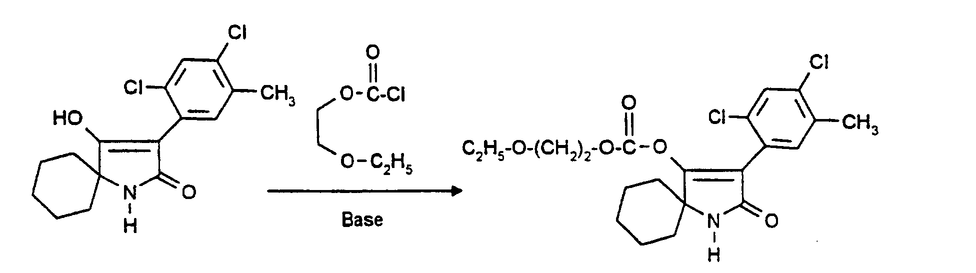

Verwendet man gemäß Verfahren (H), (Variante α) 3-[(2-Brom-4,5-dimethyl)-phenyl]-4-hydroxy-6-(3-pyridyl)-pyron und Chlormonothioameisensäuremethylester als Ausgangsprodukte, so kann der Reaktionsverlauf folgendermaßen wiedergegeben werden:

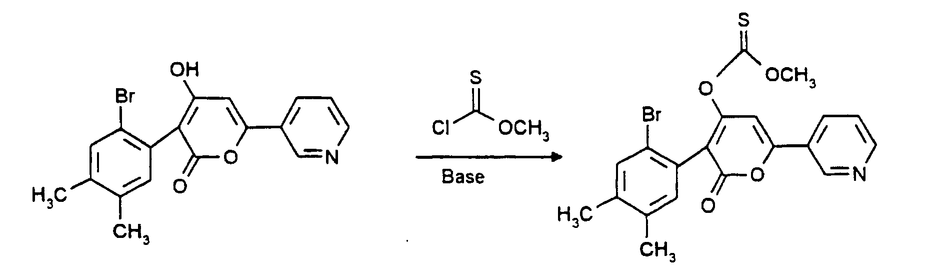

Verwendet man gemäß Verfahren (H), (Variante β) 5-[(5-Chlor-2-fluor-4-methyl)-phenyl]-6-hydroxy-2-(4-chlorphenyl)-thiazin-4-on, Schwefelkohlenstoff und Methyliodid als Ausgangskomponenten, so kann der Reaktionsverlauf wie folgt wiedergegeben werden:

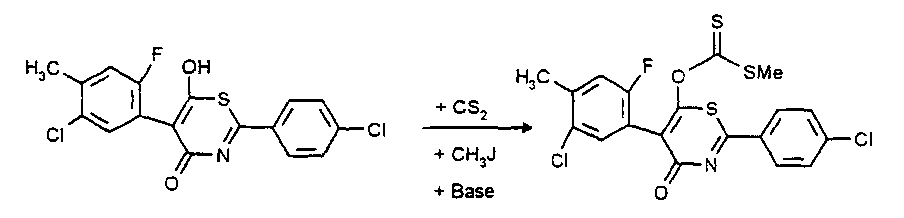

Verwendet man gemäß Verfahren (I) 2-[(2,4,5-Trimethyl)-phenyl]-5,5[-(3-methyl)-pentamethylen]-pyrrolidin-2,4-dion und Methansulfonsäurechlorid als Ausgangsprodukt, so kann der Reaktionsverlauf durch folgendes Reaktionsschema wiedergegeben werden:

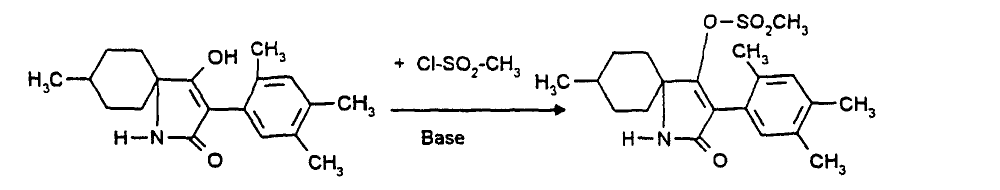

Verwendet man gemäß Verfahren (J) 2-[(2-Chlor-4,5-dimethyl)-phenyl]-4-hydroxy-5-methyl-6-(2-pyridyl)-pyron und Methanthio-phosphonsäurechlorid-(2,2,2-trifluorethylester) als Ausgangsprodukte, so kann der Reaktionsverlauf durch folgendes Reaktionsschema wiedergegeben werden:

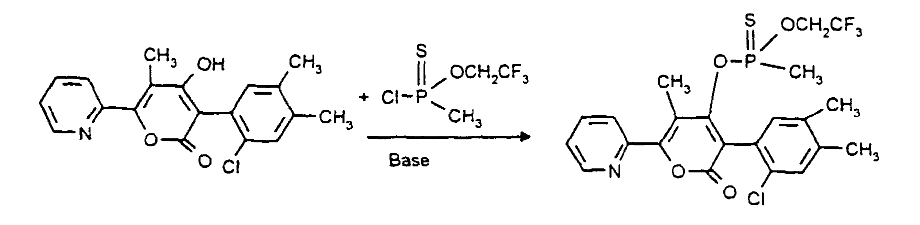

Verwendet man gemäß Verfahren (K) 3-[(2,4,5-Trichlor)-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

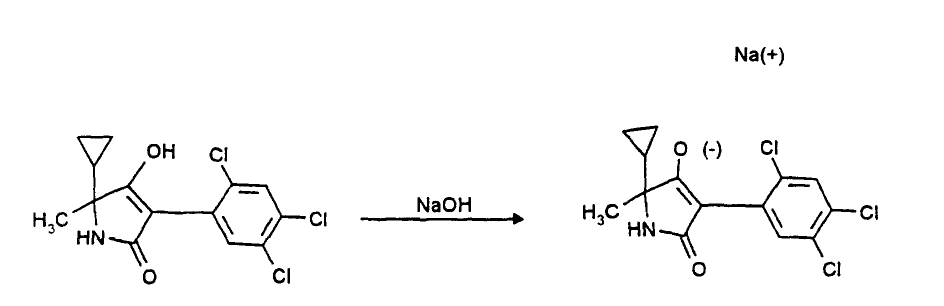

Verwendet man gemäß Verfahren (L) (Variante α) 3-[(2-Chlor-4-brom-5-methyl)-phenyl]-4-hydroxy-5,5-tetramethylen-Δ³-dihydro-furan-2-on und Ethylisocyanat als Ausgangsprodukte, so kann der Reaktionsverlauf durch folgendes Reaktionsschema wiedergegeben werden:

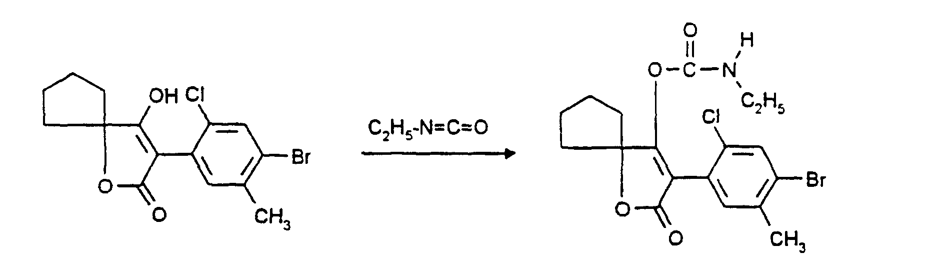

Verwendet man gemäß Verfahren (L) (Variante β) 3-[(2-Chlor-4,5-dimethyl)-phenyl]-5-methyl-pyrrolidin-2,4-dion und Dimethylcarbamidsaurechlorid als Ausgangsprodukte, so kann der Reaktionsverlauf durch folgendes Schema wiedergegeben werden:

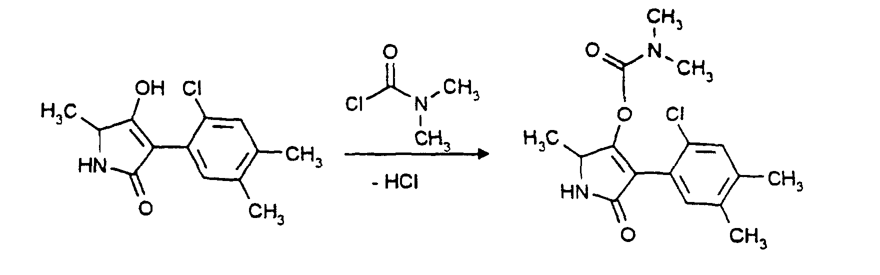

Die beim erfindungsgemäßen Verfahren (A) als Ausgangsstoffe benötigten Verbindungen der Formel (II) 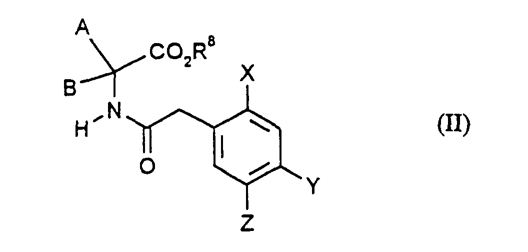 in welcher
- A, B, X, Y, Z und R⁸: die oben angegebene Bedeutungen haben,
sind neu.

Man erhält die Acylaminosäureester der Formel (II) beispielsweise, wenn man Aminosäurederivate der Formel (XIX) 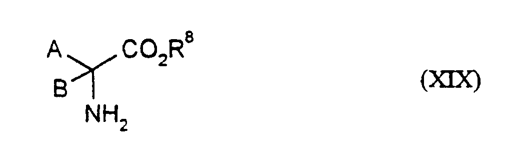 in welcher
- A, B und R⁸: die oben angegebenen Bedeutungen haben,
mit substituierten Phenylessigsäurehalogeniden der Formel (XX) 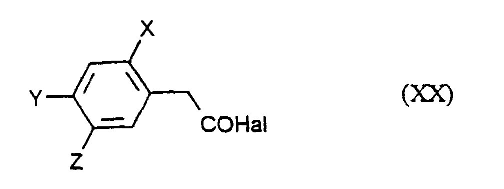 in welcher
- X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXI) 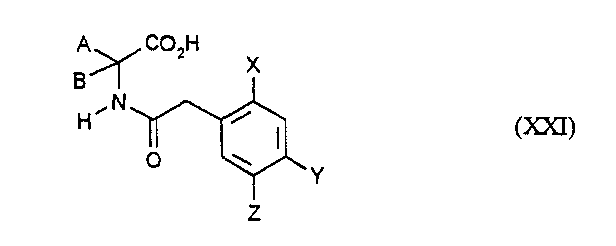 in welcher
- A, B, X, Y und Z: die oben angegebenen Bedeutungen haben,
verestert (Chem. Ind. (London) 1568 (1968)).

Die Verbindungen der Formel (XXI) 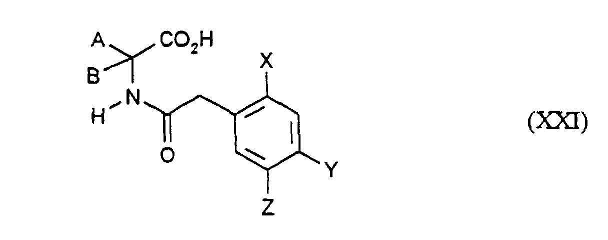 in welcher
- A, B, X, Y und Z: die oben angegebenen Bedeutungen haben,
sind neu.

Man erhält die Verbindungen der Formel (XXI), wenn man Aminosäuren der Formel (XXII) 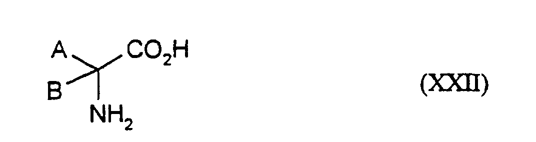 in welcher
- A und B: die oben angegebenen Bedeutungen haben,
mit substituierten Phenylessigsäurehalogeniden der Formel (XX) 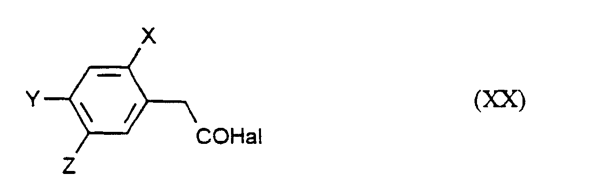 in welcher
- X, Y und Z: die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XX) sind teilweise neu und lassen sich nach bekannten Verfahren herstellen.

Man erhält die Verbindungen der Formel (XX) beispielsweise, indem man substituierte Phenylessigsäuren der Formel (XXIII) 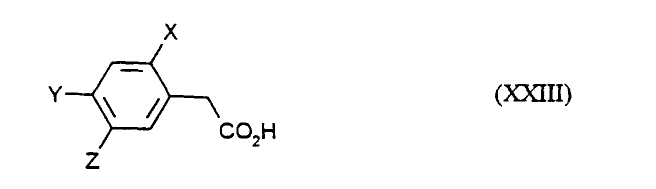 in welcher
- X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXIII) sind teilweise neu, sie lassen sich nach literaturbekannten Verfahren herstellen (Organikum 15. Auflage, S. 533, VEB Deutscher Verlag der Wissenschaften, Berlin 1977). Man erhält die Verbindungen der Formel (XXIII) beispielsweise, indem man substituierte Phenylessigsäureester der Formel (XXIV) 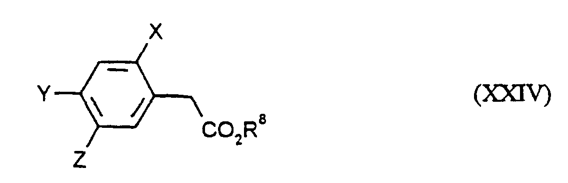 in welcher
- X, Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXIV) sind teilweise neu, sie lassen sich nach im Prinzip bekannten Verfahren herstellen.

Man erhält die Verbindungen der Formel (XXIV) beispielsweise, indem man substituierte 1,1,1-Trichlor-2-phenylethane der Formel (XXV) 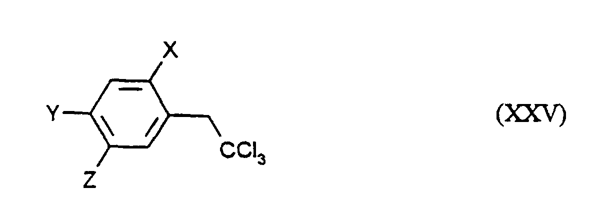 in welcher
- X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE 3 314 249).

Die Verbindungen der Formel (XXV) sind teilweise neu, sie lassen sich nach im Prinzip bekannten Verfahren herstellen.

Man erhält die Verbindungen der Formel (XXV) beispielsweise, wenn man Aniline der Formel (XXVI) 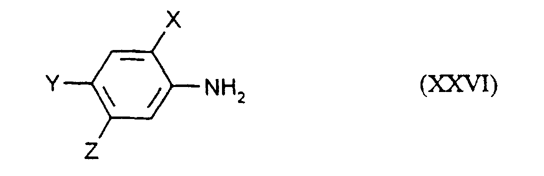 in welcher
- X, Y und Z: die oben angegebene Bedeutung haben,
in Gegenwart eines Alkylnitrits der Formel (XXVII)

R²¹-ONO (XXVII)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XXVI) und (XXVII) sind bekannte Verbindungen der Organischen Chemie. Kupfer(II)-chlorid und Vinylidenchlorid sind lange bekannt und käuflich.

Die Verbindungen der Formel (XIX) und (XXII) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 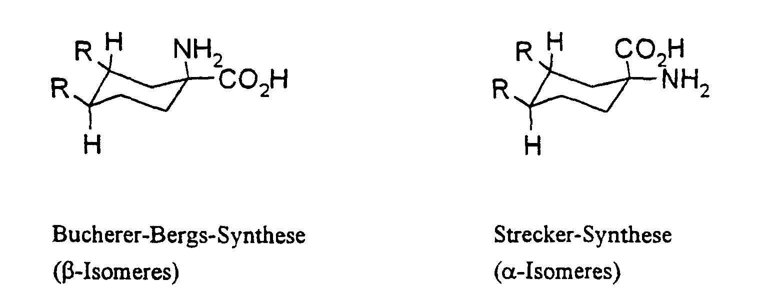 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 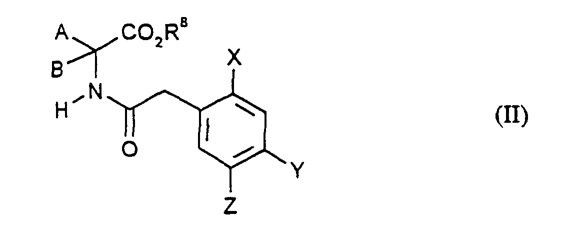 in welcher
- A, B, X, Y, Z und R⁸: die oben angegebenen Bedeutungen haben,
herstellen, wenn man Aminonitrile der Formel (XXVIII) 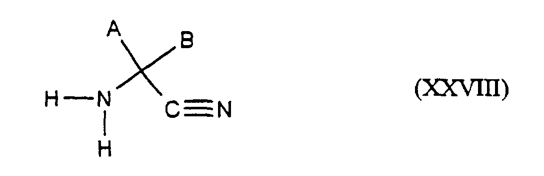 in welcher
- A und B: die oben angegebenen Bedeutungen haben,
mit substituierten Phenylessigsäurehalogeniden der Formel (XX) 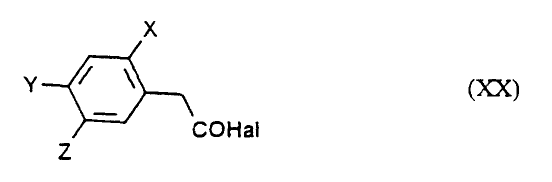 in welcher
- X, Y, Z und Hal: die oben angegebenen Bedeutungen haben,
zu Verbindungen der Formel (XXIX) 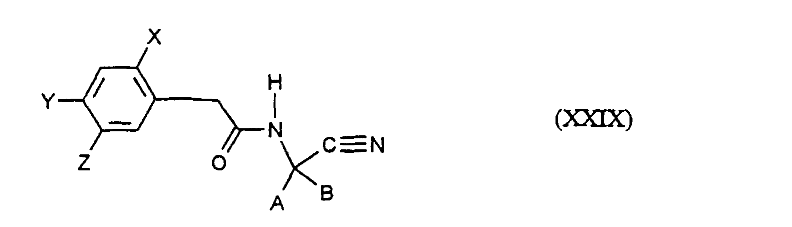 in welcher
- A, B, X, Y und Z: die oben angegebenen Bedeutungen haben,
umsetzt, und diese anschließend einer sauren Alkoholyse unterwirft.

Die Verbindungen der Formel (XXIX) sind ebenfalls neu.

Die bei dem erfindungsgemäßen Verfahren (B) als Ausgangstoffe benötigten Verbindungen der Formel (III) 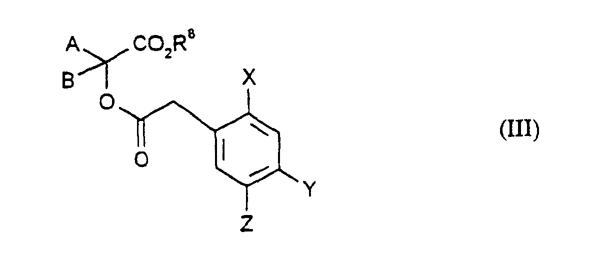 in welcher
- A, B, X, Y, Z und R⁸: die oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man
2-Hydroxycarbonsäureester der Formel (XXX) 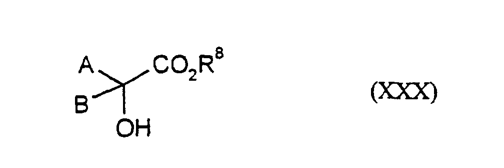 in welcher
- A, B und R⁸: die oben angegebenen Bedeutungen haben,
mit substituierten Phenylessigsäurehalogeniden der Formel (XX) 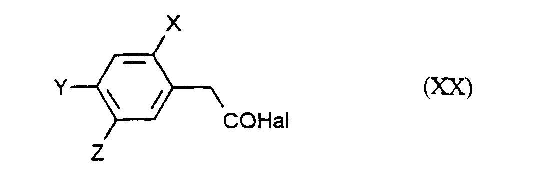 in welcher
- X, Y, Z und Hal: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXIII) 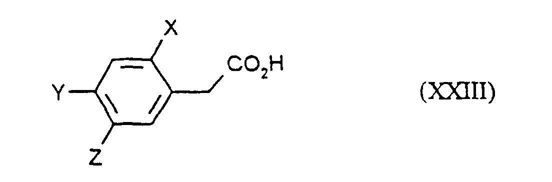 in welcher
- X, Y und Z: die oben angegebenen Bedeutungen haben,
mit α-Halogencarbonsäureestern der Formel (XXXI) 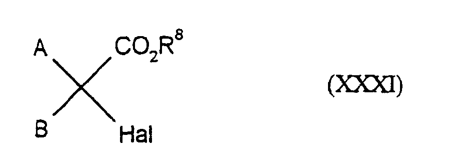 in welcher
- A, B und R⁸: die oben angegebenen Bedeutungen haben und
- Hal: für Chlor oder Brom steht,
alkyliert.

Die Verbindungen der Formel (XXXI) sind käuflich.

Die bei dem obigen Verfahren (C) als Ausgangsstoffe benötigten Verbindungen der Formel (IV) 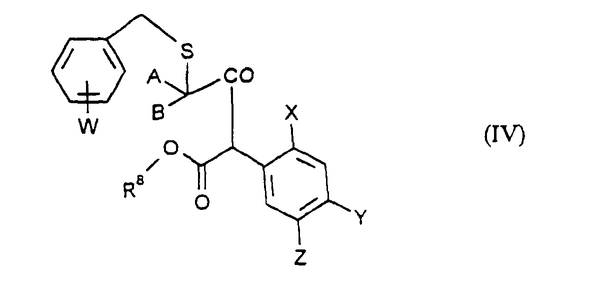 in welcher
- A, B, W, X, Y, Z und R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man
substituierte Phenylessigsäureester der Formel (XXIV) 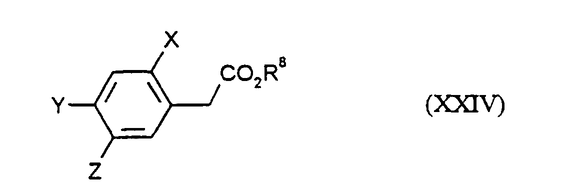 in welcher
- X, Y, R⁸ und Z: die oben angegebenen Bedeutungen haben,
mit 2-Benzylthio-carbonsäurehalogeniden der Formel (XXXII) 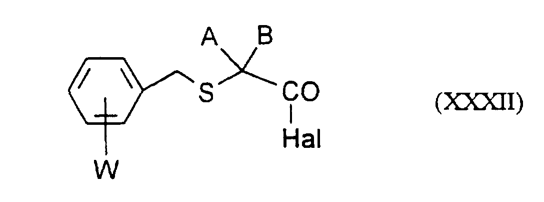 in welcher
- A, B und W: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsaurehalogenide der Formel (XXXII) sind teilweise bekannt und/oder lassen sich nach bekannten Verfahren herstellen (J. Antibiotics (1983), 26, 1589).

Die beim Verfahren (D) als Ausgangsstoffe benötigten Halogencarbonylketene der Formel (VI) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). Man erhält die Verbindungen der Formel (VI) 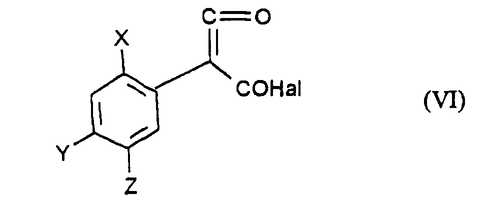 in welcher
- X, Y und Z: die oben angegebenen Bedeutungen haben und
- Hal: für Chlor oder Brom steht,
wenn man
substituierte Phenylmalonsäuren der Formel (XXXIII) 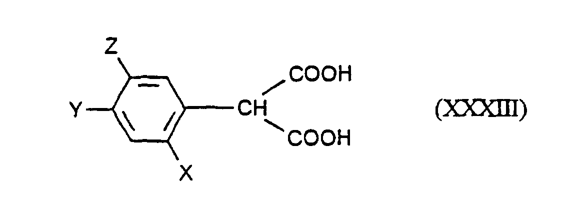 in welcher
- X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Stearylformamid oder Triphenylphosphin und gegebenenfalls in Gegenwart von Basen wie z.B. Pyridin oder Triethylamin, bei einer Temperatur zwischen -20°C und 200°C, bevorzugt zwischen 0°C und 150°C, umsetzt.

Die substituierten Phenylmalonsäuren der Formel (XXXIII) sind neu. Sie lassen sich aber in einfacher Weise nach bekannten Verfahren herstellen (vgl. z.B. Organikum, VEB Deutscher Verlag der Wissenschaften, Berlin 1977, S. 517 ff), z.B. aus substituierten Phenylmalonsäureestern der Formel (XXXIV) 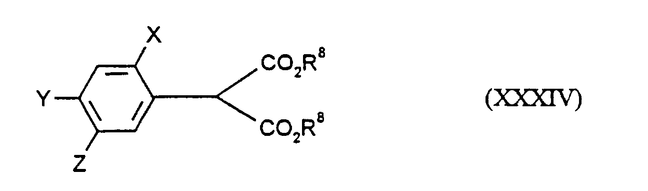 in welcher
- X, Y, Z und R⁸: die oben angegebene Bedeutung haben,
durch Verseifung erhalten.

Die für das erfindungsgemäße Verfahren (E) als Ausgangsstoffe benötigten Carbonylverbindungen der Formel (V) oder deren Silylenolether der Formel (Va) 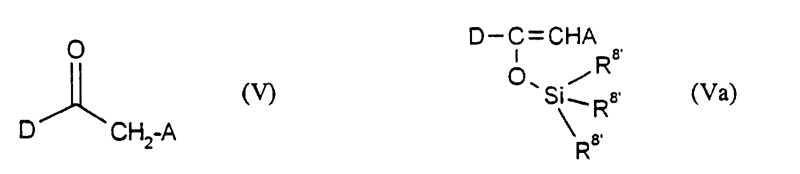 in welchen
- A, D und R^{8'}: die oben angegebenen Bedeutungen haben,
sind käufliche, allgemeine bekannte oder nach bekannten Verfahren zugangliche Verbindungen.

Die Herstellung der zur Durchführung des erfindungsgemäßen Verfahrens (E) als Ausgangsstoffe benötigten Ketensaurechloride der Formel (VI) wurden bereits beim erfindungsgemäßen Verfahren (D) beschrieben. Die zur Durchführung des erfindungsgemäßen Verfahrens (E) benötigten Thioamide der Formel (VII) 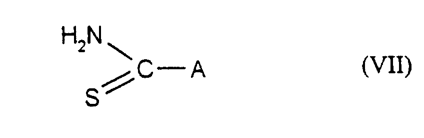 in welcher
- A: die oben angegebene Bedeutung hat,
sind allgemein in der Organischen Chemie bekannte Verbindungen.

Die Malonsäureester der Formel (XXXIV) 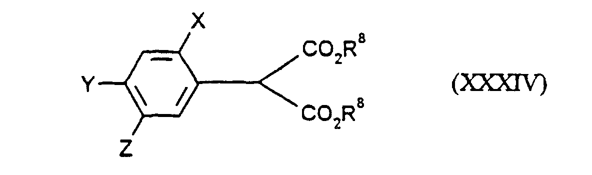 in welcher
- R⁸, X, Y und Z: die oben angegebenen Bedeutungen haben,
sind neu und lassen sich nach allgemein bekannten Methoden der Organischen Chemie darstellen (vgl. z.B. Tetrahedron Lett. 27, 2763 (1986) und Organikum VEB Deutscher Verlag der Wissenschaften, Berlin 1977, S. 587 ff.).

Die zur Durchführung der erfindungsgemäßen Verfahren (F), (G), (H), (I), (J), (K) und (L) außerdem als Ausgangsstoffe benötigten Säurehalogenide der Formel (VIII), Carbonsäureanhydride der Formel (IX), Chlorameisensäureester oder Chlorameisensäurethioester der Formel (X), Chlormonothioameisensäureester oder Chlordithioameisensäureester der Formel (XI), Alkylhalogenide der Formel (XII), Sulfonsäurechloride der Formel (XIII), Phosphorverbindungen der Formel (XIV) und Metallhydroxide, Metallalkoxide oder Amine der Formel (XV) und (XVI) und Isocyanate der Formel (XVII) und Carbamidsäurechloride der Formel (XVIII) sind allgemein bekannte Verbindungen der organischen bzw. anorganischen Chemie.

Die Verbindungen der Formeln (V), (VII) bis (XVIII), (XIX), (XXII), (XXVIII), (XXX), (XXXI), (XXXII), (XXXIII) und (XXXIV) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemaße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß man Verbindungen der Formel (III), in welcher A, B, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle gegenüber den Reaktionsteilnehmern inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden, wie z.B. Halogenwasserstoffsäuren, Schwefelsäure, Alkyl-, Aryl- und Haloalkylsulfonsäuren, insbesondere werden halogenierte Alkylcarbonsäuren wie z.B. Trifluoressigsäure verwendet.

Die Reaktionstemperatur kann bei der Durchführung des erfindungsgemäßen Verfahrens (C) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure in katalytischen Mengen einzusetzen.

Das erfindungsgemäße Verfahren (D) ist dadurch gekennzeichnet, daß man Carbonylverbindungen der Formel (V) oder deren Silylenolether der Formel (Va) mit Ketensäurehalogeniden der Formel (VI) gegebenenfalls in Gegenwart eines Verdünnungsmittels und gegebenenfalls in Gegenwart eines Säureakzeptors umsetzt.

Als Verdünnungsmittel können bei dem erfindungsgemäßen Verfahren (D) alle gegenüber den Reaktionsteilnehmern inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung des erfindungsgemäßen Verfahrens (D) alle üblichen Säureakzeptoren verwendet werden.

Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base oder N,N-Dimethyl-anilin.

Die Reaktionstemperatur kann bei der Durchführung des erfindungsgemäßen Verfahrens (D) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (D) wird vorzugsweise unter Normaldruck durchgeführt.

Bei der Durchführung des erfindungsgemäßen Verfahrens (D) setzt man die Reaktionskomponenten der Formeln (V) und (VI) und gegebenenfalls den Säureakzeptor im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (E) ist dadurch gekennzeichnet, daß man Thioamide der Formel (VII) mit Ketensäurehalogeniden der Formel (VI) gegebenenfalls in Gegenwart eines Verdünnungsmittels und gegebenenfalls in Gegenwart eines Säureakzeptors umsetzt.

Als Verdünnungsmittel können bei der erfindungsgemäßen Verfahrensvariante (E) alle inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperatur kann bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VII) und (VI) und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (Fα) ist dadurch gekennzeichnet, daß man Verbindungen der Formeln (I-1-a) bis (I-5-a) jeweils mit Carbonsäurehalogeniden der Formel (VIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Fα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Fα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fα) werden die Ausgangsstoffe der Formeln (I-1-a) bis (I-5-a) und das Carbonsäurehalogenid der Formel (VIII) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Fß) ist dadurch gekennzeichnet, daß man Verbindungen der Formeln (I-1-a) bis (I-5-a) jeweils mit Carbonsäureanhydriden der Formel (IX) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fß) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Fβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Fβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fß) werden die Ausgangsstoffe der Formeln (I-1-a) bis (I-5-a) und das Carbonsäureanhydrid der Formel (IX) im allgemeinen in jeweils angenähert äquivalenten Mengen verwendet. Es ist jedoch auch möglich, das Carbonsäureanhydrid in einem größeren Uberschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Losungsmittel oder mit Wasser entfernt.

Das Verfahren (G) ist dadurch gekennzeichnet, daß man Verbindungen der Formeln (I-1-a) bis (I-5-a) jeweils mit Chlorameisensaureestern oder Chlorameisensäurethiolestern der Formel (X) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (G) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (G) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (G) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (G) wird im allgemeinen unter Normaldruck durchgeführt.

Bei der Durchführung des erfindungsgemäßen Verfahrens (G) werden die Ausgangsstoffe der Formeln (I-1-a) bis (I-5-a) und der entsprechende Chlorameisensäureester bzw. Chlorameisensäurethiolester der Formel (X) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (H) ist dadurch gekennzeichnet, daß man Verbindungen der Formeln (I-1-a) bis (I-5-a) jeweils mit (Hα) Verbindungen der Formel (XI) in Gegenwart eines Verdünnungsmittels und gegebenenfalls in Gegenwart eines Säurebindemittels oder (Hβ) Schwefelkohlenstoff und anschließend mit Alkylhalogeniden der Formel (XII) gegebenenfalls in Gegennwart eines Verdünnungsmittels und gegebenenfalls in Gegenwart einer Base umsetzt.

Beim Herstellungsverfahren (Hα) setzt man pro Mol Ausgangsverbindung der Formeln (I-1-a) bis (I-5-a) ca. 1 Mol Chlormonothioameisensäureester bzw. Chlordithioameisensäureester der Formel (XI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-5-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Hβ) setzt man pro Mol Ausgangsverbindungen der Formeln (I-1-a) bis (I-5-a) jeweils die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus den Verbindungen der Formeln (I-1-a) bis (1-5-a) durch Zusatz einer Base (wie z.B. Kaliumtertiärbutylat oder Natriumhydrid) das entsprechende Salz herzustellen. Man setzt die Verbindungen (I-1-a) bis (I-5-a) jeweils so lange mit Schwefelkohlenstoff um, bis die Bildung der Zwischenverbindung abgeschlossen ist, z.B. nach mehrstündigem Rühren bei Raumtemperatur.

Als Basen können beim Verfahren (Hβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise sind verwendbar aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Die weitere Umsetzung mit dem Alkylhalogenid der Formel (XII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Das erfindungsgemäße Verfahren (I) ist dadurch gekennzeichnet, daß man Verbindungen der Formeln (I-1-a) bis (I-5-a) jeweils mit Sulfonsaurechloriden der Formel (XIII) gegebenenfalls in Gegenwart eines Verdünnungsmittels und gegebenenfalls in Gegenwart eines Säurebindemittels umsetzt.

Beim Herstellungsverfahren (I) setzt man pro Mol Ausgangsverbindung der Formel (I-1-a bis I-5-a) ca. 1 Mol Sulfonsäurechlorid der Formel (XIII) bei -20 bis 150°C, vorzugsweise bei 0 bis 70°C um.

Das Verfahren (I) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-5-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (J) ist dadurch gekennzeichnet, daß man Verbindungen der Formeln (I-1-a) bis (I-5-a) jeweils mit Phosphorverbindungen der Formel (XIV) gegebenenfalls in Gegenwart eines Verdunnungsmittels und gegebenenfalls in Gegenwart eines Saurebindemittels umsetzt.

Beim Herstellungsverfahren (J) setzt man zum Erhalt von Verbindungen der Formeln (I-1-e) bis (I-5-e) auf 1 Mol der Verbindungen (I-1-a) bis (I-5-a), 1 bis 2, vorzugsweise 1 bis 1,3 Mol der Phosphorverbindung der Formel (XIV) bei Temperaturen zwischen -40°C und 150°C, vorzugsweise zwischen -10 und 110°C um.

Das Verfahren (J) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (K) ist dadurch gekennzeichnet, daß man Verbindungen der Formeln (I-1-a) bis (I-5-a) jeweils mit Metallhydroxiden bzw. Metallalkoxiden der Formel (XV) oder Aminen der Formel (XVI), gegebenenfalls in Gegenwart eines Verdünnungsmittels, umsetzt.

Als Verdünnungsmittel können bei dem erfindungsgemäßen Verfahren (K) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (K) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (L) ist dadurch gekennzeichnet, daß man Verbindungen der Formeln (I-1-a) bis (I-5-a) jeweils mit (Lα) Verbindungen der Formel (XVII) gegebenenfalls in Gegenwart eines Verdünnungsmittels und gegebenenfalls in Gegenwart eines Katalysators oder (Lβ) mit Verbindungen der Formel (XVIII) gegebenenfalls in Gegenwart eines Verdünnungsmittels und gegebenenfalls in Gegenwart eines Säurebindemittels umsetzt.

Bei Herstellungsverfahren (Lα) setzt man pro Mol Ausgangsverbindung der Formeln (I-1-a) bis (I-5-a) ca. 1 Mol Isocyanat der Formel (XVII) bei 0 bis 100°C, vorzugsweise bei 20 bis 50°C um.

Das Verfahren (Lα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Lβ) setzt man pro Mol Ausgangsverbindung der Formeln (I-1-a) bis (I-5-a) ca. 1 Mol Carbamidsäurechlorid der Formel (XVIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiarbutylat) das Enolatsalz der Verbindung (I-1-a) bis (I-5-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Peripianeta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia
Aus der Ordnung der Isoptera z.B. Reticulitermes spp.

Aus der Ordnung der Anoplura z.B. Phylloxera vastatrix. Pemphigus spp.,
Pediculus humanus corporis, Haematopinus spp., Linognathus spp
Aus der Ordnung der Mallophaga z.B. Trichodectes spp., Damalinea spp
Aus der Ordnung der Thysanoptera z.B. Hercinothrips femoralis, Thrips tabaci.
Aus der Ordnung der Heteroptera z.B. Eurygaster spp., Dysdercus intermedius,
Piesma quadrata, Cimex lectularius, Rhodnius prolixus, Triatoma spp.

5 Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) oder gegen die Raupen der Kohlschabe (Plutella maculipennis) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:

Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekampfung monokotyler Unkräuter in dikotylen Kulturen im Vor- und Nachlaufverfahren. Sie können beispielsweise in Baumwolle oder Zuckerruben mit sehr gutem Erfolg zur Bekämpfung von Schadgräser eingesetzt werden

Die Wirkstoffe können in die üblichen Formulierungen uberfuhrt werden, wie Lösungen, Emulsionen, Spritzpulver, Suspensionen, Pulver, Staubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diciobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen, Quinalphos,
RH 5992,

Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

5 Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenos, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel 1 eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze

### wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen,

Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise a-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on,
sein.

Die Herstellung und die Verwendung der erfindungsgemäßen Wirkstoffe geht aus den nachfolgenden Beispielen hervor.

### Herstellungsbeispiele

### Beispiel (I-1-a-1)

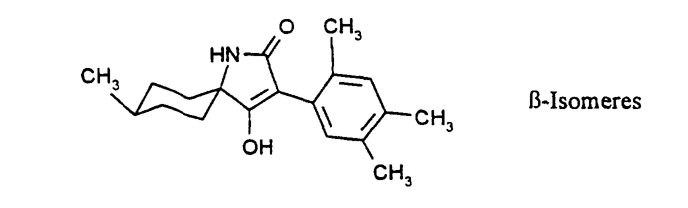

Zu 18,5 g (0,165 Mol) Kalium-tert.-butylat in 57 ml wasserfreiem Tetrahydrofuran (THF) tropft man bei Rückflußtemperatur 25,0 g (0,072 Mol) der Verbindung gemäß Beispiel (II-2) in 150 ml wasserfreiem Toluol und rührt 1,5 Stunden unter Rückfluß. Zur Aufarbeitung gibt man 220 ml Wasser zu, trennt die wäßrige Phase ab, extrahiert die Toluolphase mit 110 ml Wasser, vereinigt die wäßrigen Phasen, wäscht sie mit Toluol und säuert bei 10 bis 20°C mit etwa 26 ml konz. HCl an. Das Produkt wird abgesaugt, gewaschen, getrocknet und durch Verrühren in Methyl-tert.-butyl(MTB)-ether/n-Hexan gewaschen.
Ausbeute: 18,0 g (79 % der Theorie), Fp.: 159°C.

Analog bzw. gemäß den allgemeinen Angaben zur Herstellung erhält man die folgenden Verbindungen der Formel (I-1-a):

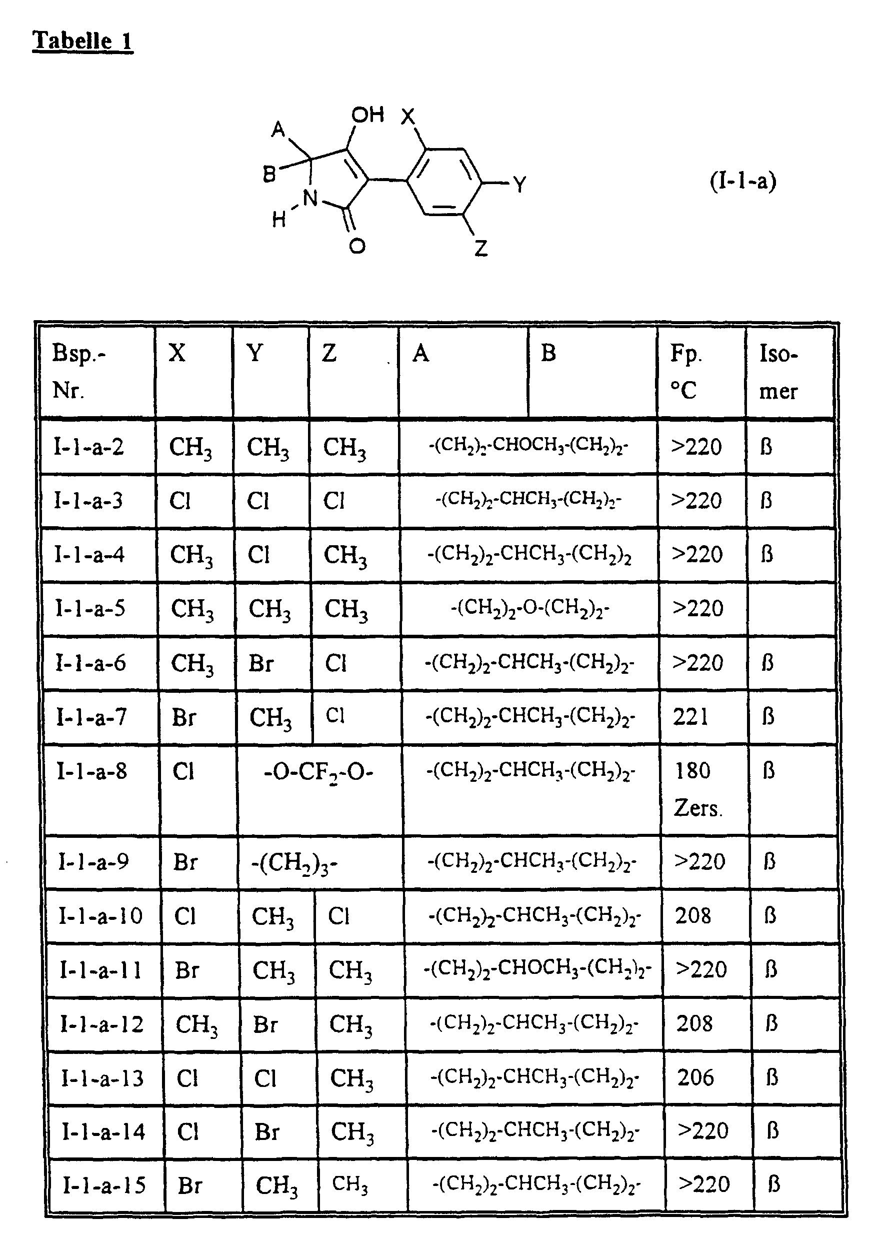

### Beispiel (I-1-b-1)

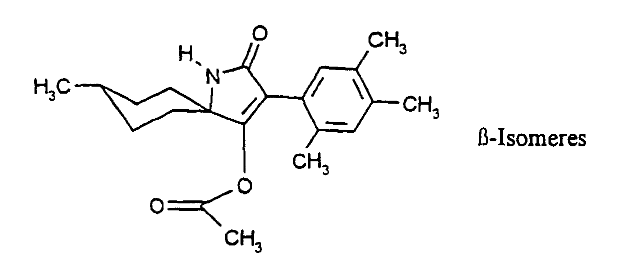

4,5 g (0,015 Mol) der Verbindung gemäß Beispiel (I-1-a-1) und 2,1 ml (15 mMol) Triethylamin in 70 ml absolutem Methylenchlorid werden bei 0 bis 10°C mit 1,13 ml (0,015 Mol) Acetylchlorid in 5 ml absolutem Methylenchlorid versetzt. Bei Raumtemperatur wird gerührt, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft. Das Rohprodukt wird aus MTB-Ether/n-Hexan umkristallisiert.
Ausbeute: 3,4 g (66 % der Theorie), Fp.: 209°C.
Analog bzw. gemäß den allgemeinen Angaben zur Herstellung erhält man die folgenden Verbindungen der Formel (I-b-1):

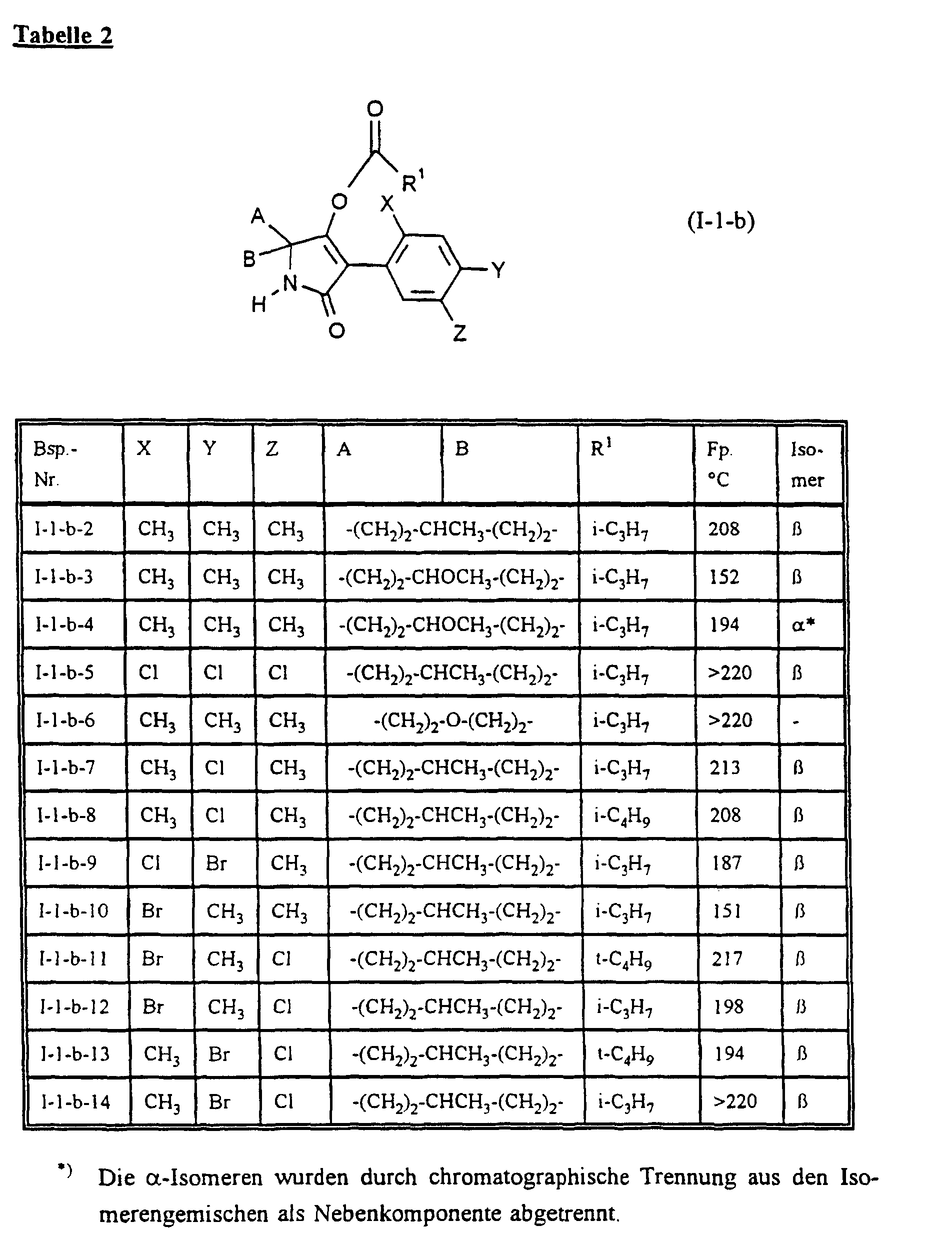

### Beispiel (I-1-c-1)

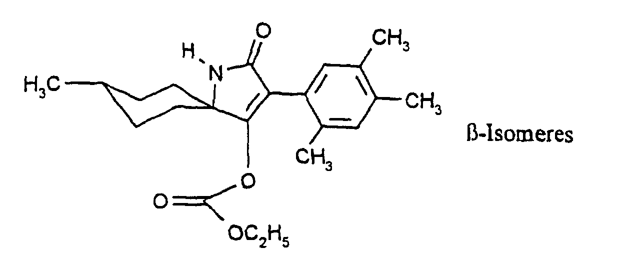

Zu 4,5 g (0,015 Mol) der Verbindung gemäß Beispiel (I-1-a-1) und 2,1 ml Triethylamin in 70 ml absolutem Methylenchlorid tropft man bei 0 bis 10°C 1,5 ml (15 mMol) Chlorameisensäureethylester in 5 ml absolutem Methylenchlorid und rührt bei Raumtemperatur, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft.
Ausbeute: 3,3 g (59 % der Theorie), Fp.: 193°C.

Analog bzw. gemäß den allgemeinen Angaben zur Herstellung erhält man die folgenden Verbindungen der Formel (I-1-c):

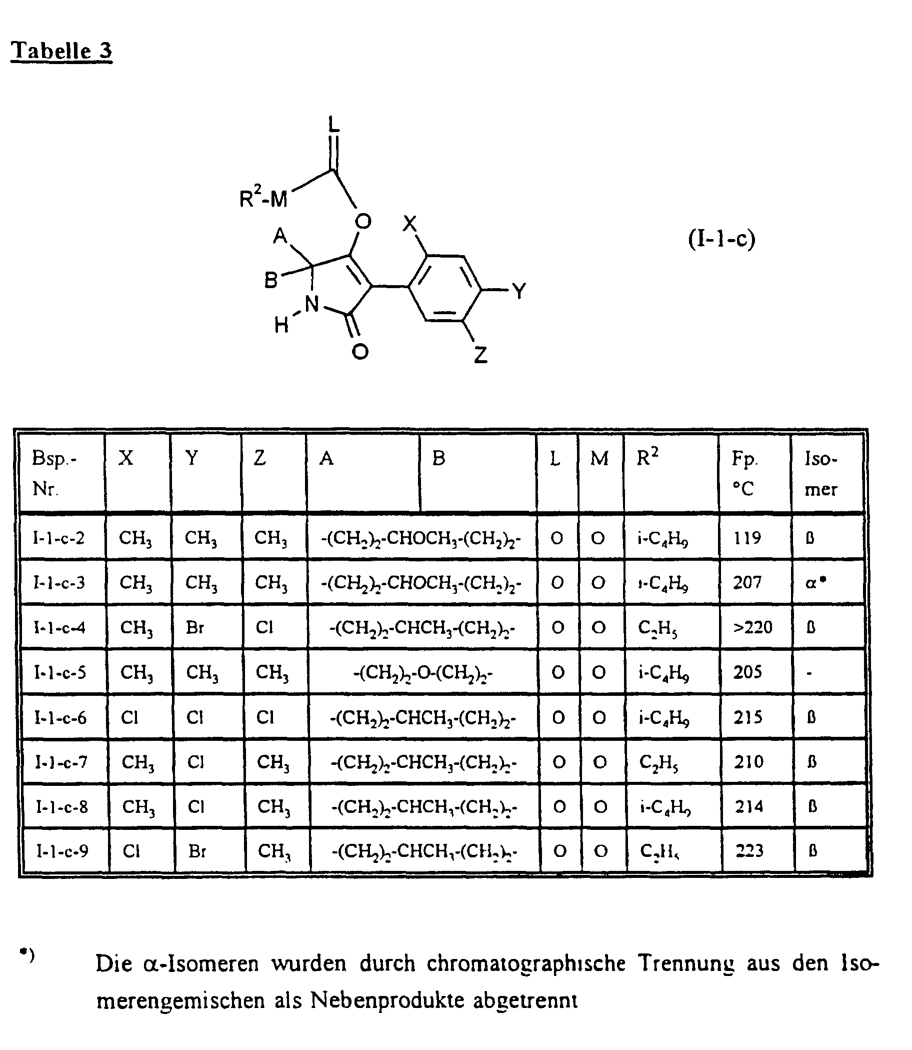

### Beispiel II-1

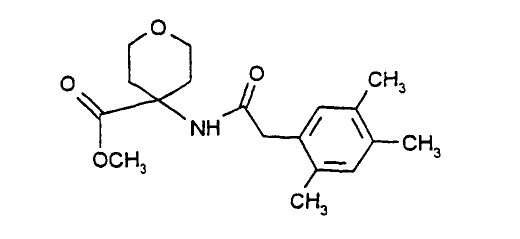

Zu 30,3 g (0,308 Mol) konz. Schwefelsäure tropft man bei 30 bis 40°C vorsichtig 17,5 g der Verbindung gemäß Beispiel (XXIX-1) in etwa 100 ml Methylenchlorid und rührt 2 Stunden bei dieser Temperatur. Dann tropft man 41 ml absolutes Methanol so zu, daß sich eine Innentemperatur von etwa 40°C einstellt und rührt noch 6 Stunden bei 40 bis 70°C.

Zur Aufarbeitung gießt man auf 0,29 kg Eis, extrahiert mit Methylenchlorid, wäscht mit wäßriger Natriumhydrogencarbonatlösung, trocknet und dampft ein. Das Rohprodukt wird durch Säulenchromatographie an Kieselgel mit dem Laufmittel Methylenchlorid/Essigester 2:1 gereinigt.
Ausbeute: 13,1 g (67 % der Theorie), Fp.: 147°C.

### Beispiel (II-2)

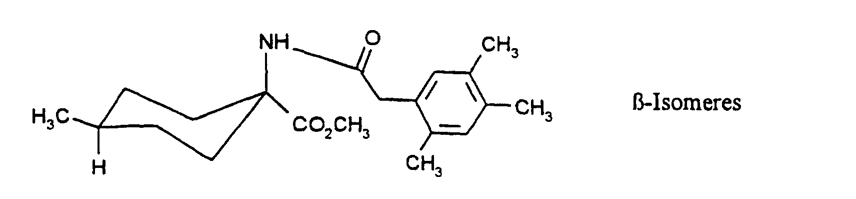

40,9 g (0,23 Mol) 2,4,5-Trimethylphenylessigsäure und 33,6 ml (0,461 Mol) Thionylchlorid werden 30 Min. bei Raumtemperatur und anschließend bei 50°C gerührt, bis die Gasentwicklung beendet ist. Überschüssiges Thionylchlorid wird bei 50°C im Vakuum entfernt. Dann gibt man 50 ml absolutes Toluol zu und dampft erneut ein. Der Rückstand wird in 100 ml absolutem THF aufgenommen (Lösung 1).

Zu 47,9 g cis-4-Methylcyclohexylamin-1-carbonsäuremethylester und 64,6 ml (0,460 Mol) Triethylamin in 600 ml absolutem THF tropft man bei 0 bis 10°C Lösung 1 und rührt anschließend 1 Stunde bei Raumtemperatur. Dann wird abgesaugt, mit absolutem THF gewaschen und eingedampft. Der Rückstand wird in Methylenchlorid aufgenommen, mit 0,5 N HCI gewaschen, getrocknet und eingedampft. Das Rohprodukt wird durch Säulenchromatographie an Kieselgel mit Methylenchlorid/Essigester 7:1 gereinigt.
Ausbeute: 25 g (32 % der Theorie), Fp.: 158°C.

Analog zu den Beispielen (II-1) und (II-2) und gemäß den allgemeinen Angaben zur Herstellung werden die folgenden Verbindungen der Formel (II) hergestellt.

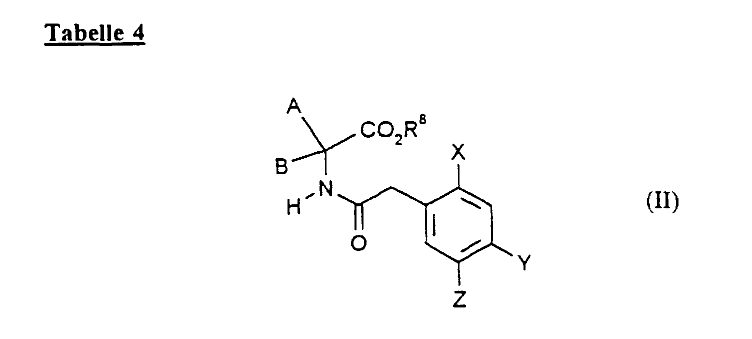

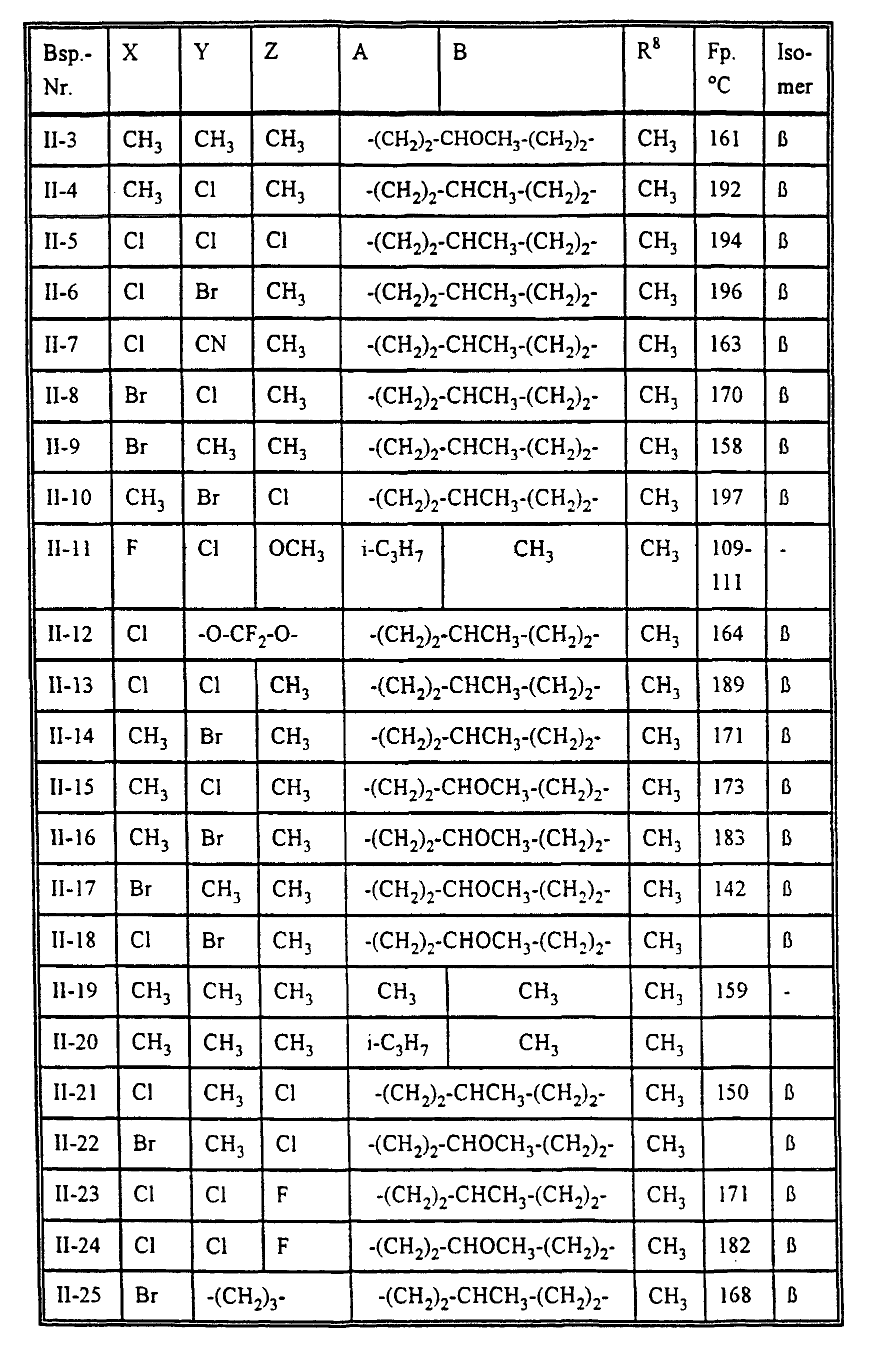

### Beispiel (XXIX-1)

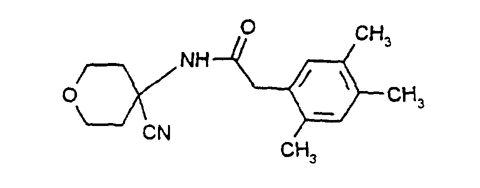

Ausgehend von 17,8 g 2,4,5-Trimethylphenylessigsäure wird wie in Beispiel (II-2) Lösung 1 hergestellt.

Zu 16,8 g 1-Amino-tetrahydropyran-1-carbonsäurenitril (70 %ig) und 16,8 ml (0,12 Mol) Triethylamin in 150 ml absolutem THF tropft man bei 0 bis 10°C Lösung 1 und rührt noch 1 Stunde bei Raumtemperatur. Dann wird eingedampft und man nimmt den Rückstand in Methylenchlorid auf, wäscht mit 0,5 N HCI, trocknet und dampft ein. Das Rohprodukt wird aus MTB-Ether/n-Hexan umkristallisiert.
Ausbeute: 17,5 g (61 % der Theorie), Fp.: 156°C.

### Beispiel (I-2-a-1)

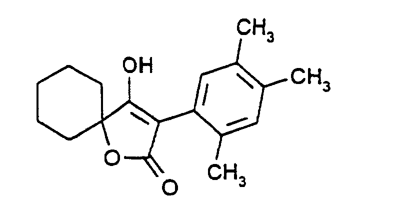

8,42 g (75 mMol) Kalium-tert.-butylat werden in 50 ml DMF vorgelegt, bei 0 bis 10°C eine Lösung von 16,6 g (50 mMol) 2,4,5-Trimethyl-phenylessigsäure-(1-ethyloxycarbonyl-cyclohexyl)-ester gemäß Beispiel (III-1) in 50 ml DMF zugetropft und über Nacht bei Raumtemperatur gerührt.

Zur Aufarbeitung tropft man das Reaktionsgemisch in 500 ml eiskalte 1 N HCI, saugt das ausgefallene Produkt ab, wäscht mit Wasser und trocknet im Vakuumtrockenschrank. Zur weiteren Reinigung wird das Rohprodukt noch mit n-Hexan/Aceton ausgekocht.
Ausbeute: 9,2 g (64 % der Theorie) vom Fp.: 209-212°C.

Analog bzw. gemäß den allgemeinen Angaben zur Herstellung erhält man die folgenden Verbindungen der Formel (I-2-a):

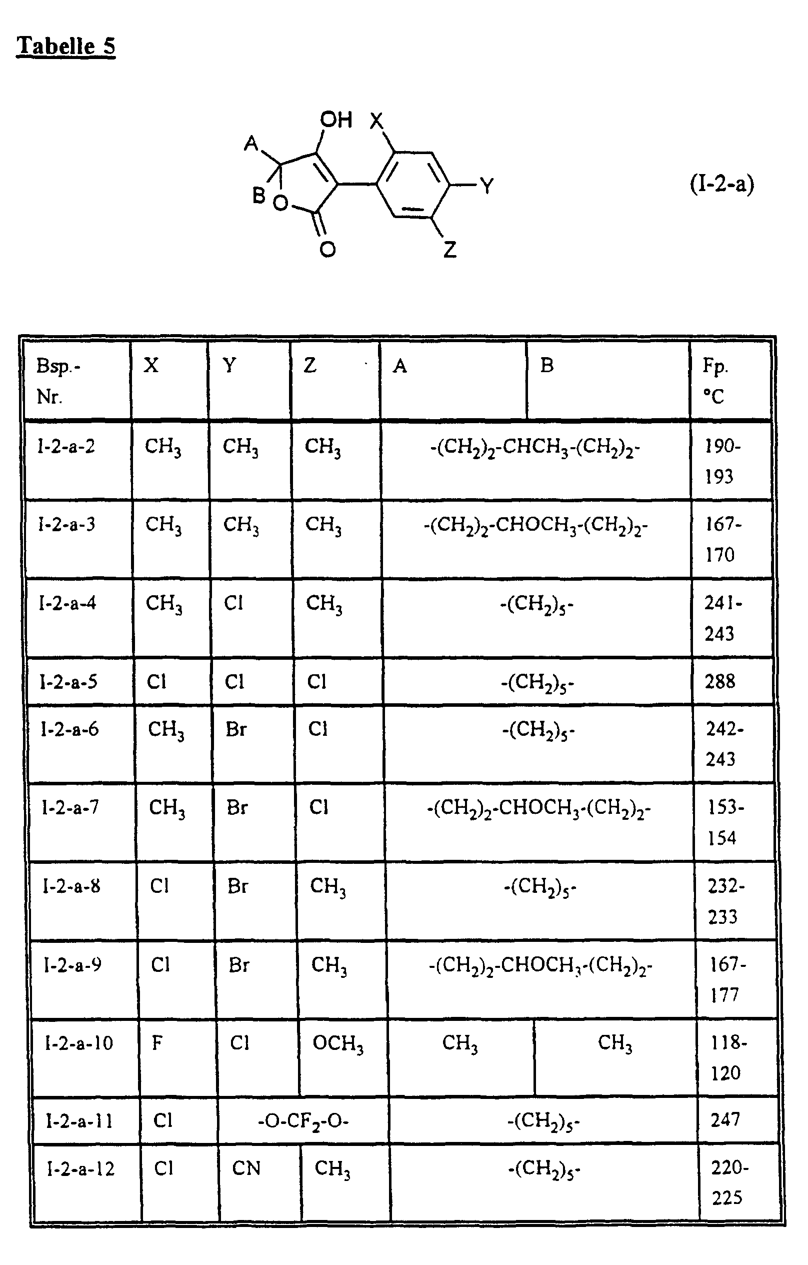

### Beispiel (I-2-b-1)

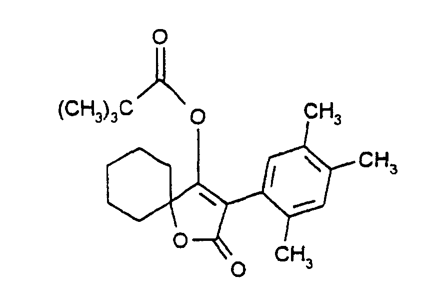

2,86 g (10 mMol) der Verbindung gemäß Beispiel I-2-a-1 werden in 40 ml Dichlormethan vorgelegt, 1,52 g (15 mMol) Triethylamin zugesetzt, unter Eiskühlung eine Lösung von 1,57 g (13 mMol) Pivaloylchlorid in 40 ml Dichlormethan zugetropft und 1 bis 2 h bei Raumtemperatur nachgerührt. Zur Aufarbeitung wäscht man nacheinander mit 10 %iger Citronensäure, 1N NaOH und NaCl-Lösung, trocknet die organische Phase über MgSO₄ und dampft ein. Das Rohprodukt wird zur weiteren Aufreinigung noch mit wenig Petrolether verrührt.
Ausbeute: 3,0 g (81 % der Theorie) vom Fp.: 128-132°C.

Analog bzw. gemäß den allgemeinen Angaben zur Herstellung erhält man die folgenden Verbindungen der Formel (I-2-b):

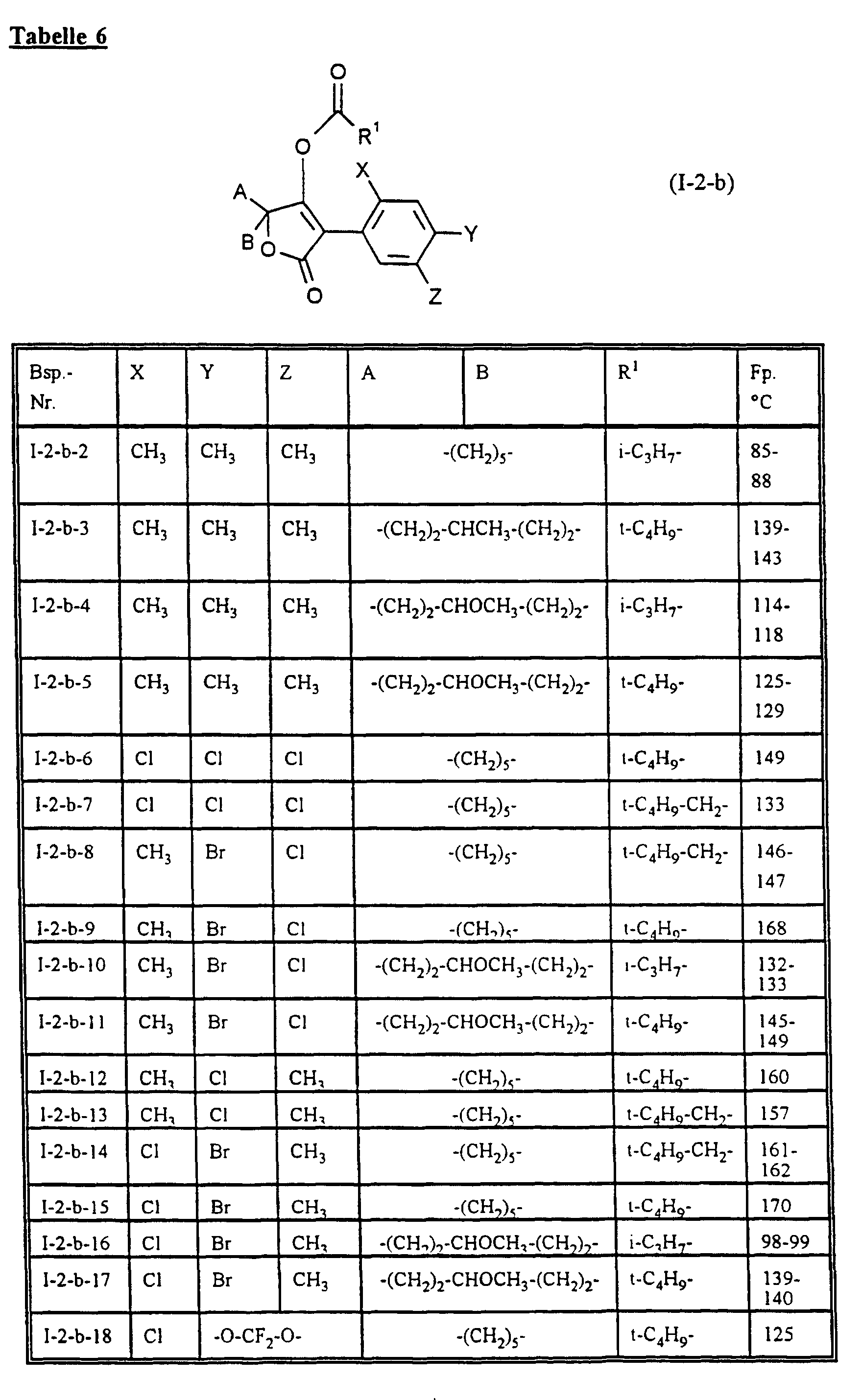

### Beispiel (III-1)

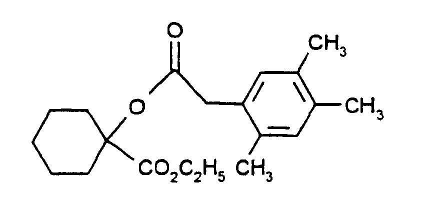

8,9 g (50 mMol) 2,4,5-Trimethyl-phenylessigsäure werden in 50 ml Toluol vorgelegt, 11,9 g (100 mMol) Thionylchlorid zugegeben, bis zur Beendigung der Chlorwasserstoffentwicklung bei 80°C gerührt und eingedampft. Das rohe Säurechlorid wird zusammen mit 8,6 g (50 mMol) 1-Hydroxy-cyclohexancarbonsäureethylester über Nacht in 50 ml Toluol gekocht, anschließend wird eingedampft.
Ausbeute: 18,6 g (quant.) 2,4,6-Trimethyl-phenylessigsäure-(1-ethoxycarbonylcyclohexyl)-ester als farbloses Öl.

Analog bzw. gemäß den allgemeinen Vorschriften zur Herstellung erhält man die folgenden Verbindungen der Formel (III):

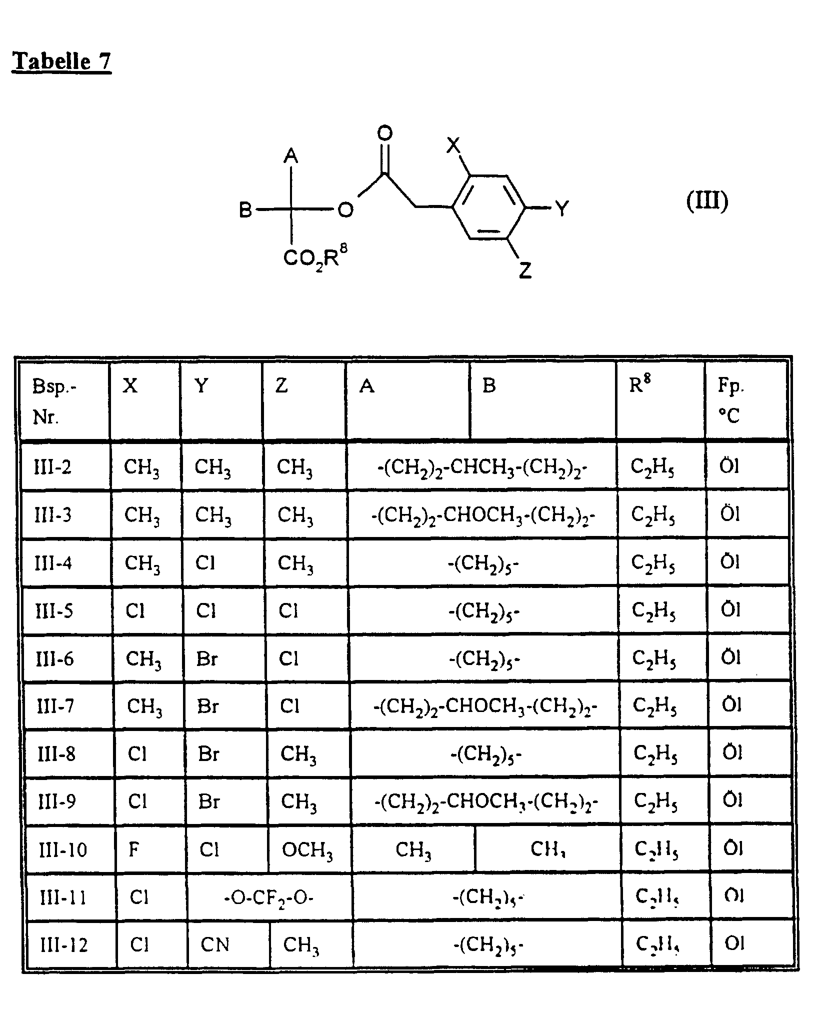

### Beispiel (I-3-a-1)

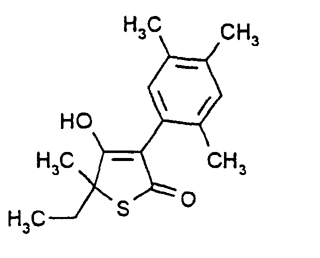

26,0 g (60,7 mmol) der Verbindung gemäß Beispiel (IV-1) werden mit 55 ml Trifluoressigsäure in 110 ml Toluol 3 Stunden unter Rückfluß erhitzt. Überschüssige Trifluoressigsäure wird im Vakuum entfernt, der Rückstand mit 400 ml Wasser und 120 ml MTB-Ether versetzt, mit NaOH wird pH 14 eingestellt. Man extrahiert 2 mal mit MTB-Ether, säuert die wäßrige Phase mit konzentriertem HCl und extrahiert 3 mal mit MTB-Ether. Die organischen Phasen werden getrocknet und eingeengt. Ausbeute 8,8 g (52 % der Theorie), Fp. 160 bis 162°C.

### Beispiel (I-3-b-1)

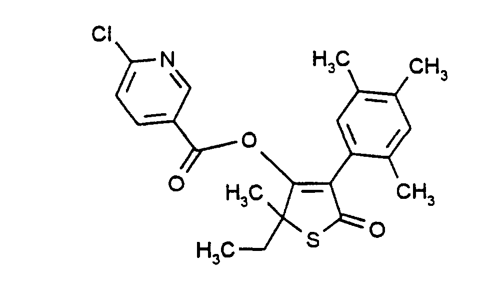

### Ansatz:

1,0 g (3,6 mmol) der Verbindung gemäß Beispiel I-3-a-1 werden in 15 ml abs. Methylenchlorid vorgelegt und mit 0,75 ml Triethylamin versetzt. Eine Lösung aus 0,82 g (4,68 mmol) 6-Chlornicotinsäurechlorid in 3 ml abs. Methylenchlorid werden unter Eiskühlung zugetropft. Der Ansatz wird 2 Stunden bei Raumtemperatur gerührt. Man wäscht 2 mal mit 10%iger Citronensäure und extrahiert die vereinigten wässrigen Phasen mit Methylenchlorid. Die vereinigten organischen Phasen werden 2 mal mit 1 N NaOH gewaschen, die wässrigen alkalischen Phasen mit Methylenchlorid extrahiert. Schließlich werden die vereinigten organischen Phasen getrocknet, eingeengt und der Rückstand mit Petrolether verührt. Ausbeute 1,37 g (91 % der Theorie), Fp. 123 bis 126°C.

Analog zu Beispiel I-3-b-1 bzw. gemäß der allgemeinen Beschreibung wurden die in der nachfolgenden Tabelle aufgeführten Verbindungen der Formel I-3-b hergestellt.

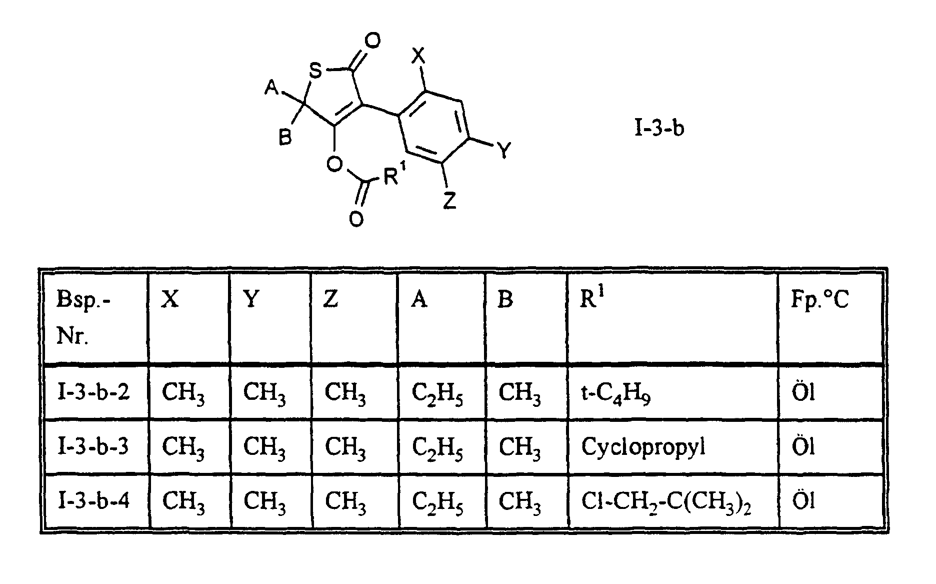

### Beispiel (I-3-c-1)

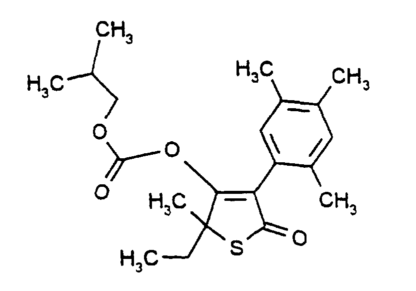

### Ansatz:

1,0 g (3,6 mmol) der Verbindung gemäß Beispiel I-3-a-1 werden in 15 ml abs. Methylenchlorid vorgelegt und mit 0,75 ml (1,5 eq) Triethylamin versetzt. Eine Lösung von 0,61 ml (0,64 g; 4,68 mmol) 6-Chlorameisensaureisobutylester in 3 ml abs. Methylenchlorid wird unter Eiskühlung zugetropft. Der Ansatz wird 2 Stunden bei Raumtemperatur gerührt. Man wäscht 2 mal mit 10%iger Citronensäure und extrahiert die vereinigten wässrigen Phasen mit Methylenchlorid. Die vereinigten organischen Phasen werden 2 mal mit 1 N NaOH gewaschen, die wäßrigen alkalischen Phasen mit Methylenchlorid extrahiert. Schließlich werden die vereinigten organischen Phasen getrocknet, eingeengt.
Ausbeute 1,32 g (97 % der Theorie), Öl.
¹H-NMR (400 MHz, DMSO):
- δ =: 0,60 - 0,70 m, 6H, CH(CH₃)₂
1,00 - 1,05 m, 3H, CH₂CH₃
1,50 - 1,60 m, 1H, CH(CH₃)₂
1,70 - 1,76 d, 3H, C(CH₃)
1,90 - 2,02 m, 2H, CH₂CH₃
2,05 - 2,20 m, 9H, ArCH₃
3,65 - 3,72 m, 2H, OCH₂
6,75 - 6,80 d, 1H, ortho Ar-H
7,01 s, 1H, meta Ar-H

### Beispiel (I-3-c-2)

Analog zu Beispiel (I-3-c-1) wurde die Verbindung der Formel (I-3-c-2) als Öl erhalten:

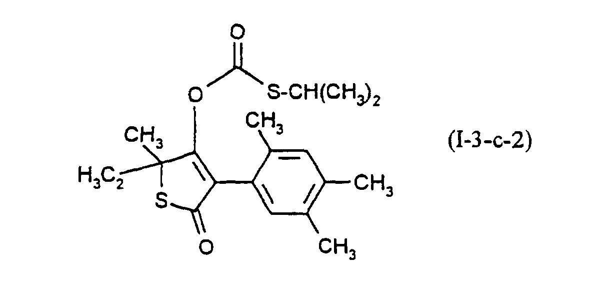

### Beispiel (IV-1)

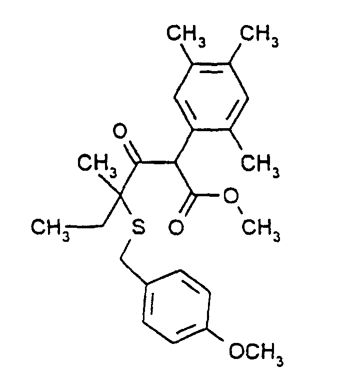

A: 25 g (98 mmol) der Verbindung der Formel (XXXII-1) 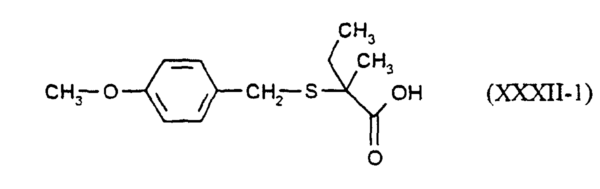 und
   1 Tropfen DMF werden mit 17,5 g (147 mmol) Thionylchlorid in 100 ml Toluol 5 Minuten bei Raumtemperatur und anschließend solange bei 100°C gerührt, bis die Gasentwicklung beendet ist. Flüchtige Bestandteile werden im Hochvakuum entfernt.
B: Zu 18 ml (130 mmol) Diisopropylamin in 100 ml THF tropft man unter Eiskühlung 72 ml (118 mmol) Butyllithium (1,6 M) und rührt noch 15 Minuten bei dieser Temperatur. Anschließend tropft man 18,8 g (108 mmol) der Verbindung gemäß Beispiel (XXTV-3) gelöst in 40 ml THF bei 0°C zu und rührt 30 Minuten bei dieser Temperatur. Anschließend wird das nach A hergestellte Säurechlorid gelöst in 40 ml THF bei 0°C zugetropft, man rührt 1 Stunde bei Raumtemperatur. Dann gibt man 350 ml MTB-Ether und einige Tropfen Wasser zu, extrahiert 2 mal mit 10%iger Ammoniumchloridlösung, trocknet und engt ein. Das Rohprodukt (40 g) wird säulenchromatographisch (Laufmittel Cyclohexan/Essigester 10/1) gereinigt. Ausbeute 27,0 g (64 % der Theorie), Öl.
   ¹H-NMR (CDCl₃, 400 MHz):
   - δ:: 0,80 - 0,95 m, 3H, CH₂CH₃ 1,42 s, 3H, C-CH₃ 1,65 - 2,05 m, 2H, CH₂CH₃ 2,15 - 2,35 m, 9H, ArCH₃ 3,10 - 3,45 m, 2H, SCH₂ 3,70 - 3,80 m, 6H, OCH₃ 6,70 - 7,30 m, 6H, Ar-H

### Beispiel (I-4-a-1)

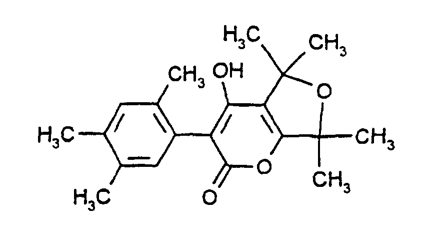

6,7 g (30 mMol) 2-(2,4,5-Trimethylphenyl)-chlorcarbonylketen werden mit 4,3 g (30 mMol) 4,5-Dihydro-2,2,5,5-tetramethyl-3-(2H)-furanon 4 h auf 200°C erwärmt. Nach Säulenchromatographie an Kieselgel mit Toluol/Ethanol 20:1 als Laufmittel erhält man 4,6 g (Δ 46 % der Theorie) vom Schmp.: 182-184°C.

Analog bzw. gemäß den allgemeinen Angaben zur Herstellung erhält man die folgenden Verbindungen der Formel (I-4-a):

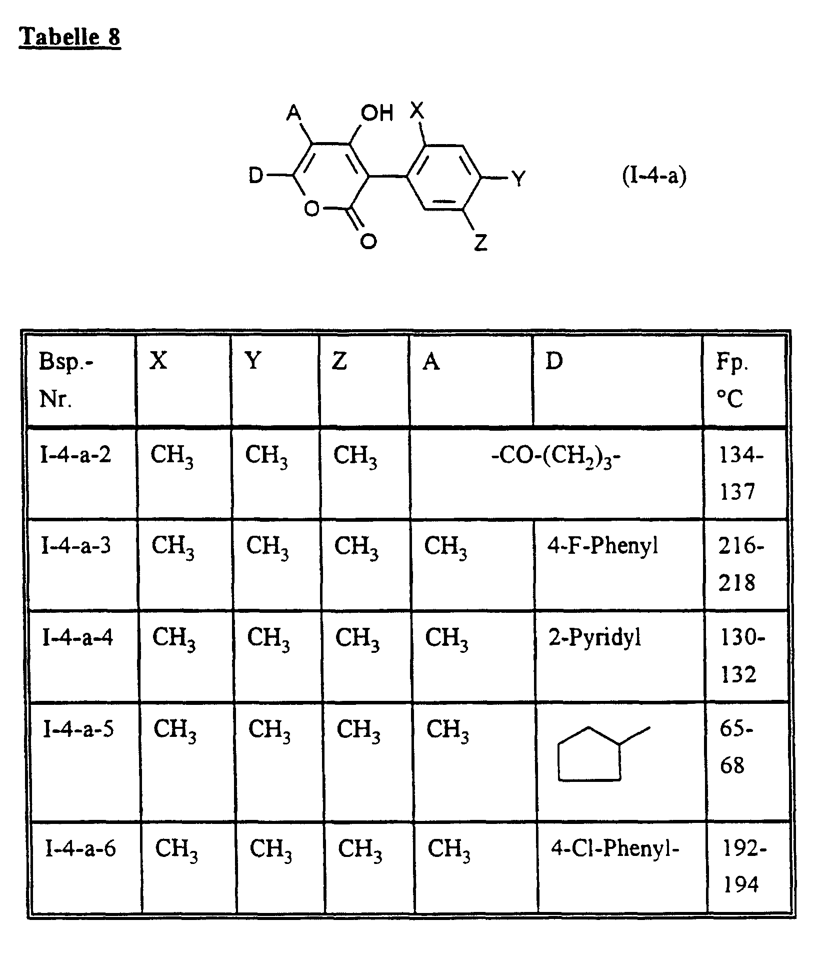

### Beispiel (I-4-b-1)

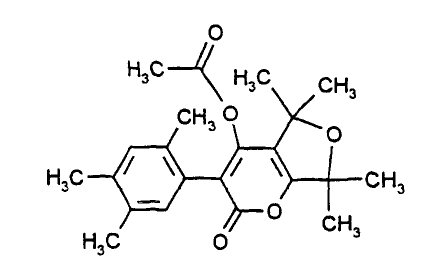

2,5 g (7,5 mMol) der Verbindung (I-4-a-1) werden in 25 ml Essigester vorgelegt, mit 0,75 g Triethylamin versetzt und bei 0°C 0,6 g Acetylchlorid in 20 ml Essigester zugetropft. Man rührt 20 h bei Raumtemperatur, trennt den Niederschlag ab, wäscht zweimal mit 50 ml halbkonz. Natriumchloridlösung, trocknet über Natriumsulfat und dampft im Vakuum ein. Der Rückstand wird an Kieselgel mit Toluol/Aceton 50:1 chromatographiert.
Ausbeute: 0,8 g (Δ 29 % der Theorie) vom Schmp.: 143-144°C.

Analog zu Beispiel I-4-b-1 bzw. gemäß der allgemeinen Angaben zur Herstellung wurden die in der nachfolgenden Tabelle aufgeführten Verbindungen der Formel I-4-b hergestellt:

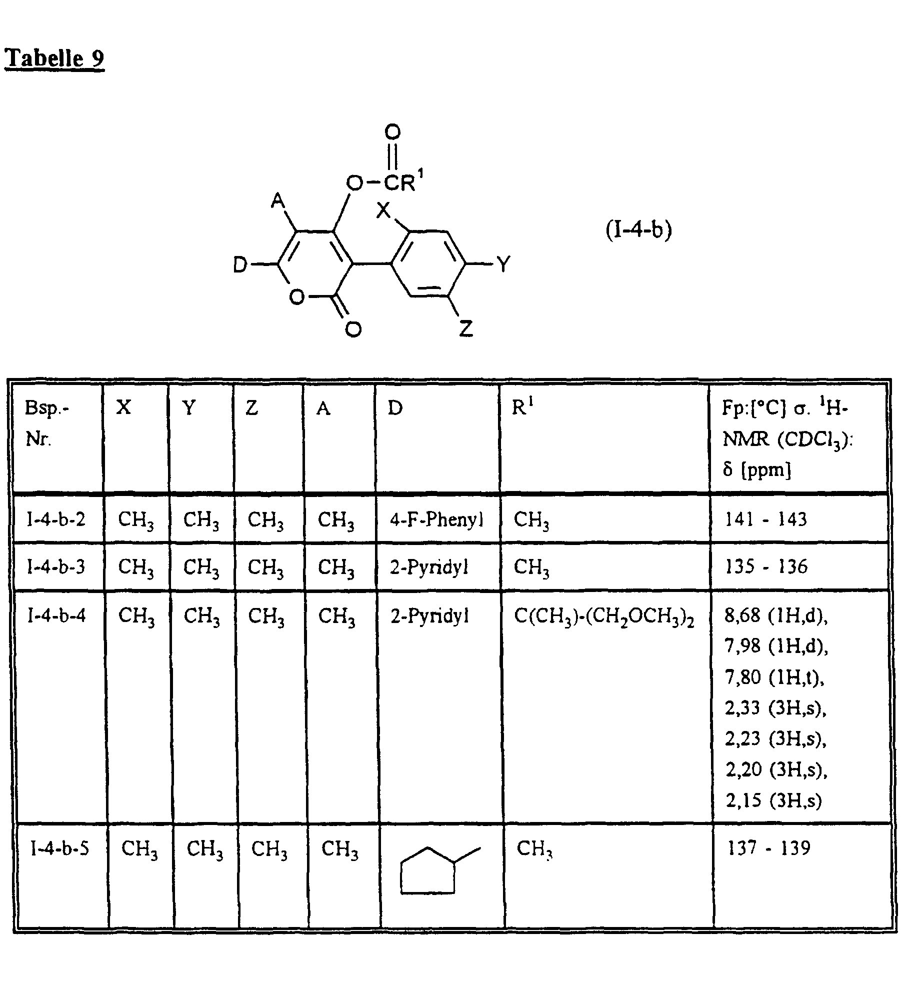

### Beispiel (I-4-c-1)

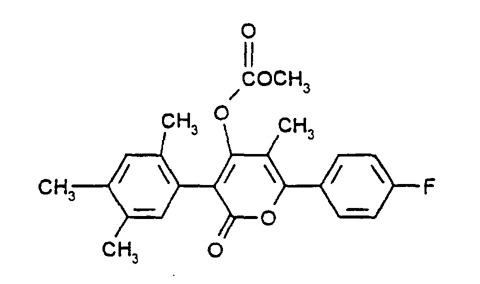

1,7 g (5 mmol) der Verbindung (I-4-a-3) werden in 20 ml Essigester vorgelegt, mit 0,5 g (5 mmol) Triethylamin versetzt und bei 0°C 0,5 g (5 mmol) Chlorameisensäuremethylester in 5 ml Essigester zugetropft. Man rührt 20 Stunden bei Raumtemperatur, trennt den Niederschlag ab, wäscht zweimal mit 50 ml halbkonzentrierter Natriumchloridlösung, trocknet über Natriumsulfat und dampft im Vakuum ein. Der Rückstand wird an Kieselgel mit Toluol/Aceton 30/1 chromatographiert. Ausbeute 1,0 g (51 % der Theorie) vom Schmelzpunkt 144 bis 146°C.

Analog bzw. gemäß den allgemeinen Angaben zur Herstellung erhält man die folgenden Verbindungen der Formel (I-4-c):

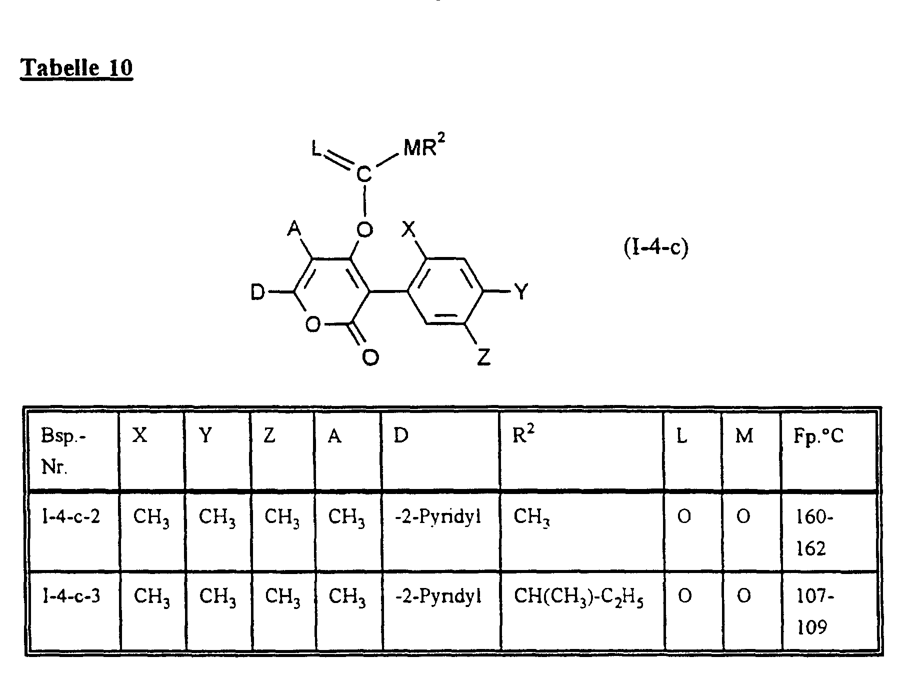

### Beispiel (I-5-a-1)

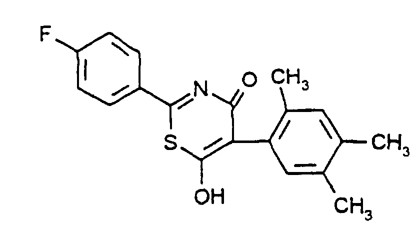

2,2 g (10 mmol) 2-(2,4,5-Trimethylphenyl)-Chlor-carbonylketen werden mit 1,6 g (10 mmol) 4-Fluorthiobenzamid in 80 ml Toluol 6 Stunden auf 50°C erwärmt. Der Niederschlag wird abgetrennt, mit Cyclohexan gewaschen und getrocknet. Man erhält 2,8 g (82 % der Theorie) vom Schmelzpunkt 215 bis 216°C.

### Beispiel (XXIII-1)

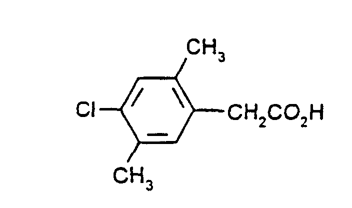

Zu einer Mischung aus 100 g (1,785 Mol) KOH in 130 ml Wasser und 260 ml Methanol tropft man bei Raumtemperatur 247 g (1,162 Mol) der Verbindung gemäß Beispiel (XXIV-1) (75 %ig) und erhitzt 5 Stunden unter Rückfluß. Nach dem Abkühlen wird mit 300 ml Wasser verdünnt und mit Essigsäureethylester extrahiert. Die wäßrige Phase wird mit halbkonzentrierter Salzsäure angesäuert, der Niederschlag abgesaugt und getrocknet.
Ausbeute: 77 g (45 % der Theorie), Fp.: 119-121°C.

### Beispiel (XXIII-2)

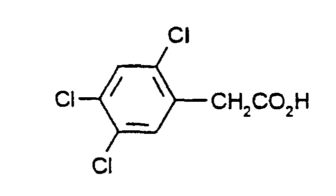

Analog zu Beispiel (XXIII-1) erhält man 2,4,5-Trichlor-phenylessigsäure, Fp.: 112-115°C.

### Darstellung von 2,4,5-Trimethyl-phenylessigsäure

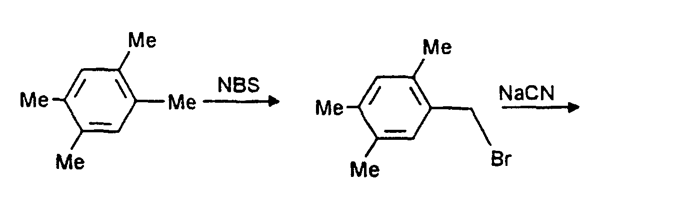

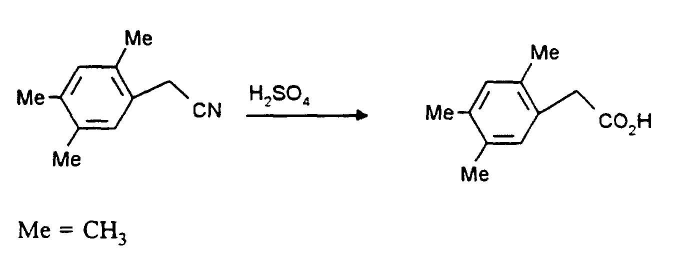

### Beispiel (XXIII-3)

286 g (1,4 Mol) 66 %iges 1,2,4,5-Tetramethylbenzol werden in 563 ml Tetrachlorkohlenstoff gelöst und bei Raumtemperatur nacheinander 27,5 g (0,15 Mol) N-Bromsuccinimid und 0,4 g Benzoylchlorid eingetragen. Es wird auf 80°C erwärmt und portionsweise 248 g (1,39 Mol) N-Bromsuccinimid zugegeben. Danach läßt man noch 30 Minuten bei 80°C nachrühren und kühlt anschließend auf Raumtemperatur ab. Der Feststoff wird abgesaugt und das Lösungsmittel bei 20 mbar über eine Feststoffbrücke abgezogen. Anschließend destilliert man den Rückstand im Hochvakuum und erhält 226 g (66 % der Theorie) 2,4,5-Trimethylbenzylbromid mit einem Kp. von 95°C bei 0,05 mbar und einer Reinheit von 86 %.

Zu einer Lösung aus 57 g (1,16 Mol) Natriumcyanid in 63 ml Wasser und 0,6 g Aliquat 336 wird bei 60 bis 80°C eine Lösung aus 226 g (0,91 Mol) 2,4,5-Trimethyl-benzylbromid (86 %ig) in 94 ml Toluol getropft und danach 4 Stunden bei 80°C nachgerührt. Nach dem Abkühlen auf Raumtemperatur werden die Phasen getrennt und die organische Phase zweimal mit Wasser und zweimal mit gesättigter NaCl-Lösung gewaschen, getrocknet und eingeengt. Nach der Destillation erhält man 103 g (70 %) 2,4,5-Trimethyl-benzylcyanid mit einer Reinheit von 99 % und einem Siedepunkt von 120°C bei 0,2 mbar.

Zu 2662 ml Wasser werden bei Raumtemperatur 2118 ml konzentrierte Schwefelsäure getropft und die Lösung auf 90°C erwärmt. Bei dieser Temperatur dosiert man 355 g (2,23 Mol) 2,4,5-Trimethyl-benzylcyanid in die halbkonzentrierte Schwefelsäure und rührt 8 Stunden bei 100°C nach. Nach dem Abkühlen gibt man das Reaktionsgemisch unter starkem Rühren in Eiswasser und saugt ab. Der Feststoff wird mehrmals mit Wasser und anschließend mit Petrolether gewaschen und getrocknet. Man erhält 358 g (90 % der Theorie) 2,4,5-Trimethyl-phenylessigsäure mit einem Schmelzpunkt von 123-125°C.

Analog zu Beispielen (XXIII-1) und (XXIII-3) erhält man die in der Tabelle 11 aufgeführten neuen Phenylessigsäuren der Formel (XXIII)

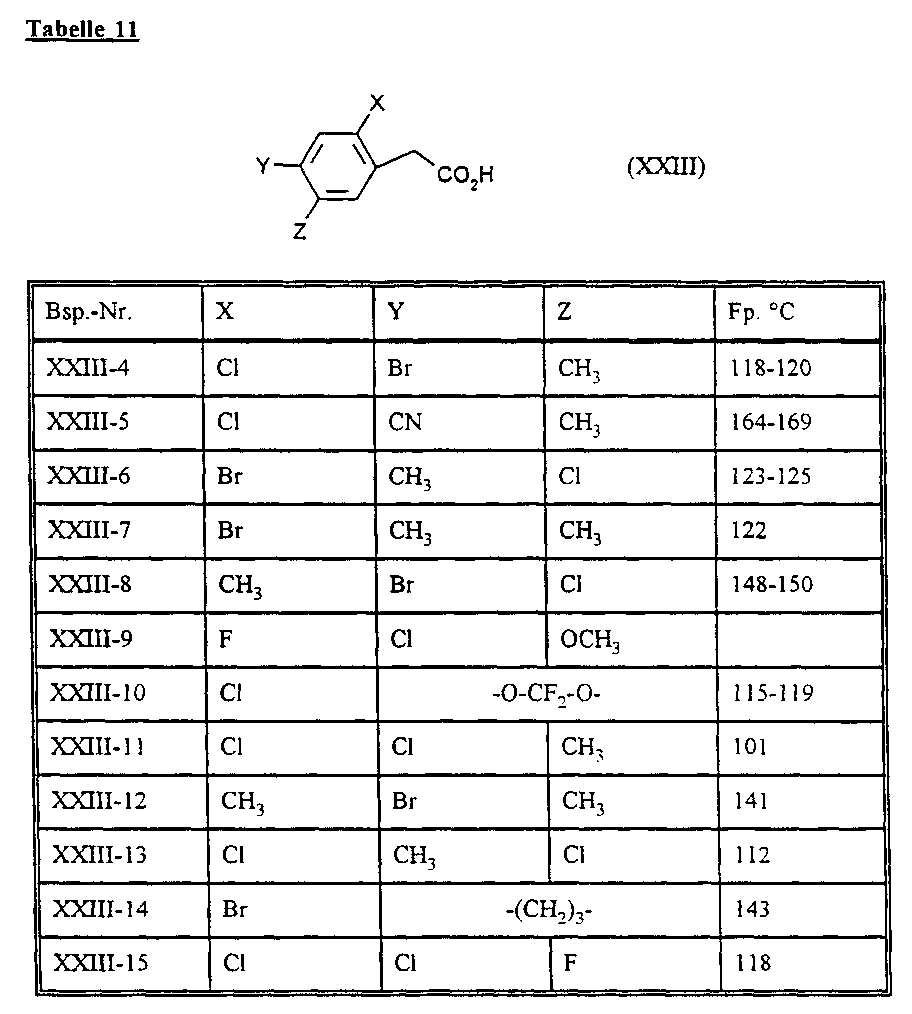

Die Verbindungen der Formel (XXIII) sind neu mit Ausnahme der Verbindungen, in welcher X, Y, Z = CH₃ und in welcher X, Y, Z = Cl

### Beispiel (XXIV-3)

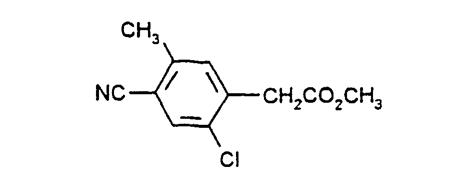

35 g (0,143 Mol) 2-Chlor-4-brom-5-methylphenylessigsäuremethylester und 31 g CuCN werden in 350 ml Dimethylformamid 1 Tag unter Rückfluß gekocht. Dann wird im Vakuum eingeengt, der Rückstand zwischen Wasser und tert.-Butylmethylether verteilt, die organische Phase getrocknet und eingeengt.
Ausbeute: 18 g

In Analogie zu Beispiel (XXIV-1) erhält man die in der Tabelle 12 aufgeführten neuen Phenylessigsäureester der Formel (XXIV).

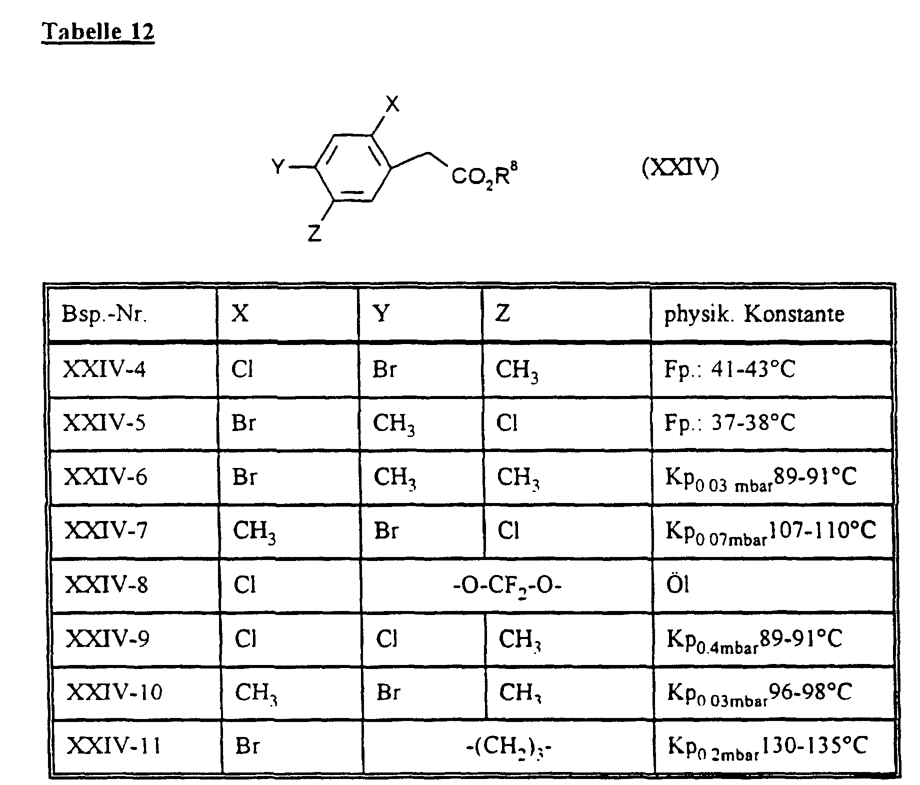

Die Verbindungen der Formel (XXIV) sind neu mit Ausnahme der Verbindung, in welcher X, Y, Z = CH₃ und in welcher X, Y, Z = Cl

### Beispiel (XXIV-1)

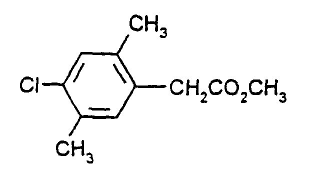

Zur Lösung von 347 g (0,948 Mol) der Verbindung gemäß Beispiel (XXV-1) (74,3 %ig) in 410 ml Methanol tropft man bei Raumtemperatur 700 ml einer 30 %igen methanolischen Lösung von Natriummethylat, erhitzt 5 Stunden unter Rückfluß, kühlt auf Raumtemperatur ab und tropft 110 ml konz. Schwefelsäure zu. Man kocht eine Stunde unter Rückfluß, destilliert das Methanol ab und nimmt den festen Rückstand in Wasser auf. Man trennt die organische Phase ab und extrahiert die wäßrige Phase 2 mal mit 1,5 1 Methylenchlorid. Die vereinigten organischen Phasen werden über Na₂SO₄ getrocknet und eingeengt.
Ausbeute: 247 g eines dunklen Öls Δ 92 % der Theorie mit einem Gehalt von 75 % (GC).

### Beispiel (XXIV-2)

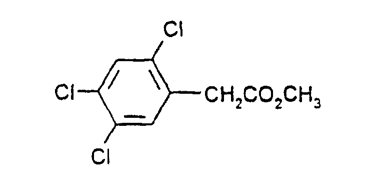

Analog zu Beispiel (XXIV-1) erhält man 2,4,5-Trichlor-phenylessigsäuremethylester als dunkles Öl in einer Ausbeute von 95 % der Theorie und einer Reinheit von 80 % (GC).

### Beispiel (XXV-1)

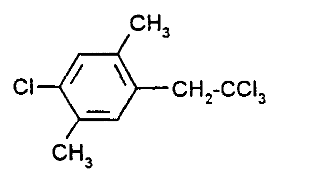

Zur gut gekühlten Mischung von 229,7 g (2,272 Mol) tert.-Butylnitrit und 254,8 g (1,775 Mol) wasserfreiem Kupfer-II-chlorid in 990 ml wasserfreiem Acetonitril tropft man 2205 g (22,7 Mol) 1,1-Dichlorethen, wobei man die Mischung auf unter 30°C hält. Dann wird bei einer Temperatur von unter 30°C eine Mischung aus 232 g (1,49 Mol) 4-Chlor-2,5-dimethylanilin und 1500 ml wasserfreiem Acetonitril zugetropft. Bei Raumtemperatur wird bis zum Ende der Gasentwicklung gerührt, die Mischung dann vorsichtig in 6 1 20 %ige HCl gegossen und mehrfach mit insgesamt 6 1 Methyl-tert.-butylether (MTBE) extrahiert. Der vereinigten organischen Phasen werden mit 20 %iger HCl gewaschen, getrocknet und eingeengt. Das zurückbleibende Öl wurde rektifiziert.
Ausbeute: 347 g dunkles Öl Δ 63 % der Theorie mit einem Gehalt von 74 % (GC).

### Beispiel (XXV-2)

Analog zu Beispiel (XXV-1) erhielt man 2-(2,4,5-Trichlorphenyl)-1,1,1-trichlorethan in Form eines dunklen Öls in einer Ausbeute von 81 % der Theorie und einem Gehalt von 78 %.

In Analogie zu Beispiel (XXV-1) erhält man die in der Tabelle 13 aufgeführten 1,1,1-Trichlor-2-phenyl-ethane der Formel (XXV).

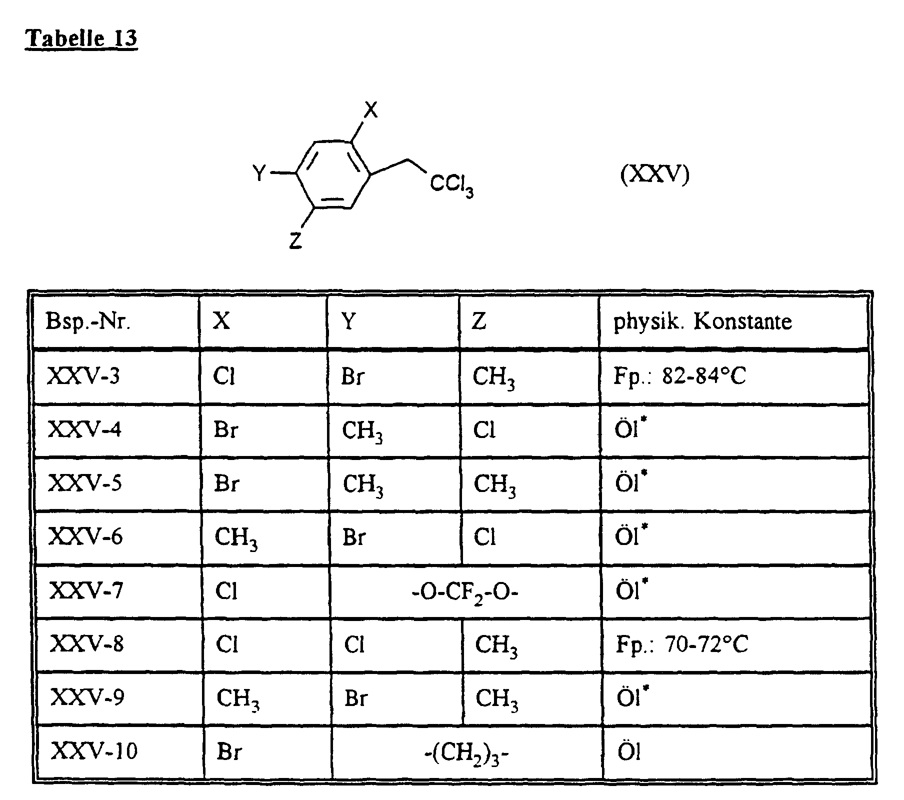

Die Öle wurden ohne weitere Reinigung zur Herstellung von Verbindungen der Formel (XXIV) verwendet. Die Verbindungen der Formel (XXV) sind neu, ausgenommen die Verbindung, in welcher X, Y Z = Cl.

### Synthese des 2,4,5-Trimethylphenylcarbonylketens

### 2,4,5-Trimethylphenylessigsäuremethylester

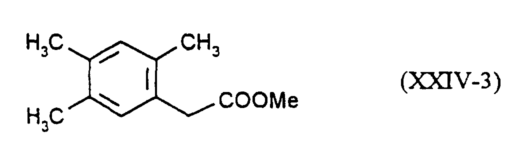

Es wurden 100 g (0,56 Mol) 2,4,5-Trimethylphenylessigsäure in 230 ml Methanol gelöst, mit 6 ml konzentrierter Schwefelsäure versetzt und 10 Stunden zum Rückfluß erhitzt. Anschließend wurde auf Raumtemperatur abgekühlt und das Methanol im Vakuum entfernt. Der Rückstand wurde in eine Lösung aus 53 g Natriumcarbonat, gelöst in 260 ml Wasser, eingerührt und die organische Phase in 200 ml Toluol aufgenommen. Die organische Phase wurde abgetrennt, getrocknet, eingeengt und destilliert. Es wurden 48,6 g 2,4,5-Trimethylphenylessigsäuremethylester mit einem Siedepunkt von 86°C bei 0,2 mbar erhalten.

### 2-(2,4,5-Trimethylphenyl)-malonsäuredimethylester

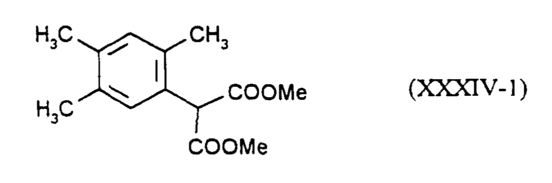

Es wurden 18,6 g (0,62 Mol, 80 %ig) Natriumhydrid in 384 ml Dimethylcarbonat vorgelegt und auf ca. 80°C erwärmt. Anschließend wurden 48 g (0,25 Mol) 2,4,5-Trimethylphenylessigsauremethylester, gelöst in 100 ml Dimethylcarbonat, zugetropft und 4 Stunden zum Rückfluß erhitzt. Die Mischung wurde auf Raumtemperatur abgekühlt, das überschüssige Natriumhydrid mit Ethanol vernichtet und danach in 1500 ml Eiswasser gegossen. Mit 6 N Salzsäure wurde ein pH-Wert von 4 bis 5 eingestellt und die organische Phase mit Toluol aufgenommen. Die organische Phase wurde abgetrennt, getrocknet, eingeengt und ohne weitere Reinigung in die nächste Stufe eingesetzt. Es wurden 51,6 g 2-(2,4,5-Trimethylphenyl)-malonsäuredimethylester als Öl erhalten.
¹H-NMR (CDCl₃): δ = 7.12 (s, 1H); 6.98 (s, 1H); 4.84 (s, 1H); 3.75 (s, 6H); 2.27 (s, 3H); 2.23 (s, 3H); 2,21 ppm (s, 3H).

### 2-(2,4,5-Trimethylphenyl)-malonsäure

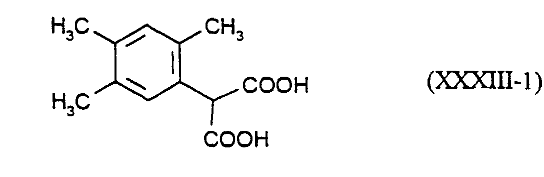

Zu einer Mischung aus 180 ml Methanol und 38,1 g (0,68 Mol) Kaliumhydroxid, gelöst in 92 ml Wasser, wurden bei Raumtemperatur 51,6 g (0,21 Mol) 2-(2,4,5-Trimethylphenyl)-malonsäuredimethylester zugetropft. Anschließend wurde fünf Stunden zum Rückfluß erhitzt, danach wieder auf Raumtemperatur abgekühlt und eingeengt.

Der Rückstand wurde in Eiswasser eingerührt und mit wenig Toluol gewaschen. Die wäßrige Lösung wurde unter Eiskühlung mit konzentrierter Salzsäure auf einen pH-Wert von 1 angesäuert, der Niederschlag abgesaugt und getrocknet. Es wurden 30,3 g 2-(2,4,5-Trimethylphenyl)-malonsäure erhalten.
¹H-NMR (d₆-DMSO): δ = 7.03 (s, 1H); 6.95 (s, 1H); 4.66 (s, 1H); 2.17 (s, 3H); 2.16 ppm (s, 6H).

### 2,4,5-Trimethylphenylchlorcarbonylketen

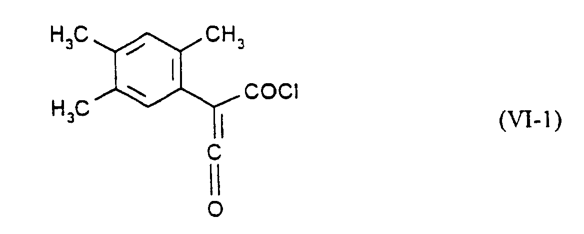

Es wurden 30 g 2-(2,4,5-Trimethylphenyl)-malonsäure in 60 ml Toluol bei 50 bis 60°C suspendiert und tropfenweise mit 62,5 ml Thionylchlorid versetzt. Die Mischung wurde anschließend 15 Stunden auf 90 bis 100°C erhitzt. Danach wurde abgekühlt, die flüchtigen Bestandteile mit Schutzgas ausgetrieben und das überschüssige Thionylchlorid abdestilliert. Als Rückstand wurden 30,6 g 2,4,5-Trimethylphenylchlorcarbonylketen isoliert.
¹H-NMR (CDCl₃): δ = 7.07 (s, 2H); 2.27 (s, 3H); 2.22 (s, 3H); 2.21 ppm (s, 3H).

### Anwendungsbeispiele:

### Beispiel A

### Phaedon-Larven-Test

- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden, 0 % bedeutet, daß keine Käfer-Larven abgetötet wurden.

In diesem Test bewirkten z.B. die Verbindungen gemäß den Herstellungsbeispielen I-4-b-1 und I-4-a-4 bei einer beispielhaften Wirkstoffkonzentration von 0,1 % eine Abtötung von 100 % nach 7 Tagen.

### Beispiel B

### Plutella-Test

- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupen abgetötet wurden, 0 % bedeutet, daß keine Raupen abgetötet wurden.

In diesem Test bewirkten z.B. die Verbindungen gemaß den Herstellungsbeispielen I-1-a-1, I-1-b-2, I-1-c-1, I-4-a-3, I-4-a-1, I-4-b-1 und I-4-a-4 bei einer beispielhaften Wirkstoffkonzentration von 0,1 % eine Abtotung von 100 % nach 7 Tagen

### Beispiel C

### Spodoptera-Test

- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden, 0 % bedeutet, daß keine Raupen abgetötet wurden.

In diesem Test bewirkten z.B. die Verbindungen gemäß den Herstellungsbeispielen I-1-a-1, I-1-b-2, I-4-a-3, 1-4-a-1, 1-4-b-1 und I-4-a-4 bei einer beispielhaften Wirkstoffkonzentration von 0,1 % eine Abtötung von 100 % nach 7 Tagen.

### Beispiel D

### Nephotettix-Test

- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeilinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden, 0 % bedeutet, daß keine Zikaden abgetötet wurden.

In diesem Test bewirkten z.B. die Verbindungen gemäß den Herstellungsbeispielen I-2-a-2, I-2-a-3, I-2-b-4, I-2-b-5, I-1-a-1, I-1-b-2, I-1-c-1, I-1-b- I-1-b-3, I-1-c-2, I-1-c-3, I-4-a-3 und I-4-a-4 bei einer beispielhaften Wirkstoffkonzentration von 0,1 % eine Abtötung von 100 % nach 6 Tagen.

### Beispiel E

### Myzus-Test

- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden, 0 % bedeutet, daß keine Blattlause abgetötet wurden.

In diesem Test bewirkten z.B. die Verbindungen gemäß den Herstellungsbeispielen I-2-a-1, I-2-b-4, I-1-b-2, I-1-b-3, I-1-c-2 und I-4-a-4 bei einer beispielhaften Wirkstoffkonzentration von 0,1 % eine Abtotung von 100 % nach 6 Tagen

### Beispiel F

**Tetranychus-Test** (OP-resistent/Tauchbehandlung)
- Lösungsmittel:: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden, 0 % bedeutet, daß keine Spinnmilben abgetötet wurden.

In diesem Test bewirkten z.B. die Verbindungen gemäß den Herstellungsbeispielen I-2-b-2, I-2-b-1, I-1-b-2 und I-4-b-1 bei einer beispielhaften Wirkstoffkonzentration von 0,1 % eine Abtötung von mindestens 98 % nach 13 Tagen.

### Beispiel G

### Pre-emergence-Test

- Lösungsmittel:: 5 Gewichtsteile Aceton
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test bewirkte z.B. die Verbindung gemäß den Herstellungsbeispiel I-4-a-3 bei einer beispielhaften Aufwandmenge von 500 g/ha und guter Verträglichkeit durch Beta vulgaris eine Schädigung von 90 % bei Alopecurus myosuroides, 96 % bei Avena fatua und 95 % bei Setaria viridis.

### Beispiel H

### Test mit Fliegenlarven / Entwicklungshemmende Wirkung

- Testtiere:: Alle larvalen Stadien von Lucilia cuprina (OP-resistent)
[Puppen und Adulte (ohne Kontakt zum Wirkstoff)]
- Lösungsmittel:: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm3) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1 1/2-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Flohentwicklung, bzw. ein Entwicklungsstillstand vor dem Adulten-Stadium. Dabei bedeutet 100 % larvizide Wirkung, daß nach 48 Stunden alle Larven abgestorben sind. 100% entwicklungsinhibitorische Wirkung bedeutet, daß keine adulte Fliegen geschlüpft sind.

In diesem Test bewirkte z.B. die Verbindung gemäß Herstellungsbeispiel I-4-a-1 bei einer beispielhaften Wirkstoffkonzentration von 1000 ppm eine Wirkung von 100 %.

### Beispiel I

### Test mit Boophilus microplus resistent/SP-resistenter Parkhurst-Stamm

- Testtiere:: adulte gesogene Weibchen
- Lösungsmittel:: Dimethylsulfoxid

20 mg Wirkstoff werden in 1 ml Dimethylsulfoxid gelöst, geringere Konzentrationen werden durch Verdünnen in dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test hatten z.B. die Verbindungen gemäß den Herstellungsbeispielen I-4-a-1 und I-4-b-1 bei einer beispielhaften Wirkstoffkonzentration von 20 µg pro Tier eine Wirkung von 100 %.

## Patentansprüche

1. Verbindungen der Formel (I) 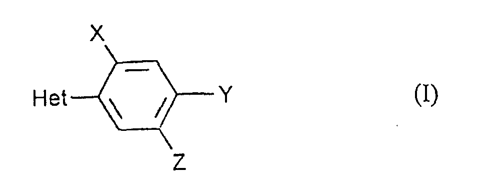 in welcher
X für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy, Cyano oder Nitro steht,
Y für Halogen, C₁-C₆-Alkyl, C₁-C₆-Alkoxy, C₁-C₄-Halogenalkyl, C₁-C₄-Halogenalkoxy, Cyano oder Nitro steht,
Z für Halogen, C₁-C₆-Alkyl, C₁-C₆-Alkoxy, C₁-C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht, oder
Y und Z gemeinsam für ein gegebenenfalls durch Halogen, C₁-C₆-Alkyl, C₁-C₆-Alkoxy oder C₁-C₄-Halogenalkyl substituiertes C₃-C₄-Alkandiyl oder C₃-C₄-Alkendiyl stehen, in welchen gegebenenfalls ein bis drei Glieder durch Sauerstoff, Schwefel, Stickstoff oder eine Carbonylgruppe unabhängig voneinander ersetzt sein können, wobei X eine der obengenannten Bedeutungen hat.
Het für eine der Gruppen 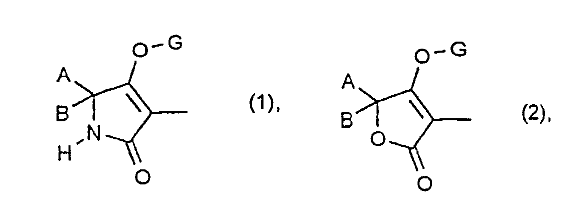 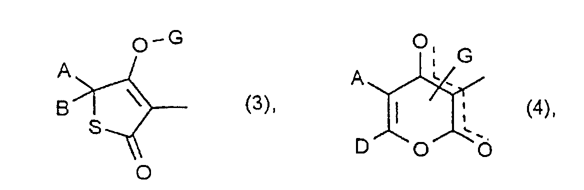 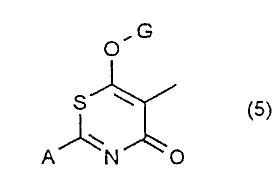 steht,
A für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Phenyl-C₁-C₆alkyl, Naphthyl-C₁-C₆-alkyl oder Hetaryl mit 5 oder 6 Ringatomen und ein bis drei Heteroatomen aus der Reihe Sauerstoff, Schwefel und Stickstoff steht,
B für Wasserstoff, C₁-C₁₂-Alkyl oder C₁-C₈-Alkoxy-C₁-C₆-alkyl oder steht,
A, B und das Kohlenstoffatom an das sie gebunden sind, für C₃-C₁₀-Cycloalkyl oder C₅-C₁₀-Cycloalkenyl stehen, worin gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
A, B und das Kohlenstoffatom, an das sie gebunden sind, für C₅-C₆-Cycloalkyl stehen, welches durch eine gegebenenfalls ein oder zwei Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind für C₃-C₈-Cycloalkyl oder C₅-C₈-Cycloalkenyl stehen, in dem zwei Substituenten gemeinsam mit Kohlenstoffatomen, an die sie gebunden sind, für jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl stehen, worin jeweils gegebenenfalls eine Methylengruppe durch Sauerstoff oder Schwefel ersetzt ist,
D für Wasserstoff, für jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈-alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 bis 6 Ringatomen und ein oder zwei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein oder zwei Heteroatomen aus der Reihe Sauerstoff, Schwefel und Stickstoff steht oder
A und D gemeinsam für eine C₃-C₆-Alkandiyl-, C₃-C₆-Alkendiyl- oder C₄-C₆-Alkadiendiylgruppe stehen, in welchen jeweils gegebenenfalls eine Methylengruppe durch Sauerstoff oder Schwefel ersetzt ist und welche jeweils gegebenenfalls substituiert sind durch Halogen oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy oder durch eine weitere, einen ankondensierten Ring bildende C₃-C₆-Alkandiyl-, C₃-C₆-Alkendiyl- oder C₄-C₆-Alkadiendiylgruppe, in welchen gegebenenfalls jeweils eine Methylengruppe durch Sauerstoff oder Schwefel ersetzt ist und welche gegebenenfalls durch C₁-C₆-Alkyl substituiert sind, oder
A und D gemeinsam für eine C₃-C₆-Alkandiyl- oder C₃-C₆-Alkendiylgruppe stehen, worin jeweils gegebenenfalls eine der folgenden Gruppen 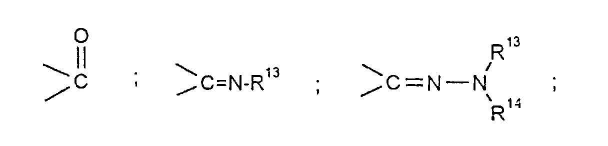 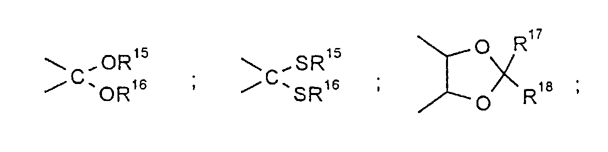 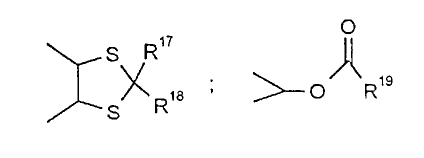 oder 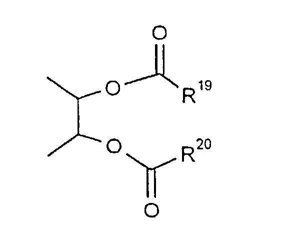
enthalten ist,
G für Wasserstoff (a) oder für eine der Gruppen 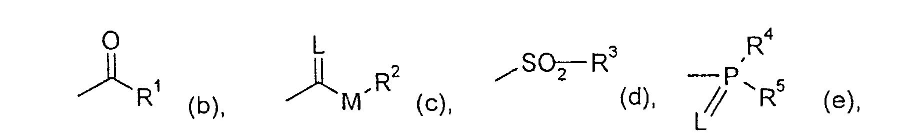
E (f) oder 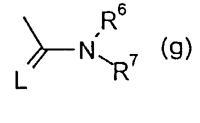 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5-oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl odr Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist,
R¹³ für Wasserstoff oder jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy steht,
R¹⁴ für Wasserstoff oder C₁-C₈-Alkyl steht oder
R¹³ und R¹⁴ gemeinsam für C₄-C₆-Alkandiyl stehen,
R¹⁵ und R¹⁶ gleich oder verschieden sind und für C₁-C₆-Alkyl stehen oder
R¹⁵ und R¹⁶ gemeinsam für einen C₂-C₄-Alkandiylrest stehen, der gegebenenfalls durch C₁-C₆-Alkyl oder durch gegebenenfalls durch Halogen, C₁-C₄-Alkyl, C₁-C₄-Halogenalkyl, C₁-C₄-Alkoxy, C₁-C₄-Halogenalkoxy, Nitro oder Cyano substituiertes Phenyl substituiert ist,
R¹⁷ und R¹⁸ unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halagenalkyl, C₁-C₄-Halogenalkoxy, Nitro oder Cyano substituiertes Phenyl stehen oder
R¹⁷ und R¹⁸ gemeinsam mit dem Kohlenstoffatom, an das sie gebunden sind, für gegebenenfalls durch C₁-C₄-Alkyl substituiertes C₅-C₇-Cycloalkyl stehen, in dem gegebenenfalls eine Methylengruppe durch Sauerstoff oder Schwefel ersetzt ist,
R¹⁹ und R²⁰ unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

2. Verbindungen der Formel (I) gemäß Anspruch 1, in welcher
X für Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht,
Y für Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht,
Z für Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy oder Benzyloxy oder
Y und Z gemeinsam für gegebenenfalls durch Fluor, Chlor, Brom, C₁-C₄-Alkyl, C₁-C₄-Alkoxy oder C₁-C₂-Halogenalkyl substituiertes C₃-C₄-Alkandiyl oder C₃-C₄-Alkendiyl stehen, in welchem gegebenenfalls ein oder zwei nicht direkt benachbarte Glieder durch Sauerstoff, Schwefel oder Stickstoff unabhängig voneinander ersetzt sein können, wobei X eine der obengenannten Bedeutungen hat,
Het für eine der Gruppen 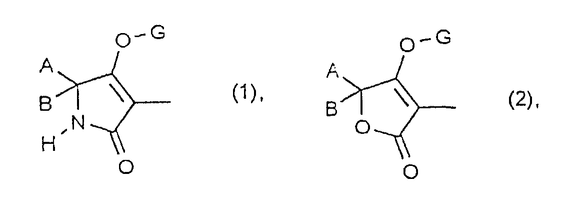 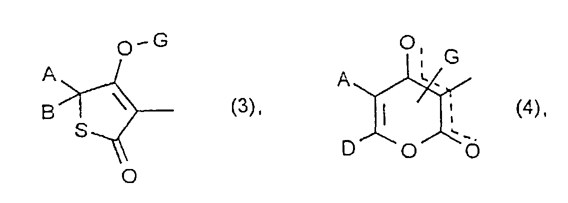 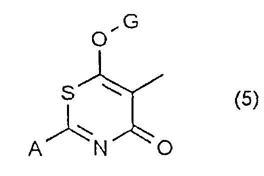 steht,
A für Wasserstoff, für jeweils gegebenenfalls durch Fluor oder Chlor substituiertes C₁-C₁₀-Alkyl, C₂-C₆-Alkenyl, C₁-C₈-Alkoxy-C₁-C₆-alkyl, Poly-C₁-C₆-alkoxy-C₁-C₆-alkyl oder C₁-C₈-Alkylthio-C₁-C₆-alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl steht,
B für Wasserstoff, C₁-C₁₀-Alkyl oder C₁-C₆-Alkoxy-C₁-C₄-alkyl steht oder
A, B und das Kohlenstoffatom an das sie gebunden sind, für C₃-C₈-Cycloalkyl oder C₅-C₈-Cycloalkenyl stehen, worin jeweils gegebenenfalls eine Methylengruppe durch Sauerstoff oder Schwefel ersetzt ist und welche gegebenenfalls durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl, substituiert sind oder
A, B und das Kohlenstoffatom, an das sie gebunden sind, für C₅-C₆-Cycloalkyl stehen, welches durch eine gegebenenfalls ein oder zwei Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- bis siebengliedrigen Ring bildet oder
A, B und das Kohlenstoffatom, an das sie gebunden sind, für C₃-C₆-Cycloalkyl oder C₅-C₆-Cycloalkenyl stehen, in dem zwei Substituenten gemeinsam mit den Kohlenstoffatomen, an die sie gebunden sind, für jeweils gegebenenfalls durch C₁-C₄-Alkyl, C₁-C₄-Alkoxy, Fluor, Chlor oder Brom substituiertes C₃-C₅-Alkandiyl, C₃-C₅-Alkendiyl oder Butadiendiyl stehen, worin jeweils gegebenenfalls eine Methylengruppe durch Sauerstoff oder Schwefel ersetzt ist,
D für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl steht oder
A und D gemeinsam für eine C₃-C₅-Alkandiyl- oder C₃-C₅-Alkendiylgruppe stehen, worin jeweils gegebenenfalls ein Kohlenstoffatom durch Sauerstoff oder Schwefel ersetzt ist und welche gegebenenfalls substituiert sind durch Fluor, Chlor oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy oder
worin jeweils gegebenenfalls eine der folgenden Gruppen: 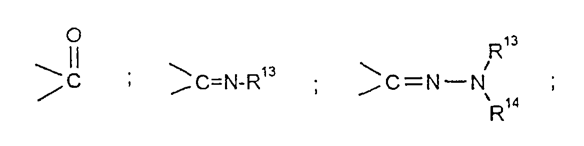 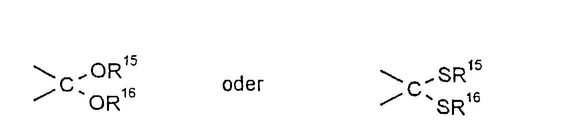
enthalten ist;
G für Wasserstoff (a) oder für eine der Gruppen 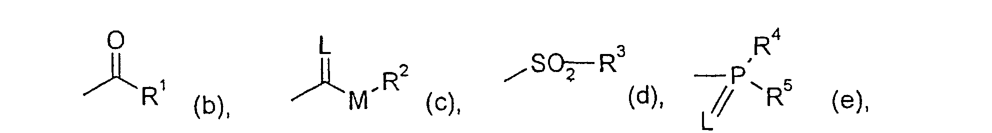
E (f) oder 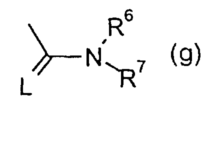 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls durch Fluor oder Chlor substituiertes C₁-C₆-Alkyl oder jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen,
R⁶ und R⁷ unabhängig voneinander für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist,
R¹³ für Wasserstoff oder jeweils gegebenenfalls durch Fluor oder Chlor substituiertes C₁-C₆-Alkyl oder C₁-C₆-Alkoxy, für gegebenenfalls durch Fluor, C₁-C₂-Alkyl oder C₁-C₂-Alkoxy substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₅-Alkyl, C₁-C₅-Alkoxy, C₁-C₂-Halogenalkyl, C₁-C₂-Halogenalkoxy, Nitro oder Cyano substituiertes Phenyl, Phenyl-C₁-C₃-alkyl oder Phenyl-C₁-C₂-alkyloxy steht,
R¹⁴ für Wasserstoff oder C₁-C₆-alkyl steht oder
R¹³ und R¹⁴ gemeinsam für C₄-C₆-Alkandiyl stehen,
R¹⁵ und R¹⁶ gleich oder verschieden sind und für C₁-C₄-Alkyl stehen oder
R¹⁵ und R¹⁶ zusammen für einen C₂-C₃-Alkandiylrest stehen, der gegebenenfalls durch C₁-C₄-Alkyl oder durch gegebenenfalls durch Fluor, Chlor, Brom, C₁-C₂-Alkyl, C₁-C₂-Halogenalkyl, C₁-C₂-Alkoxy, C₁-C₂-Halogenalkoxy, Nitro oder Cyano substituiertes Phenyl substituiert ist.

3. Verbindungen der Formel (I) gemäß Anspruch 1, in welcher
X für Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro steht,
Y für Fluor, Chlor, Brom, Methyl, Ethyl, Propyl, iso-Propyl, Butyl, iso-Butyl, tert.-Butyl, Methoxy, Ethoxy, Propoxy, iso-Propoxy, Trifluormethyl, Trifluormethoxy, Difluormethoxy, Cyano oder Nitro steht,
Z für Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro steht, oder
Y und Z gemeinsam für gegebenenfalls durch Fluor, Chlor, Methyl, Ethyl, Propyl, iso-Propyl, Methoxy, Ethoxy, Propoxy, iso-Propoxy oder Trifluormethyl substituiertes C₃-C₄-Alkandiyl stehen, in welchem gegebenenfalls zwei nicht direkt benachbarte Glieder durch Sauerstoff ersetzt sind, wobei X eine der obengenannten Bedeutungen hat,
Het für eine der Gruppen 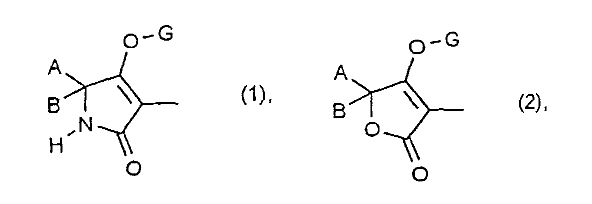 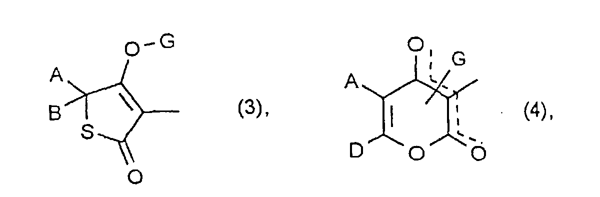 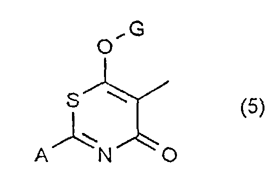 steht,
A für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄alkyl, Poly-C₁-C₄-alkoxy-C₁-C₄-alkyl oder C₁-C₆-Alkylthio-C₁-C₄alkyl, oder für gegebenenfalls durch Fluor, Chlor, Methyl oder Methoxy substituiertes C₃-C₆-Cycloalkyl, in welchem gegebenenfalls eine oder zwei Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Pyridyl oder Benzyl steht,
B für Wasserstoff, C₁-C₈-Alkyl oder C₁-C₄-Alkoxy-C₁-C₂-alkyl steht oder
A, B und das Kohlenstoffatom an das sie gebunden sind, für C₃-C₈-Cycloalkyl oder C₅-C₈-Cycloalkenyl stehen, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
A, B und das Kohlenstoffatom, an das sie gebunden sind, für C₅-C₆-Cycloalkyl stehen, welches durch eine gegebenenfalls ein Sauerstoff- oder Schwefelatom enthaltende Alkylendiyl- oder durch eine Alkylendioxyl-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind, für C₃-C₆-Cycloalkyl oder C₃-C₆-Cycloalkenyl stehen, in dem zwei Substituenten gemeinsam mit den Kohlenstoffatomen, an die sie gebunden sind, für C₃-C₄-Alkandiyl, C₃-C₄-Alkendiyl oder Butadiendiyl stehen, worin jeweils gegebenenfalls eine Methylengruppe durch Sauerstoff oder Schwefel ersetzt ist,
D für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆-Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl steht,
oder
A und D gemeinsam für eine C₃-C₅-Alkandiyl- oder C₃-C₅-Alkendiylgruppe stehen, worin jeweils gegebenenfalls eine Methylengruppe durch Sauerstoff oder Schwefel ersetzt ist und welche gegebenenfalls durch Fluor, Chlor oder durch jeweils gegebenenfalls durch Fluor oder Chlor substituiertes C₁-C₆-Alkyl, C₁-C₄-Alkoxy, C₁-C₄-Alkylthio, C₃-C₆-Cycloalkyl, Phenyl oder Benzyloxy substituiert sind,
G für Wasserstoff (a) oder für eine der Gruppen 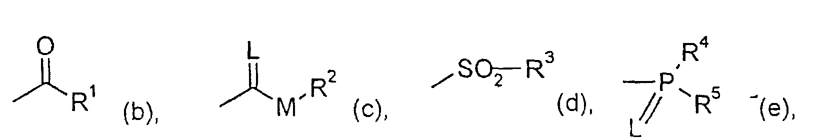
E (f) oder 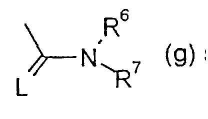 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonvl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄alkyl oder Thiazolyloxy-C₁-C₄-alkyl steht,
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für gegebenenfalls durch Fluor oder Chlor substituiertes Methyl, Ethyl, Propyl, iso-Propyl, Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebenenfalls eine Methylengruppe durch Sauerstoff oder Schwefel ersetzt ist.

4. Verfahren zur Herstellung von Verbindungen der Formel (I) gemäß Anspruch 1, **dadurch gekennzeichnet, daß** man zum Erhalt von
(A) Verbindungen der Formel (I-1-a) 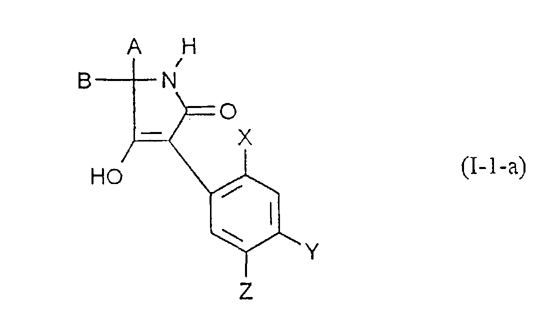 in welcher
A, B, X, Y und Z die in Anspruch 1 angegebenen Bedeutungen haben,
Verbindungen der Formel (II) 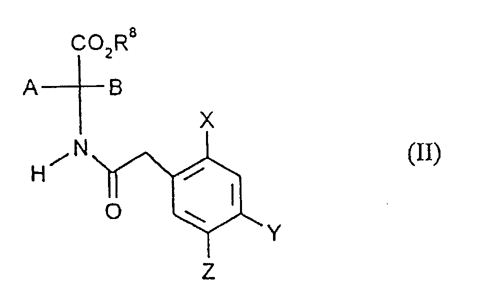 in welcher
A, B, X, Y und Z die in Anspruch 1 angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 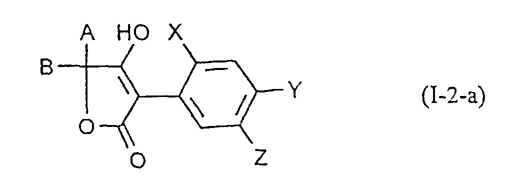 in welcher
A, B, X, Y und Z die oben angegebenen Bedeutungen haben,
Verbindungen der Formel (III) 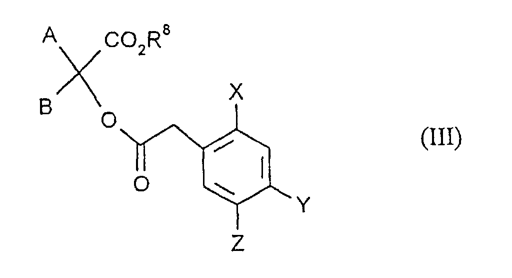 in welcher
A, B, X, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-3-a) 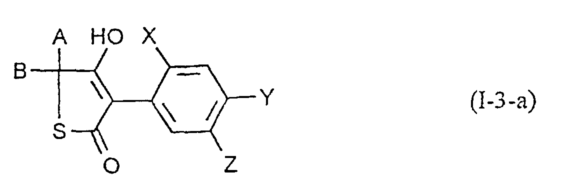 in welcher
A, B, X, Y und Z die oben angegebenen Bedeutungen haben,
Verbindungen der Formel (IV) 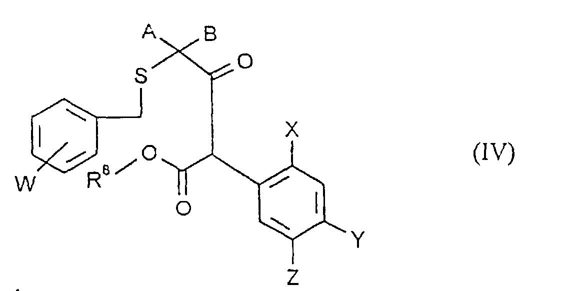 in welcher
A, B, X, Y, Z und R⁸ die oben angegebenen Bedeutungen haben und
W für Wasserstoff, Halogen, Alkyl oder Alkoxy steht,
gegebenenfalls in Gegenwart eines Verdünnungsmittels und in Gegenwart einer Säure intramolekular cyclisiert,
(D) Verbindungen der Formel (I-4-a) 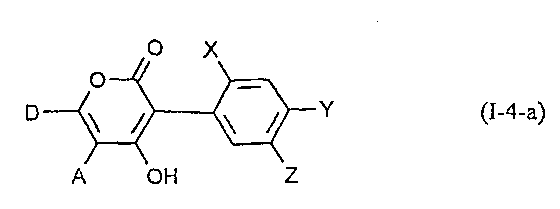 in welcher
A, D, X, Y und Z die in Anspruch 1 angegebenen Bedeutungen haben,
Verbindungen der Formel (V) 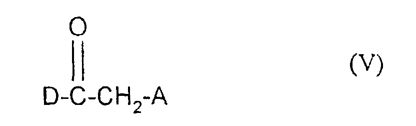 in welcher
A und D die oben angegebenen Bedeutungen haben,
oder deren Silylenolether der Formel (Va) 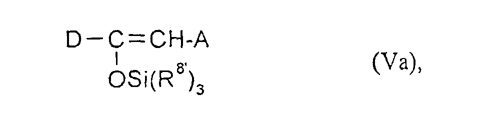 in welcher A und D die oben genannte Bedeutung haben und
R^{8'} für Alkyl steht,
mit Verbindungen der Formel (VI) 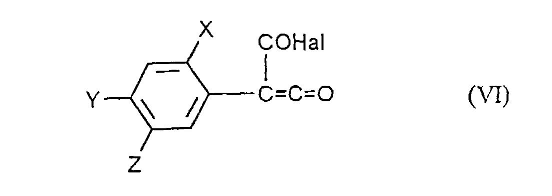 in welcher
X, Y und Z die oben angegebenen Bedeutungen haben und
Hal für Halogen steht,
gegebenenfalls in Gegenwart eines Verdünnungsmittels und gegebenenfalls in Gegenwart eines Säureakzeptors umsetzt,
(E) Verbindungen der Formel (I-5-a) 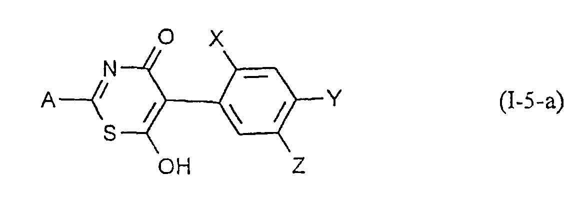 in welcher
A, X, Y und Z die oben angegebenen Bedeutungen haben,
Verbindungen der Formel (VII) 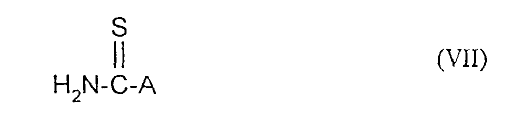 in welcher
A die oben angegebene Bedeutung hat,
mit Verbindungen der Formel (VI) 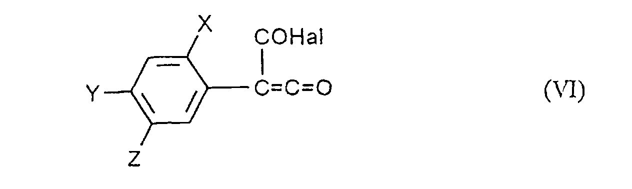 in welcher
Hal, X, Y und Z die oben angegebenen Bedeutungen haben,
gegebenenfalls in Gegenwart eines Verdünnungsmittels und gegebenenfalls in Gegenwart eines Säureakzeptors umsetzt, und gegebenenfalls anschließend die so erhaltenen Verbindungen der oben gezeigten Formeln (I-1-b) bis (I-5-b)
(F)
α) mit Säurehalogeniden der Formel (VIII) 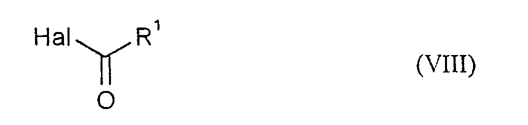 in welcher
R¹ die in Anspruch 1 angegebene Bedeutung hat und
Hal für Halogen steht
oder
β) mit Carbonsäureanhydriden der Formel (IX)
R¹-CO-O-CO-R¹ (IX)
in welcher
R¹ die oben angegebene Bedeutung hat,
gegebenenfalls in Gegenwart eines Verdünnungsmittels und gegebenenfalls in Gegenwart eines Säurebindemittels umsetzt,
oder
(G) mit Chlorameisensäureestern oder Chlorameisensäurethioestern der Formel (X)
R²-M-CO-Cl (X)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt,
oder
(H)
α) mit Chlormonothioameisensäureestern oder Chlordithioameisensäureestern der Formel (XI) 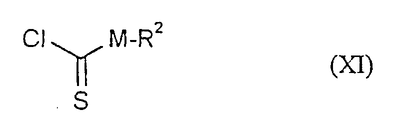 in welcher
M und R² die oben angegebenen Bedeutungen haben,
gegebenenfalls in Gegenwart eines Verdünnungsmittels und gegebenenfalls in Gegenwart eines Säurebindemittels umsetzt,
oder
β) mit Schwefelkohlenstoff und anschließend mit Verbindungen der Formel (XII)
R²-Hal (XII)
in welcher
R² die oben angegebene Bedeutung hat und
Hal für Chlor, Brom oder Iod steht,
gegebenenfalls in Gegenwart eines Verdünnungsmittels und gegebenenfalls in Gegenwart einer Base umsetzt,
oder
(I) mit Sulfonsäurechloriden der Formel (XIII)
R³-SO₂-Cl (XIII)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt,
oder
(J) mit Phosphorverbindungen der Formel (XIV) 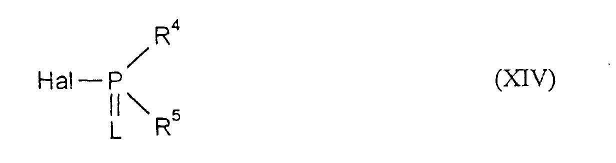 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
oder
(K) mit Metallverbindungen oder Aminen der Formeln (XV) oder (XVI)
Me(OR¹⁰)ₜ (XV) 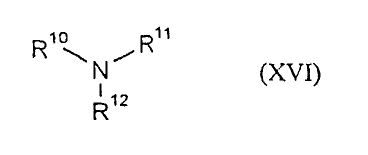
in welchen
Me für ein ein- oder zweiwertiges Metall
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
oder
(L)α) mit Isocyanaten oder Isothiocyanaten der Formel (XVII)
R⁶-N=C-L (VXII)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XVIII) 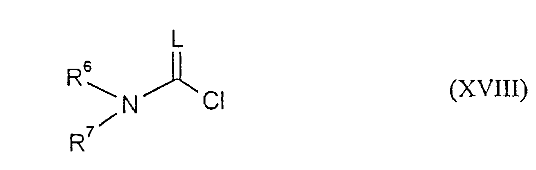 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (II) 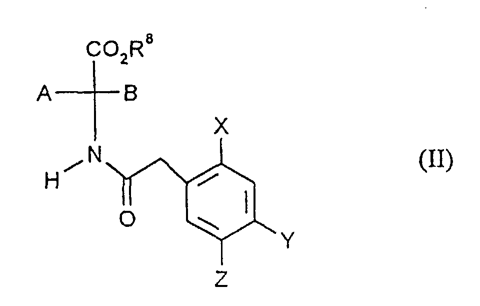 in welcher
A, B, X, Y und Z die in Anspruch 1 angegebenen Bedeutungen haben und
R⁸ für Alkyl steht.

6. Verbindungen der Formel (III) 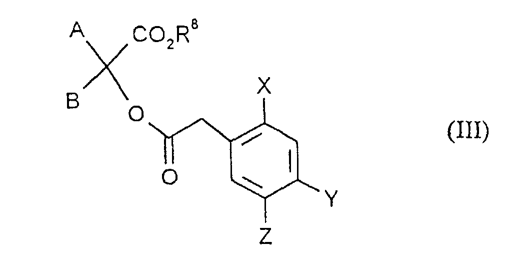 in welcher
A, B, X, Y und Z die in Anspruch 1 angegebenen Bedeutungen haben und
R⁸ für Alkyl steht.

7. Verbindungen der Formel (XXI) 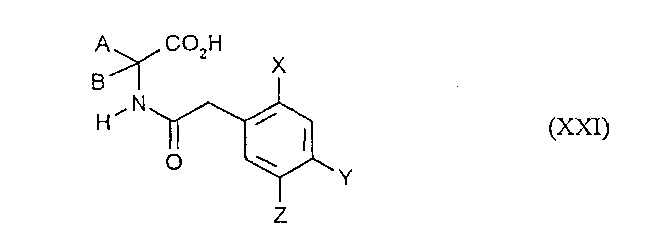 in welcher
A, B, X, Y und Z die in Anspruch 1 angegebenen Bedeutungen haben.

8. Verbindungen der Formel (XXIX) 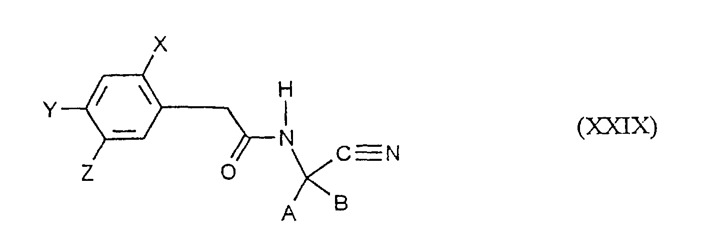 in welcher
A, B, X, Y und Z die in Anspruch 1 angegebenen Bedeutungen haben.

9. Verbindungen der Formel (IV) 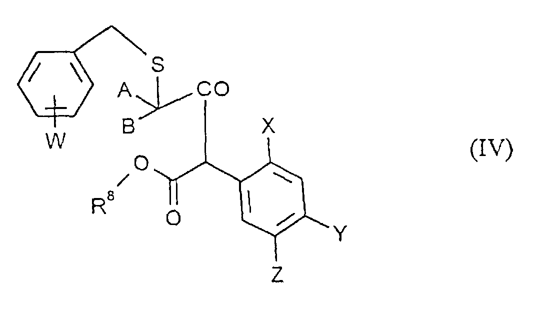 in welcher
A, B, X, Y und Z die in Anspruch 1 angegebenen Bedeutungen haben,
R⁸ für Alkyl steht und
W für Wasserstoff, Halogen, Alkyl oder Alkoxy steht.

10. Verbindungen der Formel (VI) 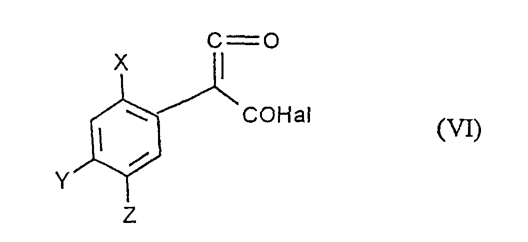 in welcher
X, Y und Z die in Anspruch 1 angegebenen Bedeutungen haben und
Hal für Chlor oder Brom steht.

11. Verbindungen der Formel (XXXIII) 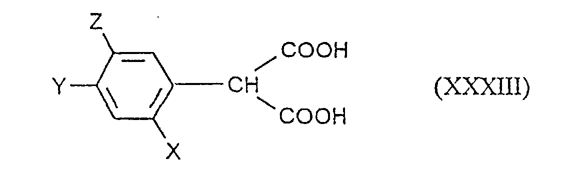 in welcher
X, Y und Z die in Anspruch 1 angegebenen Bedeutungen haben.

12. Verbindungen der Formel (XXXIV) 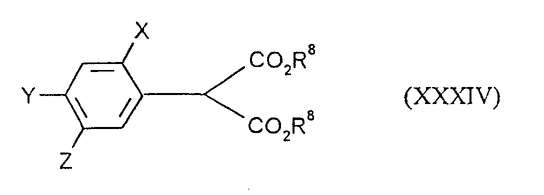 in welcher
X, Y und Z die in Anspruch 1 angegebenen Bedeutungen haben und
R⁸ für Alkyl steht.

13. Schädlingsbekämpfungsmittel und Herbizide, **gekennzeichnet durch** einen Gehalt an mindestens einer Verbindung der Formel (I) gemäß Anspruch 1.

14. Verwendung von Verbindungen der Formel (I) gemäß Anspruch 1 zur Bekämpfung von Schädlingen und unerwünschtem Pflanzenbewuchs.

15. Verfahren zur Bekämpfung von Schädlingen, **dadurch gekennzeichnet, daß** man Verbindungen der Formel (I) gemäß Anspruch 1 auf Schädlinge, unerwünschten Pflanzenbewuchs und/oder ihren Lebensraum einwirken läßt.

16. Verfahren zur Herstellung von Schädlingsbekämpfungsmitteln und Herbiziden, **dadurch gekennzeichnet, daß** man Verbindungen der Formel (I) gemäß Anspruch 1 mit Streckmitteln und/oder oberflächenaktiven Mitteln vermischt.

17. Verwendung von Verbindungen der Formel (I) gemäß Anspruch 1 zur Herstellung von Schädlungsbekämpfungsmitteln und Herbiziden.

## Claims

1. Compounds of the formula (I) 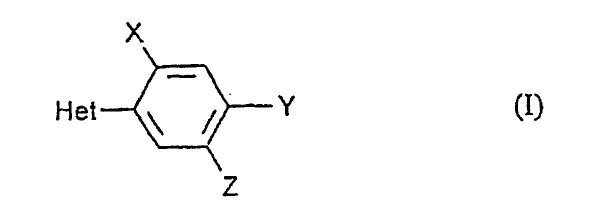 in which
X represents halogen, C₁-C₆-alkyl, C₂-C₆-alkenyl, C₂-C₆-alkinyl, C₁-C₆-alkoxy, C₁-C₄-halogenoalkyl, C₁-C₄-halogenoalkoxy, cyano or nitro,
Y represents halogen, C₁-C₆-alkyl, C₁-C₆-alkoxy, C₁-C₄-halogenoalkyl, C₁-C₄-halogenoalkoxy, cyano or nitro,
Z represents halogen, C₁-C₆-alkyl, C₁-C₆-alkoxy, C₁-C₄-halogenoalkyl, C₁-C₄-halogenoalkoxy, hydroxyl, cyano or nitro, or phenoxy, phenylthio, thiazolyloxy, pyridinyloxy, pyrimidyloxy, pyrazolyloxy, phenyl-C₁-C₄-alkyloxy or phenyl-C₁-C₄-alkylthio which are in each case optionally substituted by halogen, C₁-C₄-alkyl, C₁-C₄-alkoxy, C₁-C₄-halogenoalkyl, C₁-C₄-halogenoalkoxy, nitro or cyano, or
Y and Z together represent C₃-C₄-alkanediyl or C₃-C₄-alkenediyl which are optionally substituted by halogen, C₁-C₆-alkyl, C₁-C₆-alkoxy or C₁-C₄-halogenoalkyl and in which one to three members can optionally be replaced, independently of one another, by oxygen, sulphur, nitrogen or a carbonyl group, X having one of the abovementioned meanings,
Het represents one of the groups 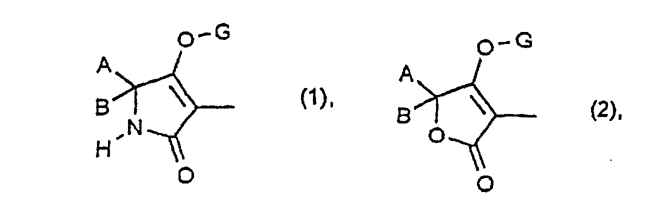 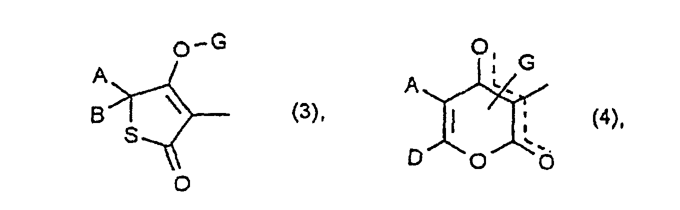 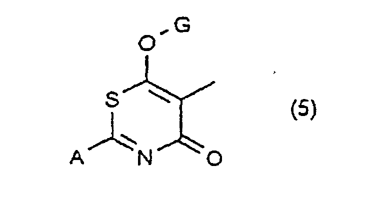
A represents hydrogen, or represents C₁-C₁₂-alkyl, C₂-C₈-alkenyl, C₁-C₁₀-alkoxy-C₁-C₈-alkyl, poly-C₁-C₈-alkoxy-C₁-C₈-alkyl or C₁-C₁₀-alkylthio-C₁-C₆-alkyl which are in each case optionally substituted by halogen, or represents C₃-C₈-cycloalkyl which is optionally substituted by halogen, C₁-C₆-alkyl or C₁-C₆-alkoxy and in which one or two methylene groups are optionally replaced by oxygen and/or sulphur, or represents phenyl, naphthyl, phenyl-C₁-C₆-alkyl, naphthyl-C₁-C₆-alkyl or hetaryl having 5 or 6 ring atoms and one to three heteroatoms from the series consisting of oxygen, sulphur and nitrogen, which are in each case optionally substituted by halogen, C₁-C₆-alkyl, C₁-C₆-halogenoalkyl, C₁-C₆-alkoxy, C₁-C₆-halogenoalkoxy, cyano or nitro,
B represents hydrogen, C₁-C₁₂-alkyl or C₁-C₈-alkoxy-C₁-C₆-alkyl, or
A, B and the carbon atom to which they are bonded represent C₃-C₁₀-cycloalkyl or C₅-C₁₀-cycloalkenyl, wherein one methylene group is optionally replaced by oxygen or sulphur and which are optionally substituted by C₁-C₈-alkyl, C₃-C₁₀-cycloalkyl, C₁-C₈-halogenoalkyl, C₁-C₈-alkoxy, C₁-C₈-alkylthio, halogen or phenyl, or
A, B and the carbon atom to which they are bonded represent C₅-C₆-cycloalkyl which is substituted by an alkylenediyl group which optionally contains one or two oxygen and/or sulphur atoms or by an alkylenedioxyl group or by an alkylenedithioyl group, this substituent forming a further five- to eight-membered ring with the carbon atom to which it is bonded, or
A, B and the carbon atom to which they are bonded represent C₃-C₈-cycloalkyl or C₅-C₈-cycloalkenyl in which two substituents, together with the carbon atom to which they are bonded, represent C₃-C₆-alkanediyl, C₃-C₆-alkenediyl or C₄-C₆-alkanedienediyl which are in each case optionally substituted by C₁-C₆-alkyl, C₁-C₆-alkoxy or halogen and wherein in each case one methylene group is optionally replaced by oxygen or sulphur,
D represents hydrogen, or represents C₁-C₁₂-alkyl, C₃-C₈-alkenyl, C₃-C₈-alkinyl, C₁-C₁₀-alkoxy-C₂-C₈-alkyl, poly-C₁-C₈-alkoxy-C₂-C₈-alkyl or C₁-C₁₀-alkylthio-C₂-C₈-alkyl which are in each case optionally substituted by halogen, or represents C₃-C₈-cycloalkyl which is optionally substituted by halogen, C₁-C₄-alkyl, C₁-C₄-alkoxy or C₁-C₄-halogenoalkyl and in which one or two methylene groups which are not directly adjacent are optionally replaced by oxygen and/or sulphur, or represent phenyl, hetaryl having 5 to 6 ring atoms and one or two heteroatoms from the series consisting of oxygen, sulphur and nitrogen, phenyl-C₁-C₆-alkyl or hetaryl-C₁-C₆-alkyl having 5 to 6 ring atoms and one or two heteroatoms from the series consisting of oxygen, sulphur and nitrogen, which are in each case optionally substituted by halogen, C₁-C₆-alkyl, C₁-C₆-halogenoalkyl, C₁-C₆-alkoxy, C₁-C₆-halogenoalkoxy, cyano or nitro, or
A and D together represent a C₃-C₆-alkanediyl, C₃-C₆-alkenediyl or C₄-C₆-alkadienediyl group in which in each case one methylene group is optionally replaced by oxygen or sulphur and which are in each case optionally substituted by halogen or C₁-C₁₀-alkyl, C₁-C₆-alkoxy, C₁-C₆-alkylthio, C₃-C₇-cycloalkyl, phenyl or benzyloxy which are in each case optionally substituted by halogen, or by a further C₃-C₆-alkanediyl, C₃-C₆-alkenediyl or C₄-C₆-alkadienediyl group which forms a fused-on ring and in which in each case one methylene group is optionally replaced by oxygen or sulphur and which are optionally substituted by C₁-C₆-alkyl, or
A and D together represent a C₃-C₆-alkanediyl or C₃-C₆-alkenediyl group which in each case optionally contains one of the following groups 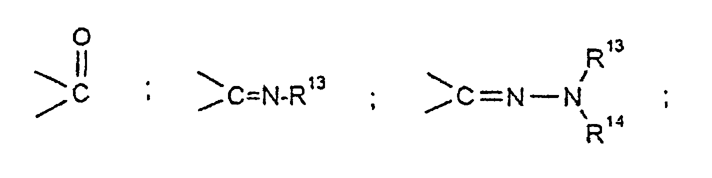 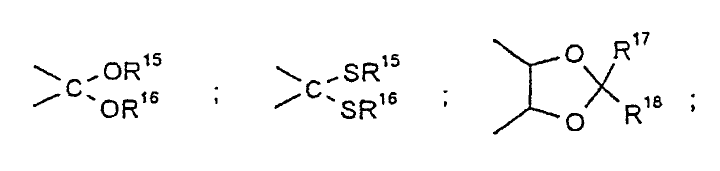 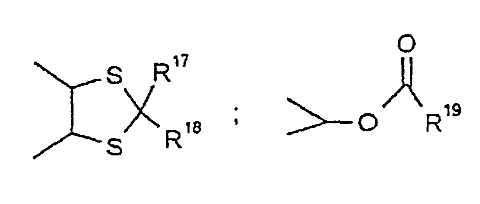 or 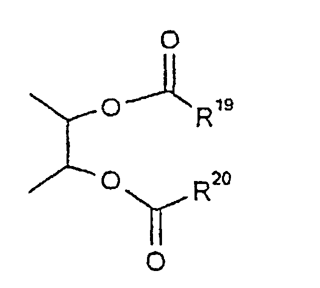
G represents hydrogen (a), or represents one of the groups 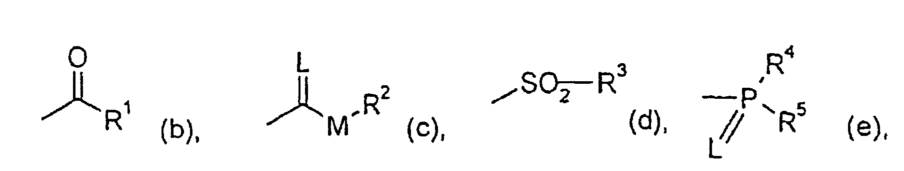
E (f) or 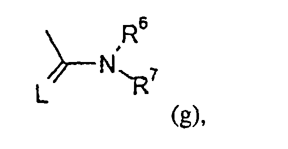 in which
E represents one metal ion equivalent or an ammonium ion,
L represents oxygen or sulphur and
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl which are in each case optionally substituted by halogen, or represents C₃-C₈-cycloalkyl which is optionally substituted by halogen, C₁-C₆-alkyl or C₁-C₆-alkoxy and in which one or two methylene groups are optionally replaced by oxygen and/or sulphur,
or represents phenyl which is optionally substituted by halogen, cyano, nitro, C₁-C₆-alkyl, C₁-C₆-alkoxy, C₁-C₆-halogenoalkyl, C₁-C₆-halogenoalkoxy, C₁-C₆-alkylthio or C₁-C₆-alkylsulphonyl,
or represents phenyl-C₁-C₆-alkyl which is optionally substituted by halogen, nitro, cyano, C₁-C₆-alkyl, C₁-C₆-alkoxy, C₁-C₆-halogenoalkyl or C₁-C₆-halogenoalkoxy,
or represents 5- or 6-membered hetaryl having one or two heteroatoms from the series consisting of oxygen, sulphur and nitrogen which is optionally substituted by halogen or C₁-C₆-alkyl,
or represents phenoxy-C₁-C₆-alkyl which is optionally substituted by halogen or C₁-C₆-alkyl or
represents 5- or 6-membered hetaryloxy-C₁-C₆-alkyl which has one or two heteroatoms from the series consisting of oxygen, sulphur and nitrogen and is optionally substituted by halogen, amino or C₁-C₆-alkyl,
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl which are in each case optionally substituted by halogen,
or represents C₃-C₈-cycloalkyl which is optionally substituted by halogen, C₁-C₆-alkyl or C₁-C₆-alkoxy, or
represents phenyl or benzyl which are in each case optionally substituted by halogen, cyano, nitro, C₁-C₆-alkyl, C₁-C₆-alkoxy, C₁-C₆-halogenoalkyl or C₁-C₆-halogenoalkoxy,
R³ represents C₁-C₈-alkyl which is optionally substituted by halogen, or phenyl or benzyl which are in each case optionally substituted by halogen, C₁-C₆-alkyl, C₁-C₆-alkoxy, C₁-C₄-halogenoalkyl, C₁-C₄-halogenoalkoxy, cyano or nitro,
R⁴ and R⁵ independently of one another represent C₁-C₈-alkyl, C₁-C₈-alkoxy, C₁-C₈-alkylamino, di-(C₁-C₈-alkyl)amino, C₁-C₈-alkylthio or C₃-C₈-alkenylthio which are in each case optionally substituted by halogen, or represent phenyl, phenoxy or phenylthio which are in each case optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl,
R⁶ and R⁷ independently of one another represent hydrogen, or represent C₁-C₈-alkyl, C₃-C₈-cycloalkyl, C₁-C₈-alkoxy, C₃-C₈-alkenyl or C₁-C₈-alkoxy-C₂-C₈-alkyl which are in each case optionally substituted by halogen, or represent phenyl or benzyl which are in each case optionally substituted by halogen, C₁-C₈-alkyl, C₁-C₈-halogenoalkyl or C₁-C₈-alkoxy, or together represent a C₃-C₆-alkylene radical which is optionally substituted by C₁-C₆-alkyl and in which one methylene group is optionally replaced by oxygen or sulphur,
R¹³ represents hydrogen, or C₁-C₈-alkyl or C₁-C₈-alkoxy which are in each case optionally substituted by halogen, or represents C₃-C₈-cycloalkyl which is optionally substituted by halogen, C₁-C₄-alkyl or C₁-C₄-alkoxy and in which one methylene group is optionally replaced by oxygen or sulphur, or represents phenyl, phenyl-C₁-C₄-alkyl or phenyl-C₁-C₄-alkoxy which are in each case optionally substituted by halogen, C₁-C₆-alkyl, C₁-C₆-alkoxy, C₁-C₄-halogenoalkyl, C₁-C₄-halogenoalkoxy, nitro or cyano,
R¹⁴ represents hydrogen or C₁-C₈-alkyl, or
R¹³ and R¹⁴ together represent C₄-C₆-alkanediyl,
R¹⁵ and R¹⁶ are identical or different and represent C₁-C₆-alkyl, or
R¹⁵ and R¹⁶ together represent a C₂-C₄-alkanediyl radical which is optionally substituted by C₁-C₆-alkyl or by phenyl which is optionally substituted by halogen, C₁-C₄-alkyl, C₁-C₄-halogenoalkyl, C₁-C₄-alkoxy, C₁-C₄-halogenoalkoxy, nitro or cyano,
R¹⁷ and R¹⁸ independently of one another represent hydrogen, or represent C₁-C₈-alkyl which is optionally substituted by halogen, or represent phenyl which is optionally substituted by halogen, C₁-C₆-alkyl, C₁-C₆-alkoxy, C₁-C₄-halogenoalkyl, C₁-C₄-halogenoalkoxy, nitro or cyano, or
R¹⁷ and R¹⁸, together with the carbon atom to which they are bonded, represent C₅-C₇-cycloalkyl which is optionally substituted by C₁-C₄-alkyl and in which one methylene group is optionally replaced by oxygen or sulphur,
R¹⁹ and R²⁰ independently of one another represent C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀-alkyl)amino or di-(C₃-C₁₀-alkenyl)amino.

2. Compounds of the formula (I) according to Claim 1, in which
X represents fluorine, chlorine, bromine, C₁-C₄-alkyl, C₁-C₄-alkoxy, C₁-C₂-halogenoalkyl, C₁-C₂-halogenoalkoxy, cyano or nitro,
Y represents fluorine, chlorine, bromine, C₁-C₄-alkyl, C₁-C₄-alkoxy, C₁-C₂-halogenoalkyl, C₁-C₂-halogenoalkoxy, cyano or nitro,
Z represents fluorine, chlorine, bromine, C₁-C₄-alkyl, C₁-C₄-alkoxy, C₁-C₂-halogenoalkyl, C₁-C₂-halogenoalkoxy, hydroxy, cyano or nitro, or phenoxy or benzyloxy which are in each case optionally substituted by fluorine, chlorine, bromine, C₁-C₄-alkyl, C₁-C₄-alkoxy, C₁-C₂-halogenoalkyl, C₁-C₂-halogenoalkoxy, nitro or cyano, or
Y and Z together represent C₃-C₄-alkanediyl or C₃-C₄-alkenediyl which are optionally substituted by fluorine, chlorine, bromine, C₁-C₄-alkyl, C₁-C₄-alkoxy or C₁-C₂-halogenoalkyl and in which one or two members which are not directly adjacent can optionally be replaced, independently of one another, by oxygen, sulphur or nitrogen, X having one of the abovementioned meanings,
Het represents one of the groups 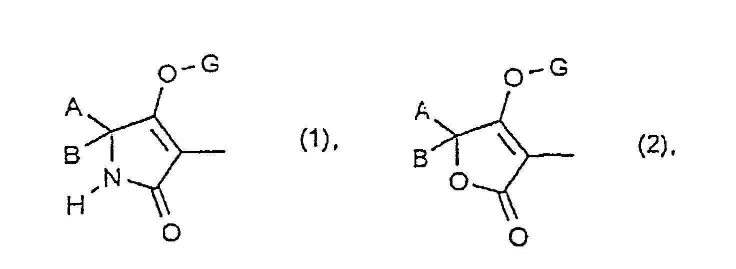 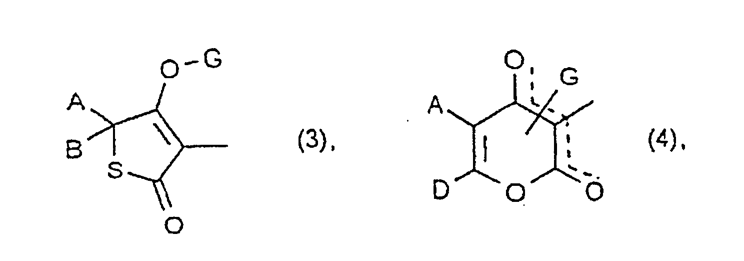 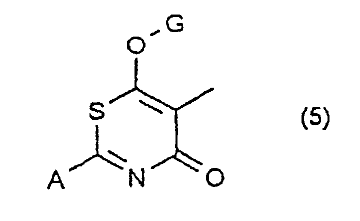
A represents hydrogen, or represents C₁-C₁₀-alkyl, C₂-C₆-alkenyl, C₁-C₈-alkoxy-C₁-C₆-alkyl, poly-C₁-C₆-alkoxy-C₁-C₆-alkyl or C₁-C₈-alkylthio-C₁-C₆-alkyl which are in each case optionally substituted by fluorine or chlorine, or represents C₃-C₇-cycloalkyl which is optionally substituted by fluorine, chlorine, C₁-C₄-alkyl or C₁-C₄-alkoxy and in which one or two methylene groups are optionally replaced by oxygen and/or sulphur, or represents phenyl, furanyl, pyridyl, imidazolyl, triazolyl, pyrazolyl, indolyl, thiazolyl, thienyl or phenyl-C₁-C₄-alkyl which are in each case optionally substituted by fluorine, chlorine, bromine, C₁-C₄-alkyl, C₁-C₄-halogenoalkyl, C₁-C₄-alkoxy, C₁-C₄-halogenoalkoxy, cyano or nitro,
B represents hydrogen, C₁-C₁₀-alkyl or C₁-C₆-alkoxy-C₁-C₄-alkyl or
A, B and the carbon atom to which they are bonded represent C₃-C₈-cycloalkyl or C₅-C₈-cycloalkenyl, wherein in each case one methylene group is optionally replaced by oxygen or sulphur and which are optionally substituted by C₁-C₆-alkyl, C₃-C₈-cycloalkyl, C₁-C₃-halogenoalkyl, C₁-C₆-alkoxy, C₁-C₆-alkylthio, fluorine, chlorine or phenyl, or
A, B and the carbon atom to which they are bonded represent C₅-C₆-cycloalkyl which is substituted by an alkylenediyl group which optionally contains one or two oxygen or sulphur atoms or by an alkylenedioxyl group or by an alkylenedithiol group, this substituent, together with the carbon atom to which it is bonded, forming a further five- to seven-membered ring, or
A, B and the carbon atom to which they are bonded represent C₃-C₆-cycloalkyl or C₅-C₆-cycloalkenyl in which two substituents, together with the carbon atom to which they are bonded, represent C₃-C₅-alkanediyl, C₃-C₅-alkenediyl or butadienediyl which are in each case optionally substituted by C₁-C₄-alkyl, C₁-C₄-alkoxy, fluorine, chlorine or bromine, wherein in each case one methylene group is optionally replaced by oxygen or sulphur,
D represents hydrogen, or represents C₁-C₁₀-alkyl, C₃-C₆-alkenyl, C₃-C₆-alkinyl, C₁-C₈-alkoxy-C₂-C₆-alkyl, poly-C₁-C₆-alkoxy-C₂-C₆-alkyl or C₁-C₈-alkylthio-C₂-C₆-alkyl which are in each case optionally substituted by fluorine or chlorine, or represents C₃-C₇-cycloalkyl which is optionally substituted by fluorine, chlorine, C₁-C₄-alkyl, C₁-C₄-alkoxy or C₁-C₂-halogenoalkyl and in which one or two methylene groups which are not directly adjacent are optionally replaced by oxygen and/or sulphur, or represents phenyl, furanyl, imidazolyl, pyridyl, thiazolyl, pyrazolyl, pyrimidyl, pyrrolyl, thienyl, triazolyl or phenyl-C₁-C₄-alkyl which are in each case optionally substituted by fluorine, chlorine, bromine, C₁-C₄-alkyl, C₁-C₄-halogenoalkyl, C₁-C₄-alkoxy, C₁-C₄-halogenoalkoxy, cyano or nitro, or
A and D together represent a C₃-C₅-alkanediyl or C₃-C₅-alkenediyl group wherein in each case one carbon atom is optionally replaced by oxygen or sulphur and which are optionally substituted by fluorine, chlorine or by C₁-C₆-alkyl, C₁-C₄-alkoxy, C₁-C₄-alkylthio, C₃-C₆-cycloalkyl, phenyl or benzyloxy which are in each case optionally substituted by fluorine or chlorine, or
which in each case optionally contain one of the following groups: 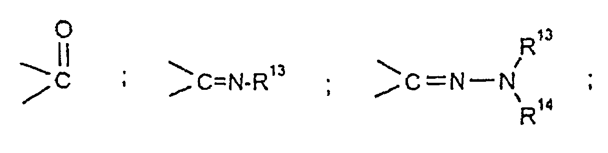 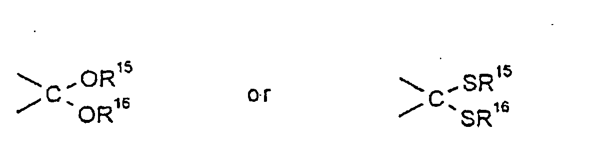
G represents hydrogen (a), or represents one of the groups 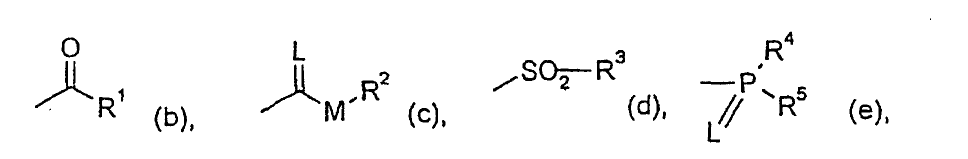
E (f) or 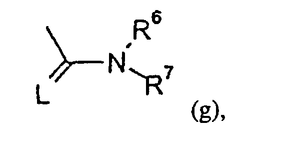 in which
E represents one metal ion equivalent or an ammonium ion,
L represents oxygen or sulphur and
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₁-C₆-alkyl which are in each case optionally substituted by fluorine or chlorine, or represents C₃-C₇-cycloalkyl which is optionally substituted by fluorine, chlorine, C₁-C₅-alkyl or C₁-C₅-alkoxy and in which one or two methylene groups are optionally replaced by oxygen and/or sulphur,
or represents phenyl which is optionally substituted by fluorine, chlorine, bromine, cyano, nitro, C₁-C₄-alkyl, C₁-C₄-alkoxy, C₁-C₃-halogenoalkyl, C₁-C₃-halogenoalkoxy, C₁-C₄-alkylthio or C₁-C₄-alkylsulphonyl,
or represents phenyl-C₁-C₄-alkyl which is optionally substituted by fluorine, chlorine, bromine, C₁-C₄-alkyl, C₁-C₄-alkoxy, C₁-C₃-halogenoalkyl or C₁-C₃-halogenoalkoxy,
or represents pyrazolyl, thiazolyl, pyridyl, pyrimidyl, furanyl or thienyl which are in each case optionally substituted by fluorine, chlorine, bromine or C₁-C₄-alkyl,
or represents phenoxy-C₁-C₅-alkyl which is optionally substituted by fluorine, chlorine, bromine or C₁-C₄-alkyl, or
represents pyridyloxy-C₁-C₅-alkyl, pyrimidyloxy-C₁-C₅-alkyl or thiazolyloxy-C₁-C₅-alkyl which are in each case optionally substituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl which are in each case optionally substituted by fluorine or chlorine,
or represents C₃-C₇-cycloalkyl which is optionally substituted by fluorine, chlorine, C₁-C₄-alkyl or C₁-C₄-alkoxy, or
represents phenyl or benzyl which are in each case optionally substituted by fluorine, chlorine, bromine, cyano, nitro, C₁-C₄-alkyl, C₁-C₃-alkoxy, C₁-C₃-halogenoalkyl or C₁-C₃-halogenoalkoxy,
R³ represents C₁-C₆-alkyl which is optionally substituted by fluorine or chlorine, or phenyl or benzyl which are in each case optionally substituted by fluorine, chlorine, bromine, C₁-C₄-alkyl, C₁-C₄-alkoxy, C₁-C₂-halogenoalkoxy, C₁-C₂-halogenoalkyl, cyano or nitro,
R⁴ and R⁵ independently of one another represent C₁-C₆-alkyl, C₁-C₆-alkoxy,
C₁-C₆-alkylamino, di-(C₁-C₆-alkyl)amino, C₁-C₆-alkylthio or C₃-C₄-alkenylthio which are in each case optionally substituted by fluorine or chlorine, or represent phenyl, phenoxy or phenylthio which are in each case optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent hydrogen, or represent C₁-C₆-alkyl, C₃-C₆-cycloalkyl, C₁-C₆-alkoxy, C₃-C₆-alkenyl or C₁-C₆-alkoxy-C₂-C₆-alkyl which are in each case optionally substituted by fluorine or chlorine, or represent phenyl or benzyl which are in each case optionally substituted by fluorine, chlorine, bromine, C₁-C₅-halogenoalkyl, C₁-C₅-alkyl or C₁-C₅-alkoxy, or together represent a C₃-C₆-alkylene radical which is optionally substituted by C₁-C₄-alkyl and in which one methylene group is optionally replaced by oxygen or sulphur,
R¹³ represents hydrogen, or C₁-C₆-alkyl or C₁-C₆-alkoxy which are in each case optionally substituted by fluorine or chlorine, or represents C₃-C₇-cycloalkyl which is optionally substituted by fluorine, C₁-C₂-alkyl or C₁-C₂-alkoxy and in which one methylene group is optionally replaced by oxygen or sulphur, or represents phenyl, phenyl-C₁-C₃-alkyl or phenyl-C₁-C₂-alkyloxy which are in each case optionally substituted by fluorine, chlorine, bromine, C₁-C₅-alkyl, C₁-C₅-alkoxy, C₁-C₂-halogenoalkyl, C₁-C₂-halogenoalkoxy, nitro or cyano,
R¹⁴ represents hydrogen or C₁-C₆-alkyl, or
R¹³ and R¹⁴ together represent C₄-C₆-alkanediyl,
R¹⁵ and R¹⁶ are identical or different and represent C₁-C₄-alkyl, or
R¹⁵ and R¹⁶ together represent a C₂-C₃-alkanediyl radical which is optionally substituted by C₁-C₄-alkyl or by phenyl which is optionally substituted by fluorine, chlorine, bromine, C₁-C₂-alkyl, C₁-C₂-halogenoalkyl, C₁-C₂-alkoxy, C₁-C₂-halogenoalkoxy, nitro or cyano.

3. Compounds of the formula (I) according to Claim 1, in which
X represents fluorine, chlorine, bromine, methyl, ethyl, propyl, butyl, iso-butyl, iso-propyl, tert-butyl, methoxy, ethoxy, propoxy, iso-propoxy, trifluoromethyl, trifluoromethoxy, difluoromethoxy, cyano or nitro,
Y represents fluorine, chlorine, bromine, methyl, ethyl, propyl, iso-propyl, butyl, iso-butyl, tert-butyl, methoxy, ethoxy, propoxy, iso-propoxy, trifluoromethyl, trifluoromethoxy, difluoromethoxy, cyano or nitro,
Z represents fluorine, chlorine, bromine, methyl, ethyl, propyl, butyl, iso-butyl, iso-propyl, tert-butyl, methoxy, ethoxy, propoxy, iso-propoxy, trifluoromethyl, trifluoromethoxy, difluoromethoxy, cyano or nitro, or
Y and Z together represent C₃-C₄-alkanediyl which is optionally substituted by fluorine, chlorine, methyl, ethyl, propyl, iso-propyl, methoxy, ethoxy, propoxy, iso-propoxy or trifluoromethyl and in which two members which are not directly adjacent are optionally replaced by oxygen, X having one of the abovementioned meanings,
Het represents one of the groups 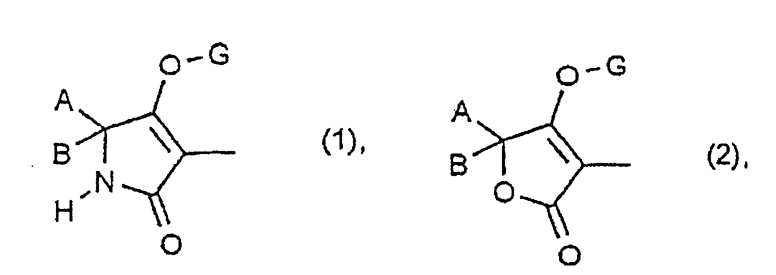 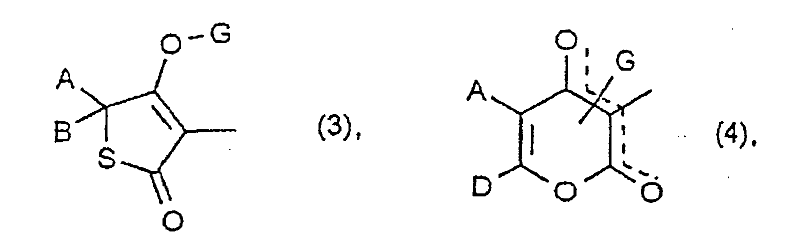 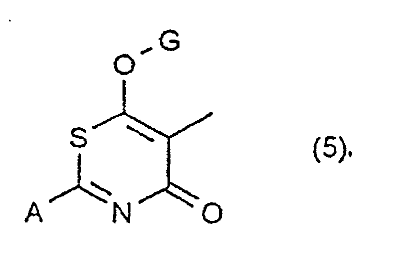
A represents hydrogen, or represents C₁-C₈-alkyl, C₂-C₄-alkenyl, C₁-C₆-alkoxy-C₁-C₄-alkyl, poly-C₁-C₄-alkoxy-C₁-C₄-alkyl or C₁-C₆-alkylthio-C₁-C₄-alkyl which are in each case optionally substituted by fluorine or chlorine, or represents C₃-C₆-cycloalkyl which is optionally substituted by fluorine, chlorine, methyl or methoxy and in which one or two methylene groups are optionally replaced by oxygen and/or sulphur, or represents phenyl, pyridyl or benzyl which are in each case optionally substituted by fluorine, chlorine, bromine, methyl, ethyl, n-propyl, iso-propyl, methoxy, ethoxy, trifluoromethyl, trifluoromethoxy, cyano or nitro,
B represents hydrogen, C₁-C₈-alkyl or C₁-C₄-alkoxy-C₁-C₂-alkyl or
A, B and the carbon atom to which they are bonded represent C₃-C₈-cycloalkyl or C₅-C₈-cycloalkenyl, wherein in each case one methylene group is optionally replaced by oxygen or sulphur and which are optionally substituted by methyl, ethyl, n-propyl, iso-propyl, butyl, iso-butyl, sec-butyl, tert-butyl, cyclohexyl, trifluoromethyl, methoxy, ethoxy, n-propoxy, iso-propoxy, butoxy, iso-butoxy, sec-butoxy, tert-butoxy, methylthio, ethylthio, fluorine, chlorine or phenyl, or
A, B and the carbon atom to which they are bonded represent C₅-C₆-cycloalkyl which is substituted by an alkylenediyl group which optionally contains an oxygen or sulphur atom or by an alkylenedioxyl group, this substituent forming a further five- or six-membered ring with the carbon atom to which it is bonded, or
A, B and the carbon atom to which they are bonded represent C₃-C₆-cycloalkyl or C₅-C₆-cycloalkenyl in which two substituents, together with the carbon atoms to which they are bonded, represent C₃-C₄-alkanediyl, C₃-C₄-alkenediyl or butadienediyl, wherein in each case one methylene group is optionally replaced by oxygen or sulphur,
D represents hydrogen, or represents C₁-C₈-alkyl, C₃-C₄-alkenyl, C₃-C₄-alkinyl, C₁-C₆-alkoxy-C₂-C₄-alkyl, poly-C₁-C₄-alkoxy-C₂-C₄-alkyl, C₁-C₄-alkylthio-C₂-C₄-alkyl or C₃-C₆-cycloalkyl which are in each case optionally substituted by fluorine or chlorine and in which one or two methylene groups which are not directly adjacent are optionally replaced by oxygen and/or sulphur, or represent phenyl, furanyl, pyridyl, thienyl or benzyl which are in each case optionally substituted by fluorine, chlorine, bromine, methyl, ethyl, n-propyl, iso-propyl, methoxy, ethoxy, trifluoromethyl, trifluoromethoxy, cyano or nitro,
or
A and D together represent a C₃-C₅-alkanediyl or C₃-C₅-alkenediyl group wherein in each case one methylene group is optionally replaced by oxygen or sulphur and which are optionally substituted by fluorine, chlorine or by C₁-C₆-alkyl, C₁-C₄-alkoxy, C₁-C₄-alkylthio, C₃-C₆-cycloalkyl, phenyl or benzyloxy which are in each case optionally substituted by fluorine or chlorine,
G represents hydrogen (a), or represents one of the groups 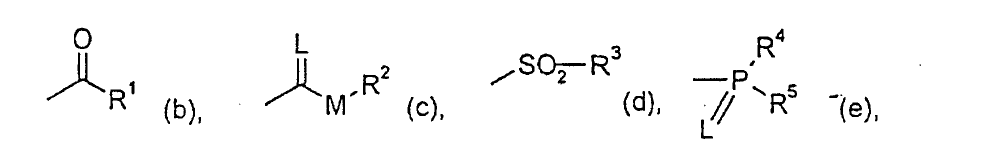
E(f) or 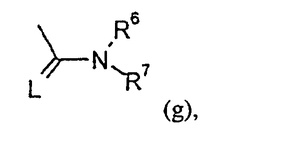 in which
E represents one metal ion equivalent or an ammonium ion,
L represents oxygen or sulphur and
M represents oxygen or sulphur,
R¹ represents C₁-C₁₄-alkyl, C₂-C₁₄-alkenyl, C₁-C₄-alkoxy-C₁-C₆-alkyl, C₁-C₄-alky]thio-C₁-C₆-a]kyl, poly-C₁-C₄-alkoxy-C₁-C₄-alkyl which are in each case optionally substituted by fluorine or chlorine, or represents C₃-C₆-cycloalkyl which is optionally substituted by fluorine, chlorine, methyl, ethyl, n-propyl, i-propyl, n-butyl, i-butyl, tert-butyl, methoxy, ethoxy, n-propoxy or iso-propoxy and in which one or two methylene groups are optionally replaced by oxygen and/or sulphur,
or represents phenyl which is optionally substituted by fluorine, chlorine, bromine, cyano, nitro, methyl, ethyl, n-propyl, i-propyl, methoxy, ethoxy, trifluoromethyl, trifluoromethoxy, methylthio, ethylthio, methylsulphonyl or ethylsulphonyl,
or represents benzyl which is optionally substituted by fluorine, chlorine, bromine, methyl, ethyl, n-propyl, i-propyl, methoxy, ethoxy, trifluoromethyl or trifluoromethoxy,
or represents furanyl, thienyl or pyridyl which are in each case optionally substituted by fluorine, chlorine, bromine, methyl or ethyl,
or represents phenoxy-C₁-C₄-alkyl which is optionally substituted by fluorine, chlorine, methyl or ethyl, or
represents pyridyloxy-C₁-C₄-alkyl, pyrimidyloxy-C₁-C₄-alkyl or thiazolyloxy-C₁-C₄-alkyl which are in each case optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl which are in each case optionally substituted by fluorine or chlorine,
or represents C₃-C₆-cycloalkyl which is optionally substituted by fluorine, chlorine, methyl, ethyl, n-propyl, iso-propyl or methoxy,
or represents phenyl or benzyl which are in each case optionally substituted by fluorine, chlorine, cyano, nitro, methyl, ethyl, n-propyl, i-propyl, methoxy, ethoxy, trifluoromethyl or trifluoromethoxy,
R³ represents methyl, ethyl, propyl, iso-propyl, butyl or tert-butyl which are optionally substituted by fluorine or chlorine, or phenyl or benzyl which are in each case optionally substituted by fluorine, chlorine, bromine, methyl, ethyl, iso-propyl, tert-butyl, methoxy, ethoxy, iso-propoxy, trifluoromethyl, trifluoromethoxy, cyano or nitro,
R⁴ and R⁵ independently of one another represent C₁-C₄-alkyl, C₁-C₄-alkoxy, C₁-C₄-alkylamino, di-(C₁-C₄-alkyl)amino or C₁-C₄-alkylthio which are in each case optionally substituted by fluorine or chlorine, or represent phenyl, phenoxy or phenylthio which are in each case optionally substituted by fluorine, chlorine, bromine, nitro, cyano, methyl, methoxy, trifluoromethyl or trifluoromethoxy,
R⁶ and R⁷ independently of one another represent hydrogen, or represent C₁-C₄-alkyl, C₃-C₆-cycloalkyl, C₁-C₄-alkoxy, C₃-C₄-alkenyl or C₁-C₄-alkoxy-C₂-C₄-alkyl which are in each case optionally substituted by fluorine or chlorine, or represent phenyl or benzyl which are in each case optionally substituted by fluorine, chlorine, bromine, methyl, methoxy or trifluoromethyl, or together represent a C₅-C₆-alkylene radical which is optionally substituted by methyl or ethyl and in which one methylene group is optionally replaced by oxygen or sulphur.

4. Process for the preparation of compounds of the formula (I) according to Claim 1, **characterized in that** to obtain
(A) compounds of the formula (I-1-a) 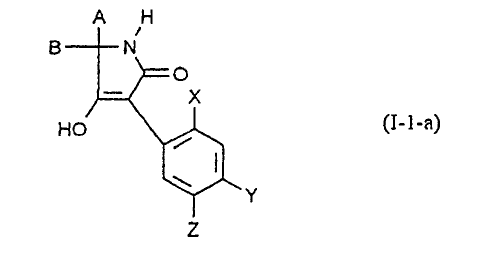 in which
A, B, X, Y and Z have the meanings given in Claim 1,
compounds of the formula (II) 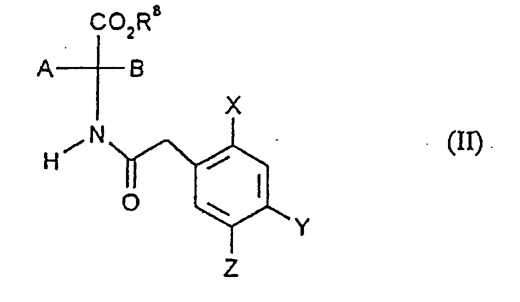 in which
A, B, X, Y and Z have the meanings given in Claim 1
and
R⁸ represents alkyl,
are subjected to intramolecular condensation in the presence of a diluent and in the presence of a base,
(B) compounds of the formula (I-2-a) 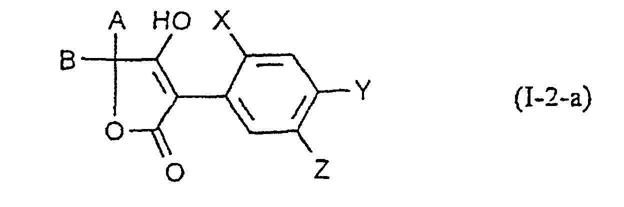 in which
A, B, X, Y and Z have the abovementioned meanings,
compounds of the formula (III) 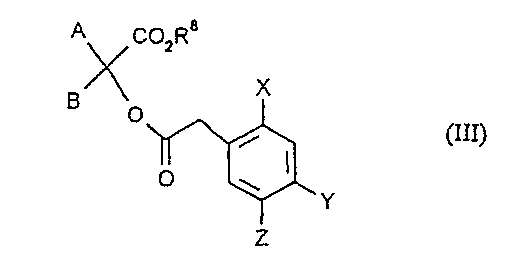 in which
A, B, X, Y, Z and R⁸ have the abovementioned meanings,
are subjected to intramolecular condensation in the presence of a diluent and in the presence of a base,
(C) compounds of the formula (I-3-a) 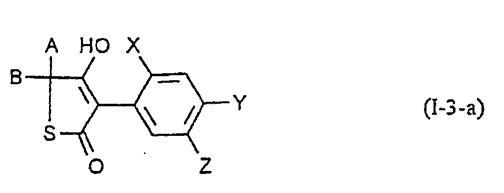 in which
A, B, X, Y and Z have the abovementioned meanings,
compounds of the formula (IV) 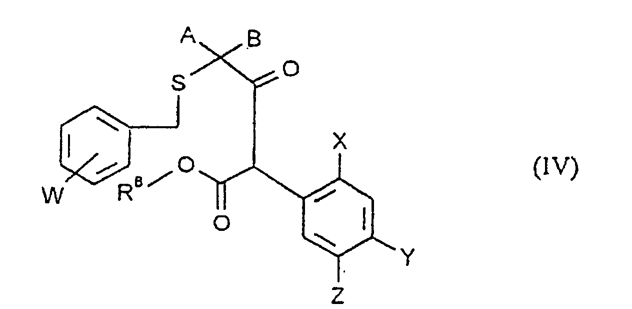 in which
A, B, X, Y, Z and R⁸ have the abovementioned meanings and
W x represents hydrogen, halogen, alkyl or alkoxy,
are subjected to intramolecular cyclization, if appropriate in the presence of a diluent and in the presence of an acid,
(D) compounds of the formula (I-4-a) 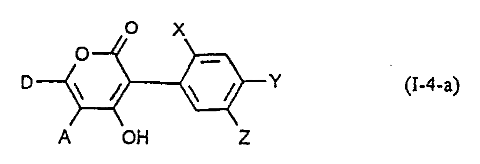 in which
A, D, X, Y and Z have the meanings given in Claim 1,
compounds of the formula (V) 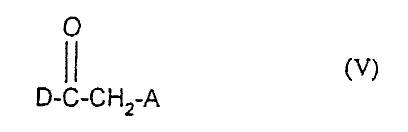 in which
A and D have the abovementioned meanings,
or silylenol ethers thereof, of the formula (Va) 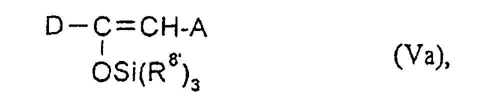 in which
A and D have the abovementioned meaning and
R^{8'} represents alkyl,
are reacted with compounds of the formula (VI) 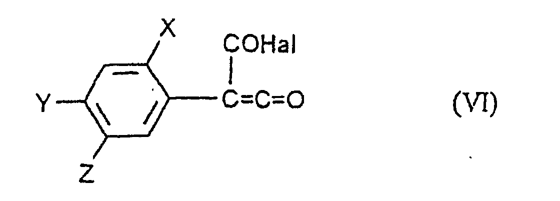 in which
X, Y and Z have the abovementioned meanings and
Hal represents halogen,
if appropriate in the presence of a diluent and if appropriate in the presence of an acid acceptor,
(E) compounds of the formula (I-5-a) 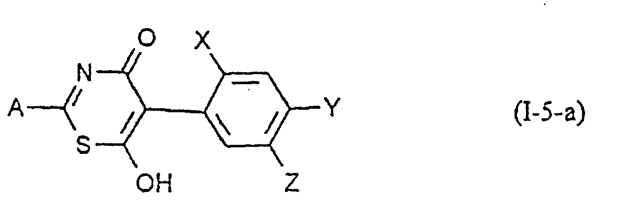 in which
A, X, Y and Z have the abovementioned meanings,
compounds of the formula (VII) 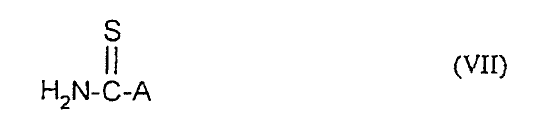 in which
A has the abovementioned meaning,
are reacted with compounds of the formula (VI) 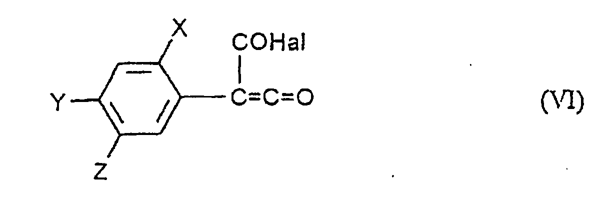 in which
Hal, X, Y and Z have the abovementioned meanings,
if appropriate in the presence of a diluent and if appropriate in the presence of an acid acceptor, and, if appropriate, the compounds thus obtained, of the formulae (I-1-b) to (I-5-b) shown above, are then
(F)α) reacted with acid halides of the formula (VIII) 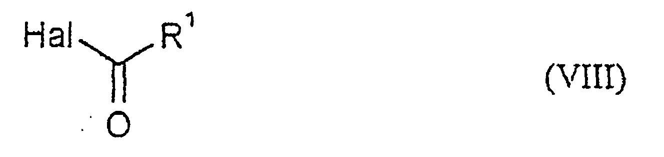 in which
R¹ has the meaning given in Claim 1 and
Hal represents halogen,
or
β) with carboxylic acid anhydrides of the formula (IX)
R¹-CO-O-CO-R¹ (IX)
in which
R¹ has the abovementioned meaning,
if appropriate in the presence of a diluent and if appropriate in the presence of an acid-binding agent,
or
(G) reacted with chloroformic acid esters or chloroformic acid thioesters of the formula (X)
R²-M-CO-Cl (X)
in which
R² and M have the meanings given in Claim 1,
if appropriate in the presence of a diluent and if appropriate in the presence of an acid-binding agent,
or
(H)α) reacted with chloromonothioformic acid esters or chlorodithioformic acid esters of the formula (XI) 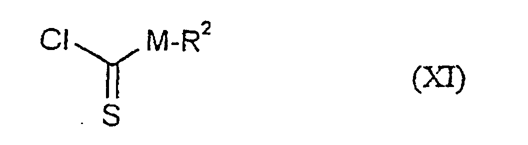 in which
M and R² have the abovementioned meanings,
if appropriate in the presence of a diluent and if appropriate in the presence of an acid-binding agent,
or
β) reacted with carbon disulphide and then with compounds of the formula (XII)
R²-Hal (XII)
in which
R² has the abovementioned meaning and
Hal represents chlorine, bromine or iodine,
if appropriate in the presence of a diluent and if appropriate in the presence of a base,
or
(I) reacted with sulphonic acid chlorides of the formula (XIII)
R³-SO₂-Cl (XIII)
in which
R³ has the meaning given in Claim 1,
if appropriate in the presence of a diluent and if appropriate in the presence of an acid-binding agent,
or
(J) reacted with phosphorus compounds of the formula (XIV) 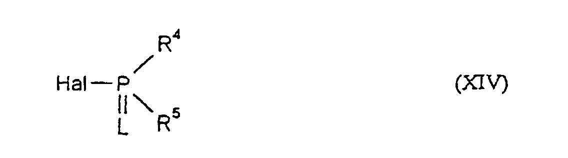 in which
L, R⁴ and R⁵ have the abovementioned meanings and
Hal represents halogen,
if appropriate in the presence of a diluent and if appropriate in the presence of an acid-binding agent,
or
(K) reacted with metal compounds or amines of the formulae (XV) or (XVI)
Me(OR¹⁰)ₜ (XV) 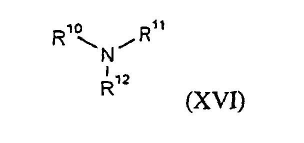
in which
Me represents a mono- or divalent metal,
t represents the number 1 or 2 and
R¹⁰, R¹¹ and R¹² independently of one another represent hydrogen or alkyl,
if appropriate in the presence of a diluent,
or
(L) α) reacted with isocyanates or isothiocyanates of the formula (XVII)
R⁶-N=C=L (XVII)
in which
R⁶ and L have the meanings given in Claim 1,
if appropriate in the presence of a diluent and if appropriate in the presence of a catalyst, or
β) reacted with carbamic acid chlorides or thiocarbamic acid chlorides of the formula (XVIII) 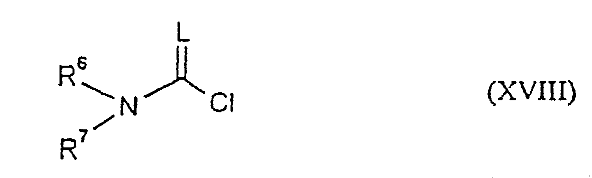 in which
L, R⁶ and R⁷ have the abovementioned meanings,
if appropriate in the presence of a diluent and if appropriate in the presence of an acid-binding agent.

5. Compounds of the formula (II) 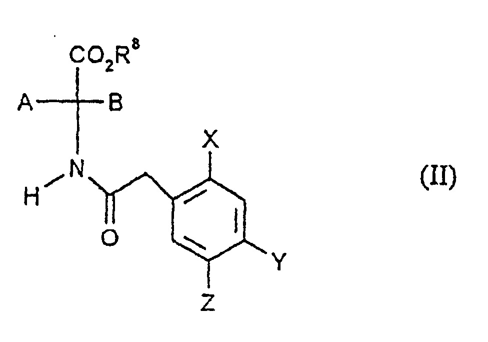 in which
A, B, X, Y and Z have the meanings given in Claim 1 and
R⁸ represents alkyl.

6. Compounds of the formula (III) 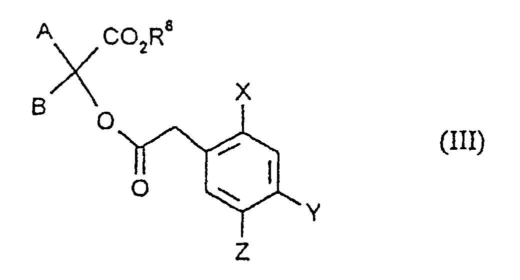 in which
A, B, X, Y and Z have the meanings given in Claim 1 and
R⁸ represents alkyl.

7. Compounds of the formula (XXI) 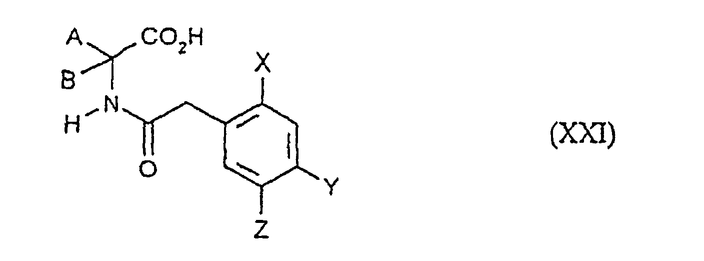 in which
A, B, X, Y and Z have the meanings given in Claim 1.

8. Compounds of the formula (XXIX) 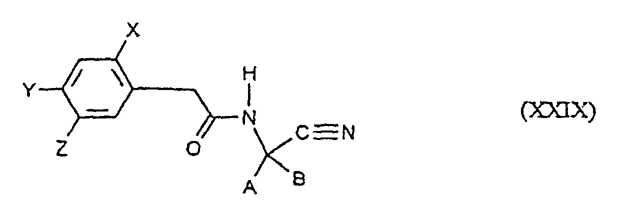 in which
A, B, X, Y and Z have the meanings given in Claim 1.

9. Compounds of the formula (IV) 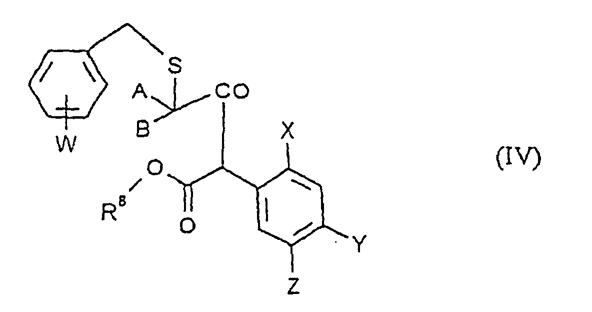 in which
A, B, X, Y and Z have the meanings given in Claim 1,
R⁸ represents alkyl and
W represents hydrogen, halogen, alkyl or alkoxy.

10. Compounds of the formula (VI) 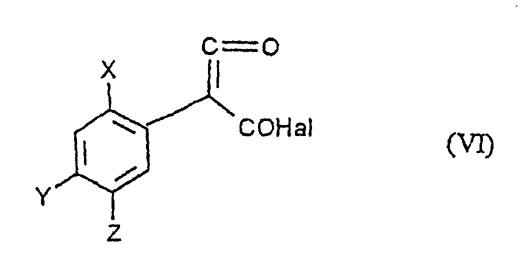 in which
X, Y and Z have the meanings given in Claim 1 and
Hal represents chlorine or bromine.

11. Compounds of the formula (XXXIII) 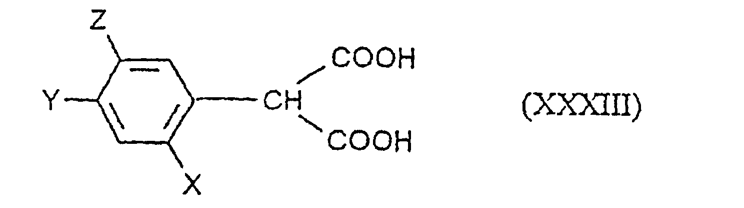 in which
X, Y and Z have the meanings given in Claim 1.

12. Compounds of the formula (XXXIV) 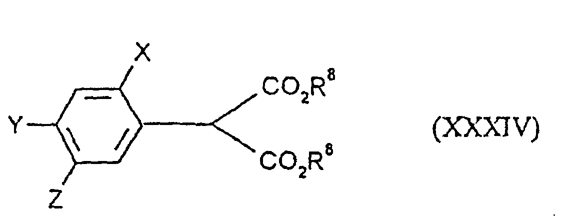 in which
X, Y and Z have the meanings given in Claim 1 and
R⁸ represents alkyl.

13. Pest control compositions and herbicides, **characterized by** a content of at least one compound of the formula (I) according to Claim 1.

14. Use of compounds of the formula (I) according to Claim 1 for controlling pests and undesirable plant growth.

15. Method of controlling pests, **characterized in that** compounds of the formula (I) according to Claim 1 are allowed to act on pests, undesirable plant growth and/or their environment.

16. Process for the preparation of pest control compositions and herbicides, **characterized in that** compounds of the formula (I) according to Claim 1 are mixed with extenders and/or surface-active agents.

17. Use of compounds of the formula (I) according to Claim 1 for the preparation of pest control compositions and herbicides.

## Revendications

1. Composés de formule (I) 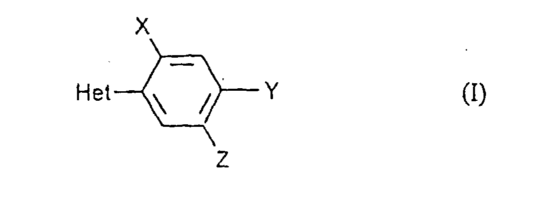 dans laquelle
X représente un halogène, un groupe alkyle en C₁ à C₆, alcényle en C₂ à C₆, alcynyle en C₂ à C₆, alkoxy en C₁ à C₆, halogénalkyle en C₁ à C₄, halogénalkoxy en C₁ à C₄, cyano ou nitro,
Y représente un halogène, un groupe alkyle en C₁ à C₆, alkoxy en C₁ à C₆, halogénalkyle en C₁ à C₄, halogénalkoxy en C₁ à C₄, cyano ou nitro,
Z représente un halogène, un groupe alkyle en C₁ à C₆, alkoxy en C₁ à C₆, halogénalkyle en C₁ à C₄, halogénalkoxy en C₁ à C₄, hydroxy, cyano, nitro ou un groupe phénoxy, phénylthio, thiazolyloxy, pyridinyloxy, pyrimidyloxy, pyrazolyloxy, phényl-(alkyloxy en C₁ à C₄) ou phényl-(alkylthio en C₁ à C₄), chacun étant éventuellement substitué par un halogène, un radical alkyle en C₁ à C₄, alkoxy en C₁ à C₄, halogénalkyle en C₁ à C₄, halogénalkoxy en C₁ à C₄, nitro ou cyano, ou bien
Y et Z forment ensemble un reste alcanediyle en C₃ ou C₄ ou un reste alcènediyle en C₃ ou C₄ portant éventuellement un substituant halogéno, alkyle en C₁ à C₆, alkoxy en C₁ à C₆ ou halogénalkyle en C₁ à C₄, dont, le cas échéant, un à trois chaînons peuvent être remplacés indépendamment les uns des autres par de l'oxygène, du soufre, de l'azote ou un groupe carbonyle, X ayant l'une des définitions indiquées ci-dessus,
Het représente l'un des groupes 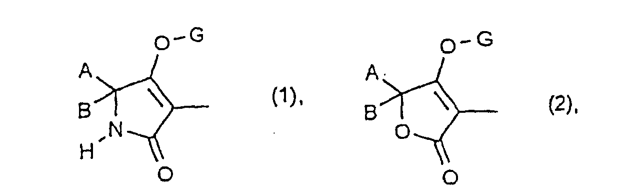 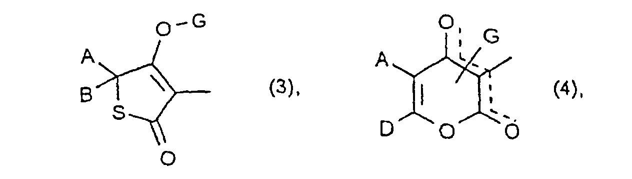 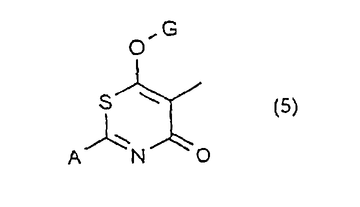
A représente l'hydrogène, un reste alkyle en C₁ à C₁₂, alcényle en C₂ à C₈, (alkoxy en C₁ à C₁₀)-(alkyle en C₁ à C₈), poly-(alkoxy en C₁ à C₈)-(alkyle en C₁ à C₈) ou (alkylthio en C₁ à C₁₀)-(alkyle en C₁ à C₆) dont chacun est éventuellement substitué par un halogène, un reste cycloalkyle en C₃ à C₈ éventuellement substitué par un halogène, un radical alkyle en C₁ à C₆ ou alkoxy en C₁ à C₆, et dont, le cas échéant, un ou deux groupes méthylène sont remplacés par de l'oxygène et/ou du soufre, ou un groupe phényle, naphtyle, phényl-(alkyle en C₁ à C₆), naphtyl-(alkyle en C₁ à C₆) ou hétaryle à noyau de 5 ou 6 atomes avec un à trois hétéroatomes de la série oxygène, soufre et azote, portant éventuellement un substituant halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un groupe alkyle en C₁ à C₁₂ ou (alkoxy en C₁ à C₈)-(alkyle en C₁ à C₆), ou bien
A, B et l'atome de carbone auquel il sont liés représentent un reste cycloalkyle en C₃ à C₁₀ ou cycloalcényle en C₅ à C₁₀, où, le cas échéant, un groupe méthylène est remplacé par de l'oxygène ou du soufre et qui sont éventuellement substitués par un radical alkyle en C₁ à C₈, cycloalkyle en C₃ à C₁₀, halogénalkyle en C₁ à C₈, alkoxy en C₁ à C₈, alkylthio en C₁ à C₈, halogéno ou phényle, ou bien
A, B et l'atome de carbone auquel ils sont liés représentent un reste cycloalkyle en C₅ ou C₆ qui est substitué par un groupe alcènediyle contenant, le cas échéant, un ou deux atomes d'oxygène et/ou de soufre ou par un groupe alkylènedioxyle ou par un groupe alkylènedithioyle qui forme un autre noyau pentagonal à octogonal avec l'atome de carbone auquel il est lié, ou bien
A, B et l'atome de carbone auquel ils sont liés représentent un reste cycloalkyle en C₃ à C₈ ou cycloalcényle en C₅ à C₈ dans lequel deux substituants forment conjointement avec les atomes de carbone auxquels ils sont liés un groupe alcanediyle en C₃ à C₆, alcènediyle en C₃ à C₆ ou alcanediènediyle en C₄ à C₆ dont chacun est éventuellement substitué par un radical alkyle en C₁ à C₆, alkoxy en C₁ à C₆ ou halogéno, et où, dans chaque cas, un groupe méthylène peut éventuellement être remplacé par de l'oxygène ou du soufre,
D représente l'hydrogène, un reste alkyle en C₁ à C₁₂, alcényle en C₃ à C₈, alcynyle en C₃ à C₈, (alkoxy en C₁ à C₁₀)-(alkyle en C₂ à C₈), poly-(alkoxy en C₁ à C₈)-(alkyle en C₂ à C₈) ou (alkylthio en C₁ à C₁₀)-(alkyle en C₂ à C₈) dont chacun est éventuellement substitué par un halogène, un reste cycloalkyle en C₃ à C₈ éventuellement substitué par un halogène, un radical alkyle en C₁ à C₄, alkoxy en C₁ à C₄ ou halogénalkyle en C₁ à C₄ et dans lequel, le cas échéant, un ou deux groupes méthylène non adjacents peuvent être remplacés par de l'oxygène et/ou du soufre, ou bien un groupe phényle, hétaryle à noyau de 5 ou 6 atomes avec un ou deux hétéroatomes de la série oxygène, soufre et azote, phényl-(alkyle en C₁ à C₆) ou hétaryl-(alkyle en C₁ à C₆) à noyau de 5 ou 6 atomes avec un ou deux hétéroatomes de la série oxygène, soufre et azote, chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou bien
A et D forment conjointement un groupe alcanediyle en C₃ à C₆, alcènediyle en C₃ à C₆ ou alcadiènediyle en C₄ à C₆, où, dans chaque cas, un groupe méthylène peut éventuellement être remplacé par de l'oxygène ou du soufre, et dont chacun est éventuellement substitué par un halogène ou par un radical alkyle en C₁ à C₁₀, alkoxy en C₁ à C₆, alkylthio en C₁ à C₆, cycloalkyle en C₃ à C₇, phényle ou benzyloxy dont chacun est éventuellement substitué par un halogène, ou par un autre groupe alcanediyle en C₃ à C₆, alcènediyle en C₃ à C₆ ou alcadiènediyle en C₄ à C₆ formant éventuellement un noyau condensé, dans chacun desquels, le cas échéant, un groupe méthylène est remplacé par de l'oxygène ou du soufre et qui sont éventuellement substitués par un radical alkyle en C₁ à C₆, ou bien
A et D forment ensemble un groupe alcanediyle en C₃ à C₆ ou alcènediyle en C₃ à C₆, contenant chacun, le cas échant, l'un des groupes suivants 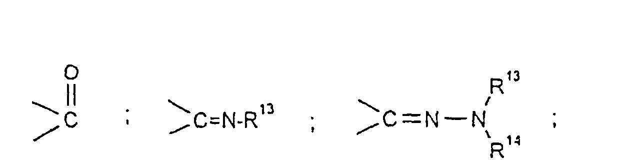 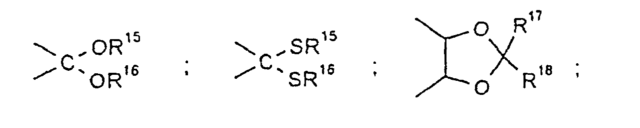 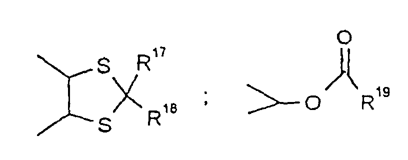 ou 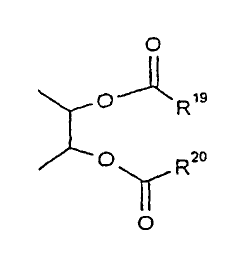
G représente l'hydrogène (a) ou l'un des groupes 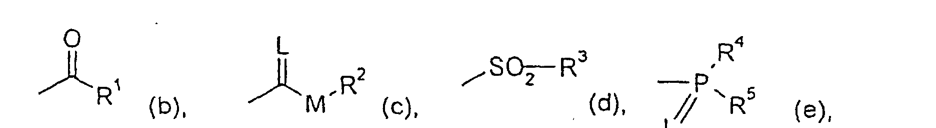
E (f) ou 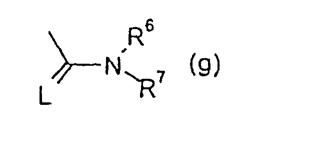 dans lesquels
E représente un équivalent d'ion métallique ou un ion ammonium,
L représente l'oxygène ou le soufre et
M représente l'oxygène ou le soufre,
R¹ représente un reste alkyle en C₁ à C₂₀, alcényle en C₂ à C₂₀, (alkoxy en C₁ à C₈)-(alkyle en C₁ à C₈), (alkylthio en C₁ à C₈)-(alkyle en C₁ à C₈) ou poly-(alkoxy en C₁ à C₈)-(alkyle en C₁ à C₈) dont chacun est éventuellement substitué par un halogène, ou bien un reste cycloalkyle en C₃ à C₈ éventuellement substitué par un radical halogéno, alkyle en C₁ à C₆ ou alkoxy en C₁ à C₆, et dans lequel, le cas échéant, un ou deux groupes méthylène sont remplacés par de l'oxygène et/ou du soufre,
un reste phényle éventuellement substitué par un halogène, un radical nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆,
un reste phényl-(alkyle en C₁ à C₆) éventuellement substitué par un halogène, un radical nitro, cyano, alkyle en C₁ à C₆, alkoxy en C₁ à C₆, halogénalkyle en C₁ à C₆ ou halogénalkoxy en C₁ à C₆,
un reste hétaryle pentagonal ou hexagonal ayant un ou deux hétéroatomes de la série oxygène, soufre et azote, éventuellement substitué par un halogène ou un radical alkyle en C₁ à C₆,
un reste phénoxy-(alkyle en C₁ à C₆) éventuellement substitué par un halogène ou un radical alkyle en C₁ à C₆, ou bien
un reste hétaryloxy-(alkyle en C₁ à C₆) pentagonal ou hexagonal ayant un ou deux hétéroatomes de la série oxygène, soufre et azote et éventuellement substitué par un radical halogéno, amino ou alkyle en C₁ à C₆,
R² représente un reste alkyle en C₁ à C₂₀, alcényle en C₂ à C₂₀, (alkoxy en C₁ à C₈)-(alkyle en C₂ à C₈) ou poly-(alkoxy en C₁ à C₈)-(alkyle en C₂ à C₈) dont chacun est éventuellement substitué par un halogène,
un groupe cycloalkyle en C₃ à C₈ éventuellement substitué par un halogène, un radical alkyle en C₁ à C₆ ou alkoxy en C₁ à C₆, ou
un reste phényle ou benzyle dont chacun est éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène, ou un reste phényle ou benzyle dont chacun est éventuellement sustitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ forment, indépendamment l'un de l'autre, un reste alkyle en C₁ à C₈, alkoxy en C₁ à C₈, alkylamino en C₁ à C₈, di(alkyle en C₁ à C₈)amino, alkylthio en C₁ à C₈ ou alcénylthio en C₃ à C₈ dont chacun est éventuellement substitué par un halogène, ou bien un groupe phényle, phénoxy ou phénylthio dont chacun est éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ et R⁷ représentent, indépendamment l'un de l'autre, l'hydrogène, un reste alkyle en C₁ à C₈, cycloalkyle en C₃ à C₈, alkoxy en C₁ à C₈, alcényle en C₃ à C₈ ou (alkoxy en C₁ à C₈)-(alkyle en C₂ à C₈) dont chacun est éventuellement substitué par un halogène, un reste phényle ou benzyle dont chacun est éventuellement substitué par un radical halogéno, alkyle en C₁ à C₈, halogénalkyle en C₁ à C₈ ou alkoxy en C₁ à C₈, ou forment ensemble un reste alkylène en C₃ à C₆ éventuellement substitué par un radical alkyle en C₁ à C₆ et dans lequel un groupe méthylène est éventuellement remplacé par de l'oxygène ou du soufre,
R¹³ représente l'hydrogène ou un reste alkyle en C₁ à C₈ ou alkoxy en C₁ à C₈ dont chacun est éventuellement substitué par un halogène, un reste cycloalkyle en C₃ à C₈ éventuellement substitué par un radical halogéno, alkyle en C₁ à C₄ ou alkoxy en C₁ à C₄ et dans lequel un groupe méthylène est éventuellement remplacé par de l'oxygène ou du soufre, ou bien un reste phényle, phényl-(alkyle en C₁ à C₄) ou phényl-(alkoxy en C₁ à C₄) dont chacun est éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
R¹⁴ représente l'hydrogène ou un reste alkyle en C₁ à C₈, ou bien
R¹³ et R¹⁴ forment ensemble un reste alcanediyle en C₄ à C₆,
R¹⁵ et R¹⁶ sont identiques ou différents et représentent un reste alkyle en C₁ à C₆, ou bien
R¹⁵ et R¹⁶ forment ensemble un reste alcanediyle en C₂ à C₄ qui est éventuellement substitué par un groupe alkyle en C₁ à C₆ ou par un groupe phényle portant éventuellement un substituant halogéno, alkyle en C₁ à C₄, halogénalkyle en C₁ à C₄, alkoxy en C₁ à C₄, halogénalkoxy en C₁ à C₄, nitro ou cyano,
R¹⁷ et R¹⁸ représentent, indépendamment l'un de l'autre, l'hydrogène, un reste alkyle en C₁ à C₈ éventuellement substitué par un halogène ou un reste phényle éventuellement substitué par un radical halogéno, alkyle en C₁ à C₆, alkoxy en C₁ à C₆, halogénalkyle en C₁ à C₄, halogénalkoxy en C₁ à C₄, nitro ou cyano, ou bien
R¹⁷ et R¹⁸ forment conjointement avec l'atome de carbone auquel ils sont liés un reste cycloalkyle en C₅ à C₇ éventuellement substitué par un radical alkyle en C₁ à C₄ et dans lequel, le cas échéant, un groupe méthylène est remplacé par de l'oxygène ou du soufre,
R¹⁹ et R²⁰ représentent, indépendamment l'un de l'autre, un reste alkyle en C₁ à C₁₀, alcényle en C₂ à C₁₀, alkoxy en C₁ à C₁₀, alkylamino en C₁ à C₁₀, alcénylamino en C₃ à C₁₀, di(alkyle en C₁ à C₁₀)amino ou di(alcényle en C₃ à C₁₀)amino.

2. Composés de formule (I) suivant la revendication 1, dans laquelle
X représente le fluor, le chlore, le brome, un groupe alkyle en C₁ à C₄, alkoxy en C₁ à C₄, halogénalkyle en C₁ ou C₂, halogénalkoxy en C₁ ou C₂, cyano ou nitro,
Y représente le fluor, le chlore, le brome, un groupe alkyle en C₁ à C₄, alkoxy en C₁ à C₄, halogénalkyle en C₁ ou C₂, halogénalkoxy en C₁ ou C₂, cyano ou nitro,
Z représente le fluor, le chlore, le brome, un groupe alkyle en C₁ à C₄, alkoxy en C₁ à C₄, halogénalkyle en C₁ ou C₂, halogénalkoxy en C₁ ou C₂, hydroxy, cyano, nitro ou un reste phénoxy ou benzyloxy dont chacun est éventuellement substitué par du fluor, du chlore, du brome, un radical alkyle en C₁ à C₄, alkoxy en C₁ à C₄, halogénalkyle en C₁ ou C₂, halogénalkoxy en C₁ ou C₂, nitro ou cyano, ou bien
Y et Z forment ensemble un reste alcanediyle en C₃ ou C₄ ou alcènediyle en C₃ ou C₄ éventuellement substitué par du fluor, du chlore, du brome, un radical alkyle en C₁ à C₄, alkoxy en C₁ à C₄ ou halogénalkyle en C₁ ou C₂ et dont, le cas échéant, un ou deux chaînons non contigus peuvent être remplacés, indépendamment l'un de l'autre, par de l'oxygène, du soufre ou de l'azote, X ayant l'une des définitions indiquées ci-dessus,
Het représente l'un des groupes 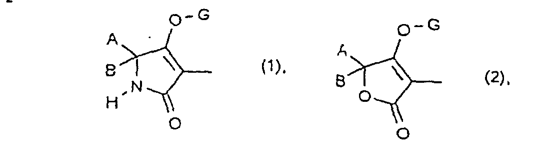 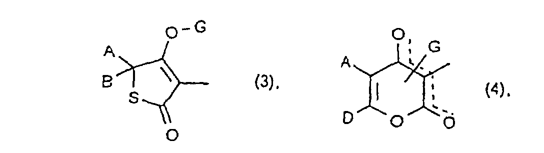 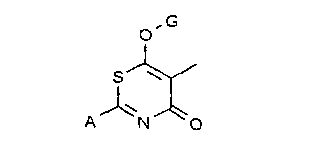
A est l'hydrogène, un reste alkyle en C₁ à C₁₀, alcényle en C₂ à C₆, (alkoxy en C₁ à C₈)-(alkyle en C₁ à C₆), poly-(alkoxy en C₁ à C₆)-(alkyle en C₁ à C₆) ou (alkylthio en C₁ à C₈)-(alkyle en C₁ à C₆) dont chacun est éventuellement substitué par du fluor ou du chlore, ou un reste cycloalkyle en C₃ à C₇ éventuellement substitué par du fluor, du chlore, un radical alkyle en C₁ à C₄ ou alkoxy en C₁ à C₄, et dans lequel éventuellement un ou deux groupes méthylène sont remplacés par de l'oxygène et/ou du soufre, ou un reste phényle, furannyle, pyridyle, imidazolyle, triazolyle, pyrazolyle, indolyle, thiazolyle, thiényle ou phényl-(alkyle en C₁ à C₄) dont chacun est éventuellement substitué par du fluor, du chlore, du brome, un radical alkyle en C₁ à C₄, halogénalkyle en C₁ à C₄, alkoxy en C₁ à C₄, halogénalkoxy en C₁ à C₄, cyano ou nitro,
B représente l'hydrogène, un groupe alkyle en C₁ à C₁₀ ou (alkoxy en C₁ à C₆)-(alkyle en C₁ à C₄), ou bien
A, B et l'atome de carbone auquel il sont liés représentent un reste cycloalkyle en C₃ à C₈ ou cycloalcényle en C₅ à C₈, dans chacun desquels, le cas échéant, un groupe méthylène est remplacé par de l'oxygène ou du soufre et qui sont éventuellement substitués par un radical alkyle en C₁ à C₆, cycloalkyle en C₃ à C₈, halogénalkyle en C₁ à C₃, alkoxy en C₁ à C₆, alkylthio en C₁ à C₆, du fluor, du chlore ou un radical phényle, ou bien
A, B et l'atome de carbone auquel ils sont liés représentent un reste cycloalkyle en C₅ ou C₆ qui est substitué par un groupe alkylènediyle contenant, le cas échéant, un ou deux atomes d'oxygène ou de soufre ou par un groupe alkylènedioxyle ou par un groupe alkylènedithiol qui forme avec l'atome de carbone auquel il est lié un autre noyau pentagonal à heptagonal, ou bien
A, B et l'atome de carbone auquel ils sont liés représentent un reste cycloalkyle en C₃ à C₆ ou cycloalcényle en C₅ ou C₆ dans lequel deux substituants forment conjointement avec l'atome de carbone auquel ils sont liés un groupe alcanediyle en C₃ à C₅, alcènediyle en C₃ à C₅ ou butadiènediyle dans chacun desquels, le cas échéant, un groupe méthylène est remplacé par de l'oxygène ou du soufre,
D représente l'hydrogène, un reste alkyle en C₁ à C₁₀, alcényle en C₃ à C₆, alcynyle en C₃ à C₆, (alkoxy en C₁ à C₈)-(alkyle en C₂ à C₆), poly-(alkoxy en C₁ à C₆)-(alkyle en C₂ à C₆) ou (alkylthio en C₁ à C₈)-(alkyle en C₂ à C₆) dont chacun est éventuellement substitué par du fluor ou du chlore, un groupe cycloalkyle en C₃ à C₇ éventuellement substitué par du fluor, du chlore, un radical alkyle en C₁ à C₄, alkoxy en C₁ à C₄ ou halogénalkyle en C₁ ou C₂, dans lequel, le cas échéant, un ou deux groupes méthylène non contigus sont remplacés par de l'oxygène et/ou du soufre, ou un groupe phényle, furannyle, imidazolyle, pyridyle, thiazolyle, pyrazolyle, pyrimidyle, pyrrolyle, thiényle, triazolyle ou phényl-(alkyle en C₁ à C₄) dont chacun est éventuellement substitué par du fluor, du chlore, du brome, un radical alkyle en C₁ à C₄, halogénalkyle en C₁ à C₄, alkoxy en C₁ à C₄, halogénalkoxy en C₁ à C₄, cyano ou nitro, ou bien
A et D forment ensemble un groupe alcanediyle en C₃ à C₅ ou alcènediyle en C₃ à C₅, dans chacun desquels, le cas échéant, un atome de carbone est remplacé par de l'oxygène ou du soufre et qui sont éventuellement substitués par du fluor, du chlore ou par un radical alkyle en C₁ à C₆, alkoxy en C₁ à C₄, alkylthio en C₁ à C₄, cycloalkyle en C₃ à C₆, phényle ou benzyloxy dont chacun est éventuellement substitué par du fluor ou du chlore, ou
dans lequel est contenu, le cas échéant, dans chaque cas, l'un des groupes suivants : 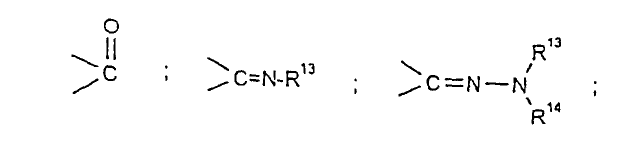 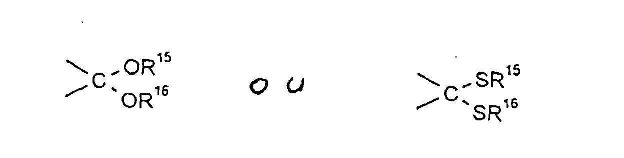
G représente l'hydrogène (a) ou l'un des groupes
E (f) ou 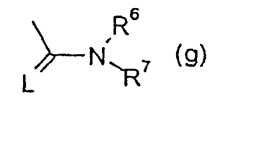 dans lesquels
E représente un équivalent d'ion métallique ou un ion ammonium,
L est l'oxygène ou le soufre et
M représente l'oxygène ou le soufre,
R¹ représente un reste alkyle en C₁ à C₁₆, alcényle en C₂ à C₁₆, (alkoxy en C₁ à C₆)-(alkyle en C₁ à C₆), (alkylthio en C₁ à C₆)-(alkyle en C₁ à C₆) ou poly-(alkoxy en C₁ à C₆)-(alkyle en C₁ à C₆) dont chacun est éventuellement substitué par du fluor ou du chlore, ou un reste cycloalkyle en C₃ à C₇ éventuellement substitué par du fluor, du chlore, un radical alkyle en C₁ à C₅ ou alkoxy en C₁ à C₅, dans lequel, le cas échéant, un ou deux groupes méthylène sont remplacés par de l'oxygène et/ou par du soufre,
un reste phényle éventuellement substitué par du fluor, du chlore, du brome, un radical cyano, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un reste phényl-(alkyle en C₁ à C₄) éventuellement substitué par du fluor, du chlore, du brome, un radical alkyle en C₁ à C₄, alkoxy en C₁ à C₄, halogénalkyle en C₁ à C₄ ou halogénalkoxy en C₁ à C₃,
un reste pyrazolyle, thiazolyle, pyridyle, pyrimidyle, furannyle ou thiényle dont chacun est éventuellement substitué par du fluor, du chlore, du brome ou un radical alkyle en C₁ à C₄,
un reste phénoxy-(alkyle en C₁ à C₅) éventuellement substitué par du fluor, du chlore, du brome ou un radical alkyle en C₁ à C₄, ou bien
un reste pyridyloxy-(alkyle en C₁ à C₅), pyrimidyloxy-(alkyle en C₁ à C₅) ou thiazolyloxy-(alkyle en C₁ à C₅) dont chacun est éventuellement substitué par du fluor, du chlore, du brome, un radical amino ou alkyle en C₁ à C₄,
R² représente un reste alkyle en C₁ à C₁₆, alcényle en C₂ à C₁₆, (alkoxy en C₁ à C₆)-(alkyle en C₂ à C₆) ou poly-(alkoxy en C₁ à C₆)-(alkyle en C₂ à C₆) dont chacun est éventuellement substitué par du fluor ou du chlore,
un reste cycloalkyle en C₃ à C₇ éventuellement substitué par du fluor, du chlore, un radical alkyle en C₁ à C₄ ou alkoxy en C₁ à C₄, ou bien
un reste phényle ou benzyle, chacun étant éventuellement substitué par du fluor, du chlore, du brome, un radical cyano, nitro, alkyle en C₁ à C₄, alkoxy en C₁ à C₃, halogénalkyle en C₁ à C₃ ou halogénalkoxy en C₁ à C₃,
R³ est un reste alkyle en C₁ à C₆ éventuellement substitué par du fluor ou du chlore ou un reste phényle ou benzyle dont chacun est éventuellement substitué par du fluor, du chlore, du brome, un radical alkyle en C₁ à C₄, alkoxy en C₁ à C₄, halogénalkoxy en C₁ ou C₂, halogénalkyle en C₁ ou C₂, cyano ou nitro,
R⁴ et R⁵ représentent, indépendamment l'un de l'autre, un reste alkyle en C₁ à C₆, alkoxy en C₁ à C₆, alkylamino en C₁ à C₆, di(alkyle en C₁ à C₆)amino, alkylthio en C₁ à C₆ ou alcénylthio en C₃ ou C₄ dont chacun est éventuellement substitué par du fluor ou du chlore, ou un reste phényle, phénoxy ou phénylthio dont chacun est éventuellement substitué par du fluor, du chlore, du brome, un radical nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃,
R⁶ et R⁷ représentent, indépendamment l'un de l'autre, l'hydrogène, un reste alkyle en C₁ à C₆, cycloalkyle en C₃ à C₆, alkoxy en C₁ à C₆, alcényle en C₃ à C₆ ou (alkoxy en C₁ à C₆)-(alkyle en C₂ à C₆) dont chacun est éventuellement substitué par du fluor ou du chlore, un reste phényle ou benzyle dont chacun est éventuellement substitué par du fluor, du chlore, du brome, un radical halogénalkyle en C₁ à C₅, alkyle en C₁ à C₅ ou alkoxy en C₁ à C₅, ou forment ensemble un reste alkylène en C₃ à C₆ éventuellement substitué par un radical alkyle en C₁ à C₄ et dans lequel, le cas échéant, un groupe méthylène est remplacé par de l'oxygène ou du soufre,
R¹³ représente l'hydrogène ou un reste alkyle en C₁ à C₆ ou alkoxy en C₁ à C₆ éventuellement substitué par du fluor ou du chlore, un reste cycloalkyle en C₃ à C₇ éventuellement substitué par du fluor, un radical alkyle en C₁ ou C₂ ou alkoxy en C₁ ou C₂ et dans lequel, le cas échéant, un groupe méthylène est remplacé par de l'oxygène ou du soufre, ou un reste phényle, phényl-(alkyle en C₁ à C₃) ou phényl-(alkyloxy en C₁ ou C₂) dont chacun est éventuellement substitué par du fluor, du chlore, du brome, un radical alkyle en C₁ à C₅, alkoxy en C₁ à C₅, halogénalkyle en C₁ ou C₂, halogénalkoxy en C₁ ou C₂, nitro ou cyano,
R¹⁴ xreprésente l'hydrogène ou un groupe alkyle en C₁ à C₆, ou bien
R¹³ et R¹⁴ forment ensemble un groupe alcanediyle en C₄ à C₆,
R¹⁵ et R¹⁶ sont identiques ou différents et représentent un reste alkyle en C₁ à C₄, ou bien
R¹⁵ et R¹⁶ forment ensemble un reste alcanediyle en C₂ ou C₃ qui est éventuellement substitué par un groupe alkyle en C₁ à C₄ ou par un groupe phényle éventuellement substitué par du fluor, du chlore, du brome, un radical alkyle en C₁ ou C₂, halogénalkyle en C₁ ou C₂, alkoxy en C₁ ou C₂, halogénalkoxy en C₁ ou C₂, nitro ou cyano.

3. Composés de formule (I) suivant la revendication 1, dans laquelle
X représente le fluor, le chlore, le brome, un groupe méthyle, éthyle, propyle, butyle, isobutyle, isopropyle, tertio-butyle, méthoxy, éthoxy, propoxy, iso-propoxy, trifluorométhyle, trifluorométhoxy, difluorométhoxy, cyano ou nitro,
Y représente le fluor, le chlore, le brome, un groupe méthyle, éthyle, propyle, isopropyle, butyle, isobutyle, tertio-butyle, méthoxy, éthoxy, propoxy, iso-propoxy, trifluorométhyle, trifluorométhoxy, difluorométhoxy, cyano ou nitro,
Z représente le fluor, le chlore, le brome, un groupe méthyle, éthyle, propyle, butyle, isobutyle, isopropyle, tertio-butyle, méthoxy, éthoxy, propoxy, isopropoxy, trifluorométhyle, trifluorométhoxy, difluorométhoxy, cyano ou nitro, ou bien
Y et Z forment ensemble un groupe alcanediyle en C₃ ou C₄ éventuellement substitué par du fluor, du chlore, un radical méthyle, éthyle, propyle, isopropyle, méthoxy, éthoxy, propoxy, isopropoxy ou trifluorométhyle, dans lequel, le cas échéant, deux chaînons non contigus sont remplacés par de l'oxygène, X ayant les définitions indiquées ci-dessus,
Het représente l'un des groupes 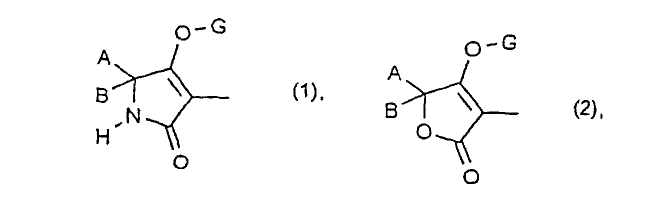 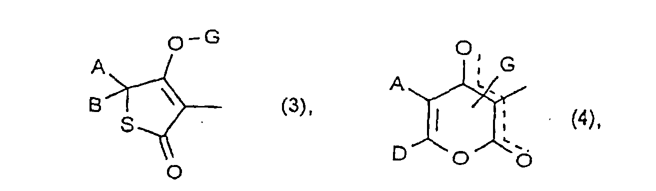 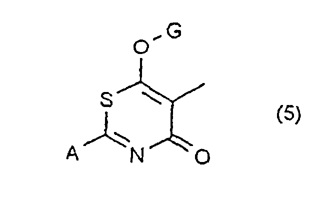
A représente l'hydrogène, un groupe alkyle en C₁ à C₈, alcényle en C₂ à C₄, (alkoxy en C₁ à C₆)-(alkyle en C₁ à C₄), poly-(alkoxy en C₁ à C₄)-(alkyle en C₁ à C₄) ou (alkylthio en C₁ à C₆)-(alkyle en C₁ à C₄) dont chacun est éventuellement substitué par du fluor ou du chlore, ou bien un groupe cycloalkyle en C₃ à C₆ éventuellement substitué par du fluor, du chlore, un radical méthyle ou méthoxy dont, le cas échéant, un ou deux groupes méthylène sont remplacés par de l'oxygène et/ou du soufre, ou bien un reste phényle, pyridyle ou benzyle dont chacun est éventuellement substitué par du fluor, du chlore, du brome, un radical méthyle, éthyle, n-propyle, isopropyle, méthoxy, éthoxy, trifluorométhyle, trifluorométhoxy, cyano ou nitro,
B représente l'hydrogène, un groupe alkyle en C₁ à C₈ ou (alkoxy en C₁ à C₄)-(alkyle en C₁ ou C₂), ou bien
A, B et l'atome de carbone auquel il sont liés représentent un groupe cycloalkyle en C₃ à C₈ ou cycloalcényle en C₅ à C₈, dans chacun desquels, le cas échéant, un groupe méthylène est remplacé par de l'oxygène ou du soufre et qui sont éventuellement substitués par un radical méthyle, éthyle, n-propyle, isopropyle, butyle, isobutyle, sec.-butyle, tertio-butyle, cyclohexyle, trifluorométhyle, méthoxy, éthoxy, n-propoxy, isopropoxy, butoxy, isobutoxy, sec.-butoxy, tertiobutoxy, méthylthio, éthylthio, fluoro, chloro ou phényle, ou bien
A, B et l'atome de carbone auquel ils sont liés représentent un groupe cycloalkyle en C₅ ou C₆ qui est substitué par un groupe alkylènediyle contenant éventuellement un atome d'oxygène ou de soufre ou par un groupe alkylènedioxyle qui forme avec l'atome de carbone auquel il est lié un noyau pentagonal ou hexagonal, ou bien
A, B et l'atome de carbone auquel ils sont liés représentent un groupe cycloalkyle en C₃ à C₆ ou cycloalcényle en C₅ ou C₆ dans lequel deux substituants forment conjointement avec les atomes de carbone auxquels ils sont liés un groupe alcanediyle en C₃ ou C₄, alcènediyle en C₃ ou C₄ ou butadiènediyle, dans chacun desquels, le cas échéant, un groupe méthylène est remplacé par de l'oxygène ou du soufre,
D représente l'hydrogène, un groupe, éventuellement substitué dans chaque cas par du fluor ou du chlore, alkyle en C₁ à C₈, alcényle en C₃ ou C₄, alcynyle en C₃ ou C₄, (alkoxy en C₁ à C₆)-(alkyle en C₂ à C₄), poly-(alkoxy en C₁ à C₄)-(alkyle en C₂ à C₄), (alkylthio en C₁ à C₄)-(alkyle en C₂ à C₄) ou cycloalkyle en C₃ à C₆ dans lequel, le cas échéant, un ou deux groupes méthylène non contigus sont remplacés par de l'oxygène et/ou du soufre, ou un groupe phényle, furannyle, pyridyle, thiényle ou benzyle dont chacun est éventuellement substitué par du fluor, du chlore, du brome, un radical méthyle, éthyle, n-propyle, isopropyle, méthoxy, éthoxy, trifluorométhyle, trifluorométhoxy, cyano ou nitro,
ou bien
A et D forment ensemble un groupe alcanediyle en C₃ à C₅ ou alcènediyle en C₃ à C₅, dans chacun desquels, le cas échéant, un groupe méthylène est remplacé par de l'oxygène ou du soufre et qui sont substitués, le cas échéant, par du fluor, du chlore ou par un groupe alkyle en C₁ à C₆, alkoxy en C₁ à C₄, alkylthio en C₁ à C₄, cycloalkyle en C₃ à C₆, phényle ou benzyloxy dont chacun est éventuellement substitué par du fluor ou du chlore,
G représente l'hydrogène (a) ou l'un des groupes 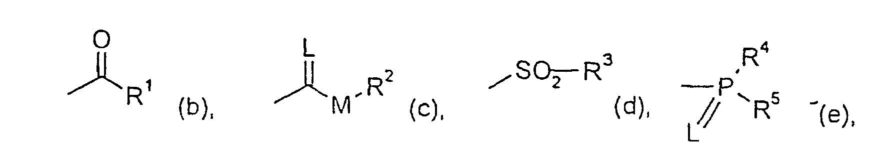
E (f) ou 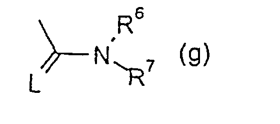 dans lesquels
E représente un équivalent d'ion métallique ou un ion ammonium,
L représente l'oxygène ou le soufre et
M représente l'oxygène ou le soufre,
R¹ représente un groupe alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly-(alkoxy en C₁ à C₄)-(alkyle en C₁ à C₄) dont chacun est éventuellement substitué par du fluor ou du chlore, ou un groupe cycloalkyle en C₃ à C₆ éventuellement substitué par du fluor, du chlore, un radical méthyle, éthyle, n-propyle, isopropyle, n-butyle, isobutyle, tertio-butyle, méthoxy, éthoxy, n-propoxy ou iso-propoxy, dans lequel, le cas échéant, un ou deux groupes méthylène sont remplacés par de l'oxygène et/ou du soufre,
un groupe phényle éventuellement substitué par du fluor, du chlore, du brome, un radical cyano, nitro, méthyle, éthyle, n-propyle, isopropyle, méthoxy, éthoxy, trifluorométhyle, trifluoromethoxy, méthylthio, éthylthio, méthylsulfonyle ou éthylsulfonyle,
un groupe benzyle éventuellement substitué par du fluor, du chlore, du brome, un groupe méthyle, éthyle, n-propyle, isopropyle, méthoxy, éthoxy, trifluorométhyle ou trifluorométhoxy,
un groupe furannyle, thiényle ou pyridyle dont chacun est éventuellement substitué par du fluor, du chlore, du brome, un radical méthyle ou éthyle,
un groupe phénoxy-(alkyle en C₁ à C₄) éventuellement substitué par du fluor, du chlore, du brome, un radical méthyle ou éthyle, ou bien
un groupe pyridyloxy- (alkyle en C₁ à C₄), pyrimidyloxy-(alkyle en C₁ ou C₄) ou thiazolyloxy-(alkyle en C₁ à C₄) dont chacun est éventuellement substitué par du fluor, du chlore, un radical amino, méthyle ou éthyle,
R² représente un groupe alkyle en C₁ à C₁₄, alcényle en C₂ à C₁₄, (alkoxy en C₁ à C₄)-(alkyle en C₂ à C₆) ou poly-(alkoxy en C₁ à C₄)-(alkyle en C₂ à C₆) dont chacun est éventuellement substitué par du fluor ou du chlore,
un groupe cycloalkyle en C₃ à C₆ éventuellement substitué par du fluor, du chlore, un radical méthyle, éthyle, n-propyle, isopropyle ou méthoxy, ou
un groupe phényle ou benzyle dont chacun est éventuellement substitué par du fluor, du chlore, un radical cyano, nitro, méthyle, éthyle, n-propyle, isopropyle, méthoxy, éthoxy, trifluorométhyle ou trifluorométhoxy,
R³ représente un groupe méthyle éventuellement substitué par du fluor ou du chlore, un groupe éthyle, propyle, isopropyle, butyle, tertio-butyle ou un groupe phényle ou benzyle dont chacun est éventuellement substitué par du fluor, du chlore, du brome, un radical méthyle, éthyle, isopropyle, tertio-butyle, méthoxy, éthoxy, isopropoxy, trifluorométhyle, trifluorométhoxy, cyano ou nitro,
R⁴ et R⁵ représentent, indépendamment l'un de l'autre, un groupe alkyle en C₁ à C₄, alkoxy en C₁ à C₄, alkylamino en C₁ à C₄, di(alkyle en C₁ à C₄)amino ou alkylthio en C₁ à C₄ dont chacun est éventuellement substitué par du fluor ou du chlore, ou un groupe phényle, phénoxy ou phénylthio dont chacun est éventuellement substitué par du fluor, du chlore, du brome, un radical nitro, cyano, méthyle, méthoxy, trifluorométhyle ou trifluorométhoxy,
R⁶ et R⁷ représentent, indépendamment l'un de l'autre, l'hydrogène, un groupe alkyle en C₁ à C₄, cycloalkyle en C₃ à C₆, alkoxy en C₁ à C₄, alcényle en C₃ ou C₄ ou (alkoxy en C₁ à C₄)-(alkyle en C₂ à C₄) dont chacun est éventuellement substitué par du fluor ou du chlore, un groupe phényle ou benzyle dont chacun est éventuellement substitué par du fluor, du chlore, du brome, un radical méthyle, méthoxy ou trifluorométhyle, ou forment ensemble un reste alkylène en C₅ ou C₆ éventuellement substitué par un radical méthyle ou éthyle et dans lequel, le cas échéant, un groupe méthylène est remplacé par de l'oxygène ou du soufre.

4. Procédé de production de composés de formule (I) suivant la revendication 1, **caractérisé en ce que** :
(A) pour obtenir des composés de formule (I-1-a) 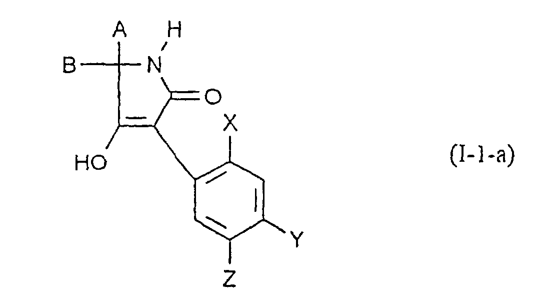 dans laquelle
A, B, X, Y et Z ont les définitions indiquées dans la revendication 1,
on effectue la condensation intramoléculaire de composés de formule (II) 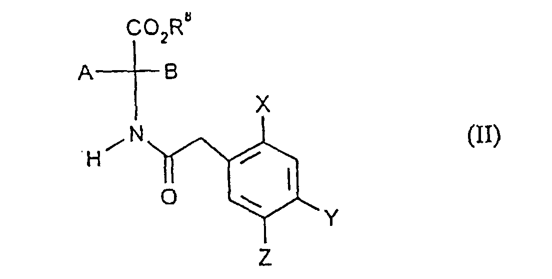 dans laquelle
A, B, X, Y et Z ont les définitions indiquées dans la revendication 1,
et
R⁸ xreprésente un groupe alkyle,
en présence d'un diluant et en présence d'une base,
(B) pour obtenir des composés de formule (I-2-a) 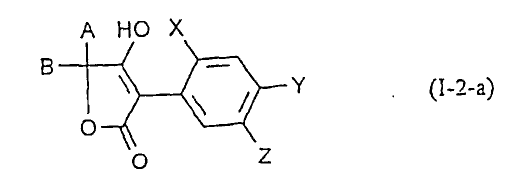 dans laquelle
A, B, X, Y et Z ont les définitions indiquées ci-dessus,
on effectue la condensation intramoléculaire de composés de formule (III) 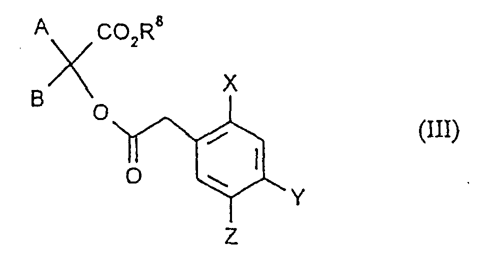 dans laquelle
A, B, X, Y, Z et R⁸ ont les définitions indiquées ci-dessus, en présence d'un diluant et en présence d'une base,
(C) pour obtenir des composés de formule (I-3-a) 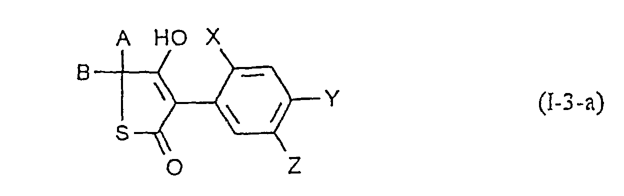 dans laquelle
A, B, X, Y et Z ont les définitions indiquées ci-dessus,
on effectue la cyclisation intramoléculaire de composés de formule (IV) 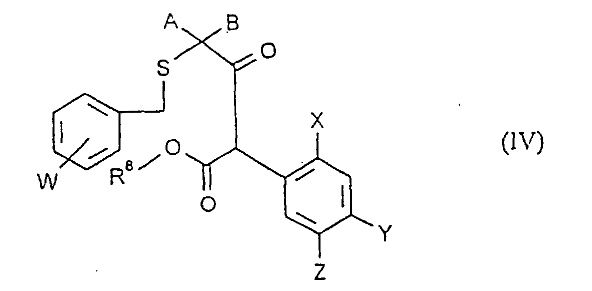 dans laquelle
A, B, X, Y, Z et R⁸ ont les définitions indiquées ci-dessus et
W représente l'hydrogène, un halogène, un groupe alkyle ou un groupe alkoxy,
le cas échéant en présence d'un diluant et en présence d'un acide,
(D) pour obtenir des composés de formule (I-4-a) 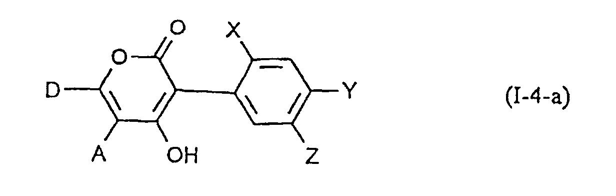 dans laquelle
A, D, X, Y et Z ont les définitions indiquées dans la revendication 1,
on fait réagir des composés de formule (V) 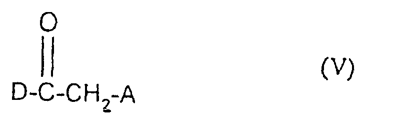 dans laquelle
A et D ont les définitions indiquées ci-dessus,
ou leurs éthers de silylénol de formule (Va) 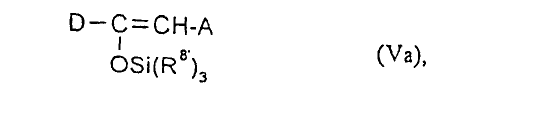 dans laquelle A et D ont la définition mentionnée ci-dessus et
R^{8'} représente un groupe alkyle,
avec des composés de formule (VI) 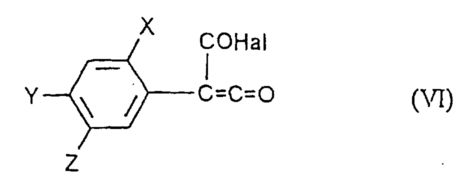 dans laquelle
X, Y et Z ont les définitions indiquées ci-dessus et
Hal représente un halogène,
le cas échéant en présence d'un diluant et en présence éventuelle d'un accepteur d'acide,
(E) pour obtenir des composés de formule (I-5-a) 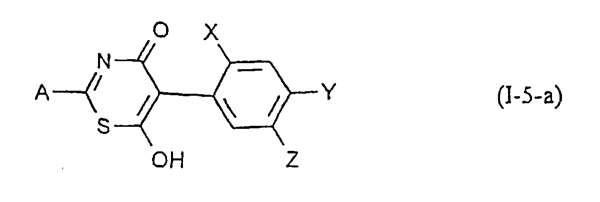 dans laquelle
A, X, Y et Z ont les définitions indiquées ci-dessus,
on fait réagir des composés de formule (VII) 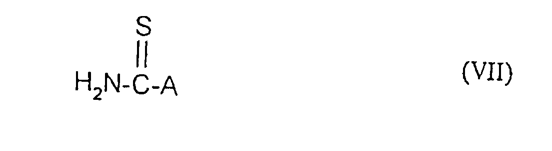 dans laquelle
A a la définition indiquée ci-dessus,
avec des composés de formule (VI) 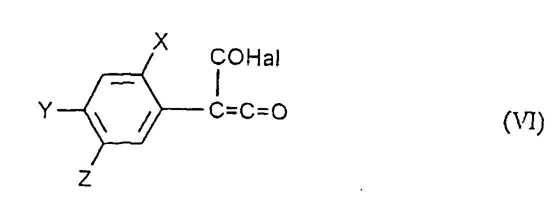 dans laquelle
Hal, X, Y et Z ont les définitions indiquées ci-dessus,
éventuellement en présence d'un diluant et en présence éventuelle d'un accepteur d'acide, après quoi, le cas échéant, on fait réagir les composés ainsi obtenus de formules (I-1-b) à (I-5-b) indiquées ci-dessus
(F)
α avec des halogénures d'acides de formule (VIII) 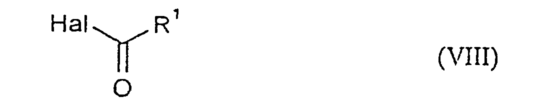 dans laquelle
R¹ a la définition indiquée dans la revendication 1 et
Hal représente un halogène,
ou bien
β) avec des anhydrides d'acides carboxyliques de formule (IX)
R¹-CO-O-CO-R¹ (IX)
dans laquelle
R¹ a la définition indiquée ci-dessus,
le cas échéant en présence d'un diluant et en présence éventuelle d'un accepteur d'acide,
ou bien
(G) on les fait réagir avec des esters d'acide chloroformique ou des thioesters d'acide chloroformique de formule (X)
R²-M-CO-Cl (X)
dans laquelle
R² et M ont les définitions indiquées dans la revendication 1,
éventuellement en présence d'un diluant et en présence éventuelle d'un accepteur d'acide,
ou bien
(H)
α on les fait réagir avec des esters d'acide chloro-monothioformique ou des esters d'acide chlorodithioformique de formule (XI) 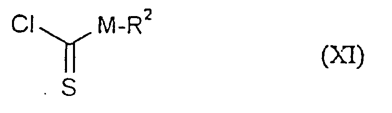 dans laquelle
M et R² ont les définitions indiquées ci-dessus,
éventuellement en présence d'un diluant et en présence éventuelle d'un accepteur d'acide,
ou bien
β) on les fait réagir avec le sulfure de carbone, puis avec des composés de formule (XII)
R²-Hal (XII)
dans laquelle
R² a la définition indiquée ci-dessus et
Hal représente le chlore, le brome ou l'iode,
éventuellement en présence d'un diluant et en présence éventuelle d'une base,
ou bien
(I) on les fait réagir avec des chlorures d'acide sulfonique de formule (XIII)
R³-SO₂-Cl (XIII)
dans laquelle
R³ a la définition indiquée dans la revendication 1, éventuellement en présence d'un diluant et en présence éventuelle d'un accepteur d'acide,
ou bien
(J) on les fait réagir avec des composés phosphorés de formule (XIV) 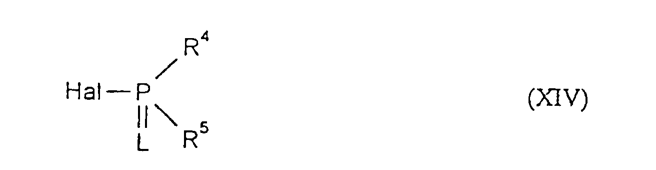 dans laquelle
L, R⁴ et R⁵ ont les définitions indiquées ci-dessus et Hal représente un halogène, le cas échéant en présence d'un diluant et en présence éventuelle d'un accepteur d'acide,
ou bien
(K) on les fait réagir avec des composés métalliques ou des aminés de formules (XV) ou (XVI)
Me(OR¹⁰)ₜ (XV) 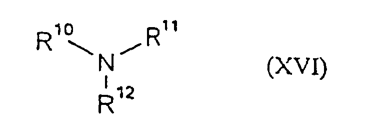
dans lesquelles
Me représente un métal monovalent ou divalent
t représente le nombre 1 ou 2 et
R¹⁰, R¹¹ et R¹² représentent, indépendamment les uns des autres, l'hydrogène ou un groupe alkyle,
le cas échéant en présence d'un diluant,
ou bien
(L)
α) on les fait réagir avec des isocyanates ou des isothiocyanates de formule (XVII)
R⁶-N=C=L (XVIII)
dans laquelle
R⁶ et L ont les définitions indiquées dans la revendication 1,
éventuellement en présence d'un diluant et en présence éventuelle d'un catalyseur, ou bien
β) on les fait réagir avec des chlorures d'acide carbamique ou des chlorures d'acide thiocarbamique de formule (XVIII) 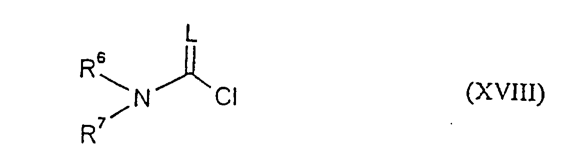 dans laquelle
L, R⁶ et R⁷ ont les définitions indiquées ci-dessus,
le cas échéant en présence d'un diluant et en présence éventuelle d'un accepteur d'acide.

5. Composés de formule (II) 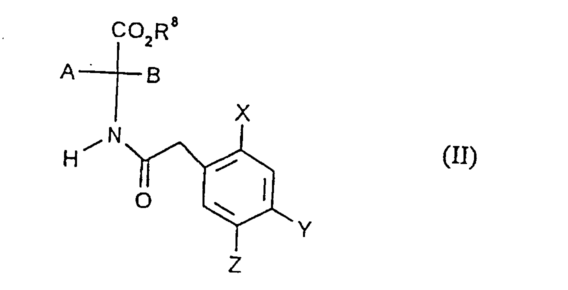 dans laquelle
A, B, X, Y et Z ont les définitions indiquées dans la revendication 1 et
R⁸ représente un groupe alkyle.

6. Composés de formule (III) 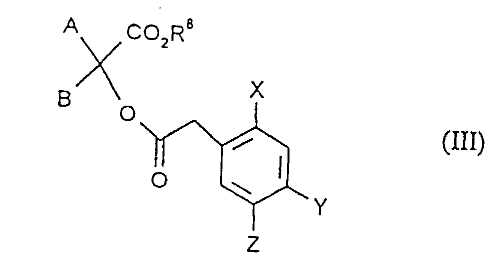 dans laquelle
A, B, X, Y et Z ont les définitions indiquées dans la revendication 1 et
R⁸ est un groupe alkyle.

7. Composés de formule (XXI) 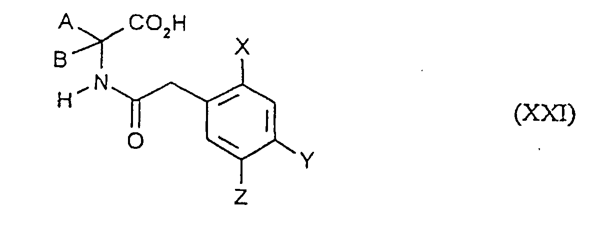 dans laquelle
A, B, X, Y et Z ont les définitions indiquées dans la revendication 1.

8. Composés de formule (XXIX) 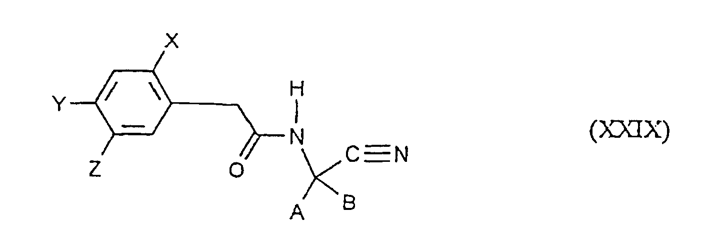 dans laquelle
A, B, X, Y et Z ont les définitions indiquées dans la revendication 1.

9. Composés de formule (IV) 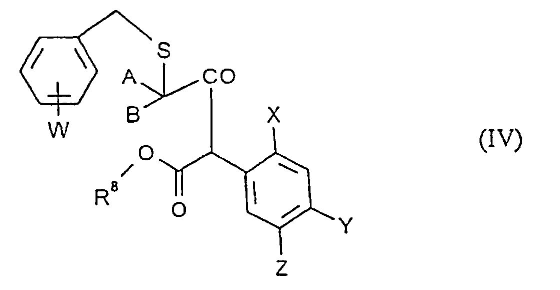 dans laquelle
A, B, X, Y et Z ont les définitions indiquées dans la revendication 1,
R⁸ est un groupe alkyle et
W est l'hydrogène, un halogène, un groupe alkyle ou alkoxy.

10. Composés de formule (VI) 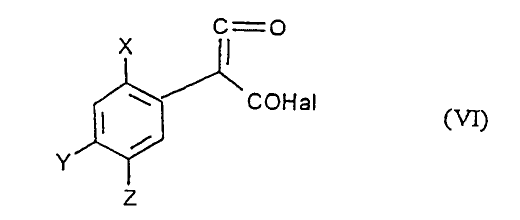 dans laquelle
X, Y et Z ont les définitions indiquées dans la revendication 1 et
Hal représente le chlore ou le brome.

11. Composés de formule (XXXIII) 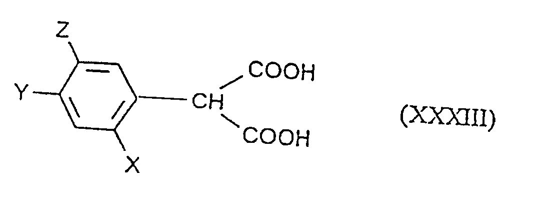 dans laquelle
X, Y et Z ont les définitions indiquées dans la revendication 1.

12. Composés de formule (XXXIV) 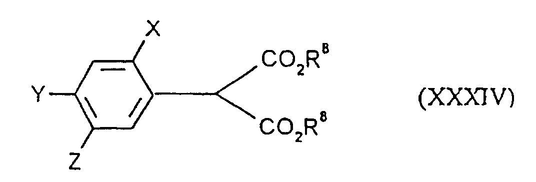 dans laquelle
X, Y et Z ont les définitions indiquées dans la revendication 1 et
R⁸ représente un groupe alkyle.

13. Pesticides et herbicides, **caractérisés par** une teneur en au moins un composé de formule (I) suivant la revendication 1.

14. Utilisation de composés de formule (I) suivant la revendication 1 pour combattre des parasites et une végétation indésirable.

15. Procédé pour combattre des parasites, **caractérisé en ce qu'**on fait agir des composés de formule (I) suivant la revendication 1 sur les parasites, sur une végétation indésirable et/ou sur leur milieu.

16. Procédé de préparation de pesticides et herbicides, **caractérisé en ce qu'**on mélange des composés de formule (I) suivant la revendication 1 avec des diluants et/ou des agents tensio-actifs.

17. Utilisation de composés de formule (I) suivant la revendication 1 pour la préparation de pesticides et herbicides.
